# EUROPEAN PATENT APPLICATION

(11) **EP 4 696 691 A1**
(43) Date of publication of application: **18.02.2026**
(21) Application number: 24814528.6
(22) Date of filing: 30.05.2024
(51) Int. Cl.: C07D 498/04, C07D 401/00, C07D 519/00, A61P 25/00, A61P 9/00, A61P 29/00, A61K 31/519

(54) **TRICYCLIC COMPOUND, AND PREPARATION METHOD THEREFOR AND USE THEREOF**

(30) Priority: 30.05.2023 CN 202310624222; 22.12.2023 CN 202311782129
(71) Applicant: Shanghai Huilun Pharmaceutical Co., Ltd., Shanghai 200241 (CN)
(72) Inventor: WANG, Xinfeng, Shanghai 200241 (CN); GOU, Jun, Shanghai 200241 (CN); DU, Shuqi, Shanghai 200241 (CN); CHEN, Lei, Shanghai 200241 (CN); QIN, Jihong, Shanghai 200241 (CN)
(74) Representative: Ipsilon
(86) International application number: PCT/CN2024/096278
(87) International publication number: WO 2024/245331

(57) **Abstract**

A tricyclic compound, and a preparation method therefor and the use thereof. The present invention relates to a compound as shown in general formula (I), a preparation method therefor, a pharmaceutical composition containing the compound, and the use thereof in the preparation of a drug for treating or preventing autoimmune diseases, inflammatory diseases, cancers, viral diseases, neurodegenerative diseases, genetic disorders, hormone-related diseases, metabolic disorders, organ-transplantation-related diseases, immunodeficiency disorders, destructive bone diseases, proliferative disorders, infectious diseases, cell-death-related conditions, or cardiovascular diseases. Each substituent in general formula (I) is as defined in the description.

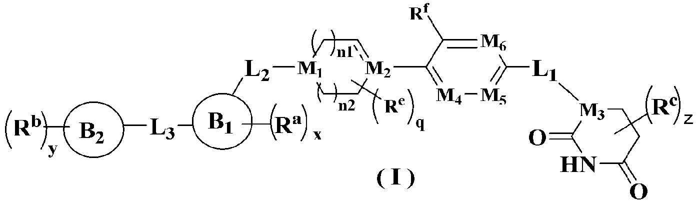

## Description

The present application claims priority to Chinese Patent Application No. 202310624222X filed on May 30, 2023, and priority to Chinese Patent Application No. 2023117821298 filed on December 22, 2023, the full text of which is incorporated herein by reference.

### TECHNICAL FIELD

The present application belongs to the field of medicine, and in particular relates to a tricyclic compound, and a preparation method therefor and use thereof.

### BACKGROUND

Interleukin-1 receptor-associated kinase 4 (IRAK4) is a serine/threonine specific protein kinase, belongs to the tyrosine kinase (TLK) family, and is a key node in innate immune response involving interleukin-1, 18, 33 receptors and Toll-like receptors. After binding to interleukin receptors or Toll-like receptors, extracellular signal molecules recruit to form an MyD88:IRAK4:IRAK1/2 multiprotein complex, leading to IRAK1/2 phosphorylation and mediating a series of downstream signaling, thereby activating p38, JNK, and NF-kB signaling pathways, and ultimately resulting in expression of proinflammatory cytokines. Clinical pathological studies have shown that individuals with IRAK4 mutations have a protective function against chronic lung diseases and inflammatory bowel diseases. IRAK4 deficiency is not lethal, individuals with IRAK4 deficiency can survive into adulthood, and the risk of infection decreases with age. Therefore, IRAK4 has become an important therapeutic target and attracted widespread research and development interests.

Proteolysis targeting chimeria (PROTAC) is a technology that differs from traditional small molecule inhibitors. Traditional small molecule inhibitors typically need to act on an active site of a target protein to inhibit its activity. However, PROTAC is a heterogeneous bifunctional molecule, with one end being a small molecule inhibitor that can recognize a target protein, and the other end being an E3 ubiquitin ligase ligand that can recognize E3 ubiquitin ligase, and the two ends are connected through a connecting chain. The bifunctional molecule recognizes the target protein and the E3 ubiquitin ligase in vivo, bringing the target protein and the E3 ubiquitin ligase closer together to form a ternary complex. After ubiquitination of the target protein, the target protein is degraded in vivo through a ubiquitin-proteasome pathway. Compared to traditional small molecule inhibitors, in a first aspect, PROTAC only requires bringing the target protein and the E3 ubiquitin ligase closer together to degrade a substrate, and such a mode of action allows the technology to be applied to some undruggable targets. In a second aspect, because PROTAC molecules can be released to participate in a degradation process of the next protein after the target protein is degraded, such a catalytic degradation effect allows for efficient degradation with a low drug dose of PROTAC. In a third aspect, traditional small molecule inhibitors are prone to developing drug resistance often due to point mutations, which cause the small molecule inhibitors to lose the inhibitory effect on a target, while PROTAC can directly degrade a target protein, and can to some extent avoid the drug resistance caused by point mutations. Therefore, compared to traditional small molecule inhibitors, development of new small molecule drugs using the PROTAC technology has high advantages and feasibility, and the new small molecule drugs are expected to become the next generation of highly promising new drugs.

Therefore, it is necessary to develop novel IRAK4 inhibitors and E3 ubiquitin ligase PROTAC drugs for treating IRAK4-related diseases.

### SUMMARY

The present application provides An objective of the present application is to provide a compound as shown in general formula (I), a stereoisomer thereof, or a pharmaceutically acceptable salt thereof, where a structure of the compound as shown in general formula (I) is as follows: 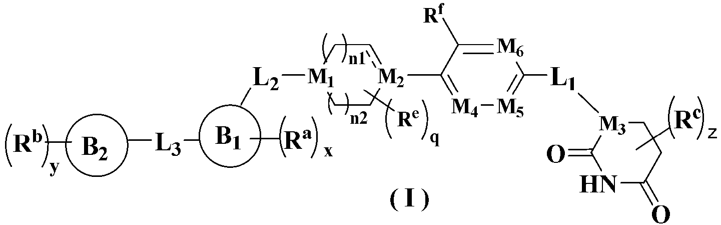 where
------ represents a bond or non-existence;
M₁ is selected from N or CR₁;
M₂ is selected from N, C, or CR₂;
M₃ is selected from N or CR₃;
M₄ is selected from N or CR₄;
M₅ is selected from N or CR₅;
M₆ is selected from N or CR₆;
R₁, R₂, R₃, R₄, R₅, and R₆ are each independently selected from hydrogen, deuterium, halogen, cyano, hydroxyl, aminoalkyl, oxo, alkyl, alkoxyl, hydroxyalkyl, haloalkyl, haloalkoxyl, cycloalkyl, or heterocyclyl;
ring B₁ is selected from aryl or heteroaryl;
ring B₂ is selected from aryl, heteroaryl, or heterocyclyl;
R^{a} is each independently selected from hydrogen, deuterium, hydroxyl, halogen, cyano, alkyl, alkoxyl, hydroxyalkyl, haloalkyl, haloalkoxyl, -P(O)RR', cycloalkyl, or heterocyclyl, the alkyl, alkoxyl, hydroxyalkyl, haloalkyl, haloalkoxyl, cycloalkyl, and heterocyclyl being optionally further substituted with one or more substituents selected from deuterium, halogen, hydroxyl, cyano, or alkyl;
R and R' are each independently selected from hydrogen, deuterium, halogen, alkyl, alkoxyl, haloalkyl, or haloalkoxyl;
R^{b}, R^{c}, R^{e}, and R^{f} are each independently selected from hydrogen, deuterium, halogen, cyano, oxo, alkyl, alkoxyl, hydroxyalkyl, haloalkyl, haloalkoxyl, cycloalkyl, or heterocyclyl, the alkyl, alkoxyl, hydroxyalkyl, haloalkyl, haloalkoxyl, cycloalkyl, and heterocyclyl being further substituted with one or more substituents selected from deuterium, halogen, alkyl, alkoxyl, hydroxyalkyl, haloalkyl, or haloalkoxyl;
or, R^{e} and R^{f} are linked to form cycloalkyl, heterocyclyl, aryl, or heteroaryl, the cycloalkyl, heterocyclyl, aryl, and heteroaryl being optionally further substituted with one or more substituents selected from deuterium, halogen, oxo, hydroxyl, alkyl, alkoxyl, hydroxyalkyl, haloalkyl, haloalkoxyl, cycloalkyl, or heterocyclyl;
or, R₂ and R^{f} are linked to form cycloalkyl, heterocyclyl, aryl, or heteroaryl, the cycloalkyl, heterocyclyl, aryl, and heteroaryl being optionally further substituted with one or more substituents selected from deuterium, halogen, oxo, alkyl, alkoxyl, hydroxyalkyl, haloalkyl, haloalkoxyl, cycloalkyl, or heterocyclyl;
or, R^{f} is linked to C or N on a ring where M₂ is located to form cycloalkyl, heterocyclyl, aryl, or heteroaryl, the cycloalkyl, heterocyclyl, aryl, and heteroaryl being optionally further substituted with one or more substituents selected from deuterium, halogen, oxo, alkyl, alkoxyl, hydroxyalkyl, haloalkyl, haloalkoxyl, cycloalkyl, or heterocyclyl;
or, any two R^{b} are linked to form cycloalkyl, heterocyclyl, aryl, or heteroaryl, the cycloalkyl, heterocyclyl, aryl, and heteroaryl being optionally further substituted with one or more substituents selected from deuterium, halogen, cyano, amino, cyano, oxo, alkyl, alkoxyl, hydroxyalkyl, haloalkyl, haloalkoxyl, cycloalkyl, or heterocyclyl;
or, L₂ and R^{e} are linked to form cycloalkyl, heterocyclyl, aryl, or heteroaryl, the cycloalkyl, heterocyclyl, aryl, and heteroaryl being optionally further substituted with one or more substituents selected from deuterium, halogen, cyano, amino, cyano, oxo, alkyl, alkoxyl, hydroxyalkyl, haloalkyl, haloalkoxyl, cycloalkyl, or heterocyclyl;
L₁ is selected from a bond, -NH-, -S-, -O-, -CH₂-, CH₂CH₂-, -C(O)NH-, -NHC(O)-, or -C(O)-;
L₂ is -Ak1-Cyl-Ak2-Cy2-Ak3-,
Ak1, Ak2, and Ak3 being each independently selected from -(CH₂)ₙ₃-, -O-, -C(O)-, - NH-, -NR₇-, -CH₂NR₇-, -(CR₈R₉)ₙ₄-, alkynylene, or a bond,
Cy1 and Cy2 being each independently selected from a bond, cycloalkylene, heterocyclylene, arylene, or heteroarylene, the cycloalkylene, heterocyclylene, arylene, and heteroarylene being optionally further substituted with 1 to 4 substituents selected from deuterium, halogen, amino, hydroxyl, cyano, nitro, oxo, alkyl, haloalkyl, alkoxyl, hydroxyalkyl, or haloalkoxyl;
R₇, R₈, and R₉ are each independently selected from hydrogen, deuterium, halogen, alkyl, cyano, hydroxyl, cycloalkyl, haloalkyl, deuterated alkyl, halocycloalkyl, hydroxyalkyl, or alkoxyl; or, R₈ and R₉ are linked to form cycloalkyl, heterocyclyl, aryl, or heteroaryl, the cycloalkyl, heterocyclyl, aryl, and heteroaryl being optionally further substituted with one or more substituents selected from deuterium, halogen, hydroxyl, amino, cyano, oxo, alkyl, alkoxyl, haloalkyl, haloalkoxyl, or hydroxyalkyl;
or, R^{a} and L₂ are linked to form cycloalkyl, heterocyclyl, aryl, or heteroaryl, the cycloalkyl, heterocyclyl, aryl, and heteroaryl being optionally further substituted with one or more substituents selected from deuterium, halogen, amino, cyano, hydroxyl, oxo, alkyl, alkoxyl, hydroxyalkyl, haloalkyl, haloalkoxyl, cycloalkyl, or heterocyclyl;
L₃ is selected from a bond, -NH-C(O)-, -C(O)-NH-, -NH-C(S)-, or -C(S)-NH-;
x, y, z, and q are each independently selected from 0, 1, 2, 3, or 4; and
n1, n2, n3, and n4 are each independently selected from 0, 1, 2, or 3.

In a preferred embodiment of the present application, the aforementioned compound is further shown in general formula (II-A): 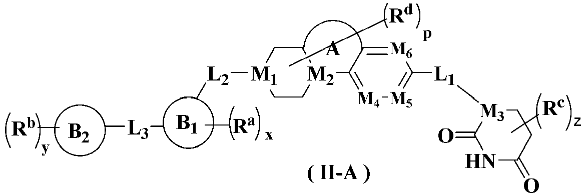 where
R^{d} is each independently selected from hydrogen, deuterium, halogen, oxo, hydroxyl, C₁₋₆ alkyl, C₁₋₆ alkoxyl, hydroxy C₁₋₆ alkyl, C₁₋₆ haloalkyl, C₁₋₆ haloalkoxyl, C₃₋₆ cycloalkyl, or 3-6 membered heterocyclyl;
preferably, R^{d} is each independently selected from hydrogen, deuterium, fluoro, chloro, oxo, hydroxyl, C₁₋₃ alkyl, C₁₋₃ alkoxyl, hydroxy C₁₋₃ alkyl, C₁₋₃ haloalkyl, C₁₋₃ haloalkoxyl, C₃₋₆ cycloalkyl, or 3-6 membered heterocyclyl;
or, R^{d} and L₂ are linked to form a C₃₋₆ cycloalkyl or 3-6 membered heterocyclyl, the C₃₋₆ cycloalkyl and 3-6 membered heterocyclyl being optionally further substituted with one or more substituents selected from deuterium, halogen, cyano, oxo, C₁₋₆ alkyl, C₁₋₆ alkoxyl, hydroxy C₁₋₆ alkyl, C₁₋₆ haloalkyl, C₁₋₆ haloalkoxyl, C₃₋₆ cycloalkyl, or 3-6 membered heterocyclyl;
ring A is selected from 5-7 membered heterocyclyl or 5-6 membered heteroaryl; and
p is selected from 0, 1, 2, 3, or 4.

In a preferred embodiment of the present application, the aforementioned compound is further shown in general formula (II-B): 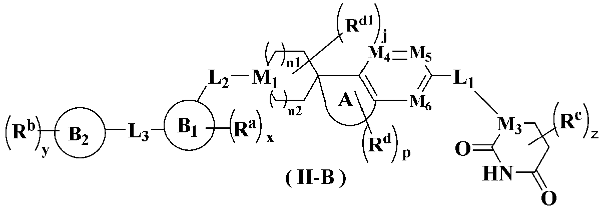 where
R^{d} is each independently selected from hydrogen, deuterium, halogen, oxo, C₁₋₆ alkyl, C₁₋₆ alkoxyl, hydroxy C₁₋₆ alkyl, C₁₋₆ haloalkyl, C₁₋₆ haloalkoxyl, C₃₋₆ cycloalkyl, or 3-6 membered heterocyclyl;
ring A is selected from 5-6 membered heterocyclyl or 5-6 membered heteroaryl; and
R^{d1} is each independently selected from hydrogen, deuterium, halogen, oxo, C₁₋₆ alkyl, C₁₋₆ alkoxyl, hydroxy C₁₋₆ alkyl, C₁₋₆ haloalkyl, C₁₋₆ haloalkoxyl, C₃₋₆ cycloalkyl, or 3-6 membered heterocyclyl; and
p and j are each independently selected from 0, 1, 2, 3, or 4.

In a preferred embodiment of the present application, 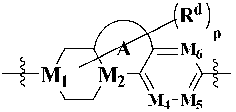 in the aforementioned general formula (II-A) is selected from 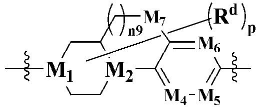 or 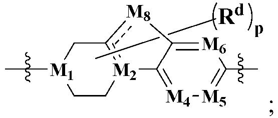
preferably, the 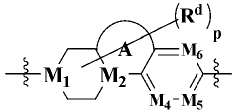 is selected from 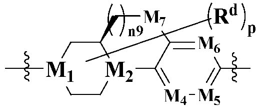 or 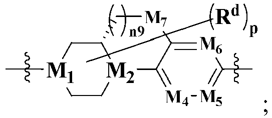
more preferably, 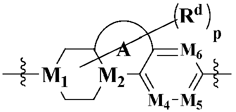 is selected from 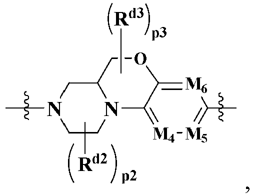 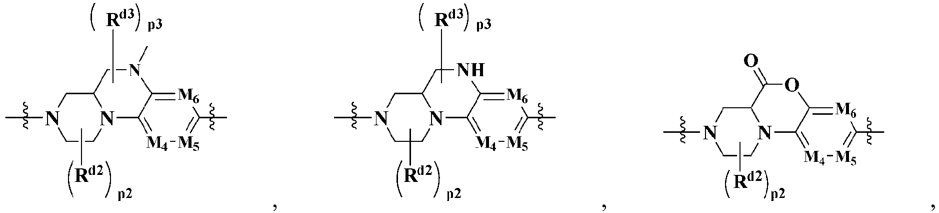 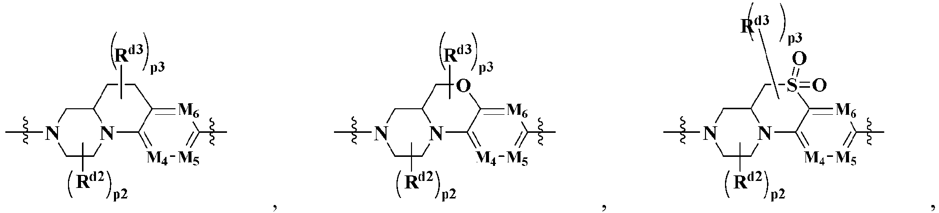 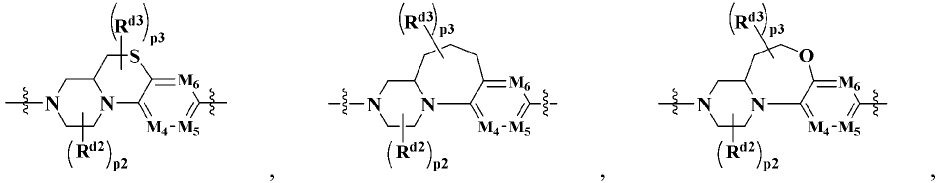 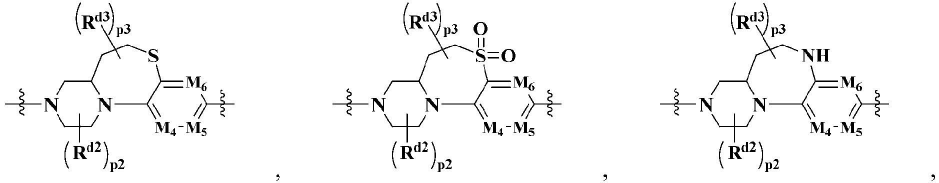 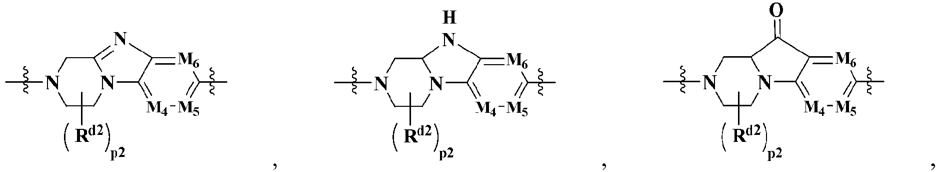 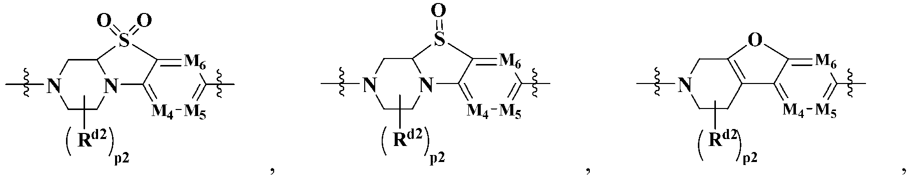 or 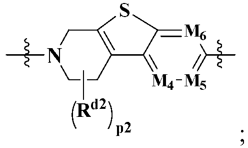
further preferably, the 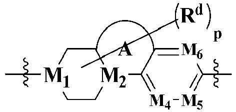 is selected from 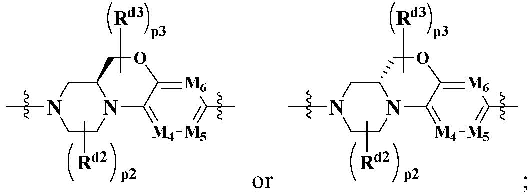
M₇ is selected from O, CH₂, C(O), S, S(O), S(O)₂, or NR₁₀; R₁₀ is selected from hydrogen, deuterium, C₁₋₃ alkyl, C₁₋₃ alkoxyl, C₁₋₃ haloalkyl, C₁₋₃ haloalkoxyl, C₃₋₆ cycloalkyl, or 3-6 membered heterocyclyl, the C₁₋₃ alkyl, C₁₋₃ alkoxyl, C₁₋₃ haloalkyl, C₁₋₃ haloalkoxyl, C₃₋₆ cycloalkyl, and 3-6 membered heterocyclyl being optionally further substituted with one or more substituents selected from deuterium, hydroxyl, cyano, amino, oxo, fluoro, or chloro;
M₈ is selected from N, O, S, C(O), CH₂, CH, S(O), or S(O)₂;
R^{d2} and R^{d3} are each independently selected from hydrogen, deuterium, halogen, amino, cyano, oxo, hydroxyl, C₁₋₃ alkyl, C₁₋₃ alkoxyl, C₁₋₃ haloalkyl, C₁₋₃ haloalkoxyl, C₃₋₆ cycloalkyl, or 3-6 membered heterocyclyl, the C₁₋₃ alkyl, C₁₋₃ alkoxyl, C₁₋₃ haloalkyl, C₁₋₃ haloalkoxyl, C₃₋₆ cycloalkyl, and 3-6 membered heterocyclyl being optionally further substituted with one or more substituents selected from deuterium, hydroxyl, cyano, amino, oxo, fluoro, or chloro;
p2 and p3 are each independently selected from 1, 2, 3, or 4; and
n9 is selected from 1, 2, or 3.

In a preferred embodiment of the present application, 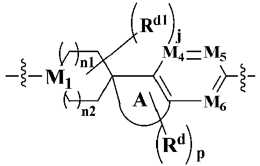 in general formula (II-B) is selected from 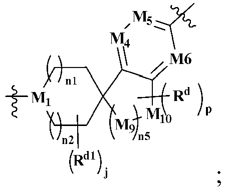
preferably, the 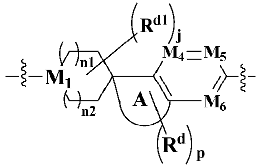 is selected from 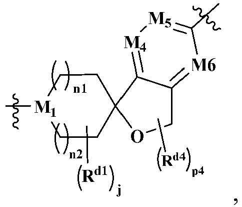 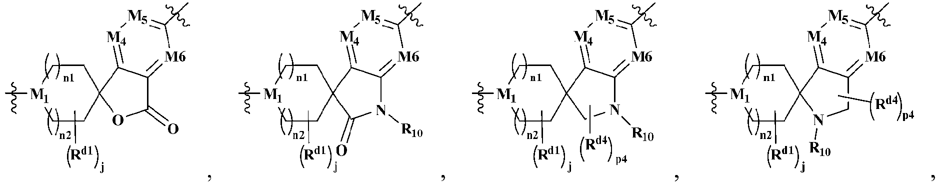 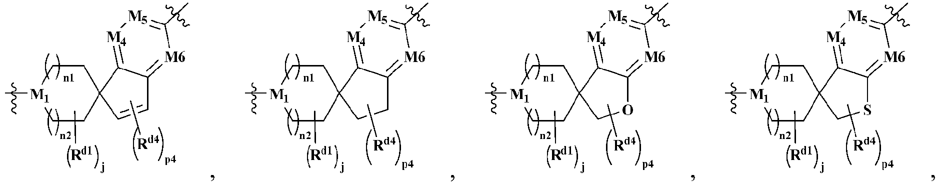 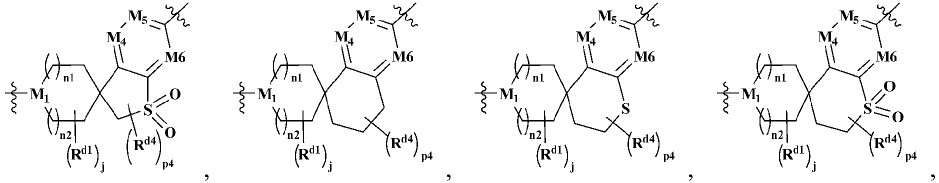 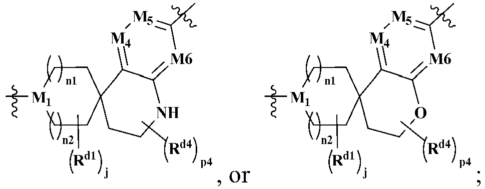
M₉ and M₁₀ are each independently selected from CH₂, C(O), NR₁₀, CH, O, S, S(O), or S(O)₂;
R₁₀ is selected from hydrogen, deuterium, halogen, C₁₋₃ alkyl, C₁₋₃ alkoxyl, C₁₋₃ haloalkyl, C₁₋₃ haloalkoxyl, C₃₋₆ cycloalkyl, or 3-6 membered heterocyclyl, the C₁₋₃ alkyl, C₁₋₃ alkoxyl, C₁₋₃ haloalkyl, C₁₋₃ haloalkoxyl, C₃₋₆ cycloalkyl, and 3-6 membered heterocyclyl being optionally further substituted with one or more substituents selected from deuterium, hydroxyl, cyano, amino, oxo, fluoro, or chloro;
R^{d4} is each independently selected from hydrogen, deuterium, halogen, amino, cyano, oxo, C₁₋₃ alkyl, C₁₋₃ alkoxyl, C₁₋₃ haloalkyl, C₁₋₃ haloalkoxyl, C₃₋₆ cycloalkyl, or 3-6 membered heterocyclyl, the C₁₋₃ alkyl, C₁₋₃ alkoxyl, C₁₋₃ haloalkyl, C₁₋₃ haloalkoxyl, C₃₋₆ cycloalkyl, and 3-6 membered heterocyclyl being optionally further substituted with one or more substituents selected from deuterium, hydroxyl, cyano, amino, oxo, fluoro, or chloro;
p4 is each independently selected from 1, 2, 3, or 4; and
n5 is selected from 1, 2, or 3.

In a preferred embodiment of the present application, the aforementioned ring B₁ is selected from indazolyl, pyrazolyl, benzimidazolyl, pyridinotriazolyl, pyridinopyrazolyl, pyridinoimidazolyl, or pyrimidoimidazolyl;
preferably, the 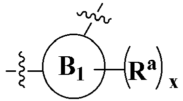 is selected from 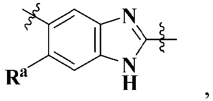 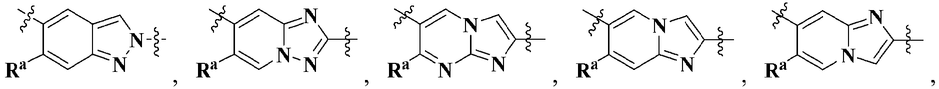 or 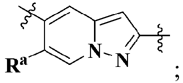
the ring B₂ is selected from pyridyl, phenyl, pyridonyl, pyridazinonyl, pyrimidopyrazolyl, pyridinopyrrolyl, pyrimidinyl, pyrimidopyrrolyl, or pyridinopyrazolyl;
preferably, the 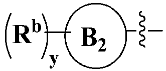 is selected from 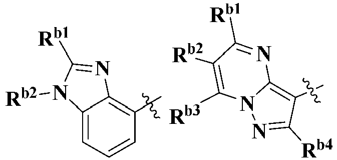 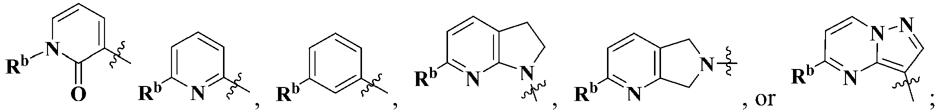
R^{b1}, R^{b2}, R^{b3}, and R^{b4} are each independently selected from hydrogen, deuterium, halogen, cyano, hydroxyl, C₁₋₆ alkyl, C₁₋₆ alkoxyl, C₁₋₆ haloalkyl, C₁₋₆ hydroxyalkyl, C₁₋₆ haloalkoxyl, C₃₋₆ cycloalkyl, or 3-6 membered heterocyclyl;
or, R^{b1} and R^{b2} are linked to form 5-6 membered heterocyclyl, the 5-6 membered heterocyclyl being optionally further substituted with one or more substituents selected from deuterium, halogen, cyano, hydroxyl, C₁₋₃ alkyl, C₁₋₃ alkoxyl, C₁₋₃ haloalkyl, C₁₋₃ haloalkoxyl, C₃₋₆ cycloalkyl, or 3-6 membered heterocyclyl.

In a preferred embodiment of the present application, the aforementioned R^{a} is each independently selected from hydrogen, deuterium, halogen, cyano, C₁₋₆ alkyl, C₁₋₆ alkoxyl, hydroxy C₁₋₆ alkyl, C₁₋₆ haloalkyl, C₁₋₆ haloalkoxyl, -P(O)RR', C₃₋₆ cycloalkyl, or 3-6 membered heterocyclyl, the C₁₋₆ alkyl, C₁₋₆ alkoxyl, hydroxy C₁₋₆ alkyl, C₁₋₆ haloalkyl, C₁₋₆ haloalkoxyl, C₃₋₆ cycloalkyl, and 3-6 membered heterocyclyl being optionally further substituted with one or more substituents selected from deuterium, halogen, hydroxyl, cyano, or C₁₋₃ alkyl;
preferably, R^{a} is each independently selected from hydrogen, deuterium, halogen, cyano, C₁₋₃ alkyl, C₁₋₃ alkoxyl, hydroxy C₁₋₃ alkyl, C₁₋₃ haloalkyl, C₁₋₃ haloalkoxyl, -P(O)RR', C₃₋₆ cycloalkyl, or 3-6 membered heterocyclyl, the C₁₋₃ alkyl, C₁₋₃ alkoxyl, hydroxy C₁₋₃ alkyl, C₁₋₃ haloalkyl, C₁₋₃ haloalkoxyl, C₃₋₆ cycloalkyl, and 3-6 membered heterocyclyl being optionally further substituted with one or more substituents selected from deuterium, halogen, hydroxyl, cyano, or C₁₋₃ alkyl;
or, the R^{b}, R^{c}, R^{e}, and R^{f} are each independently selected from hydrogen, deuterium, halogen, cyano, oxo, C₁₋₆ alkyl, C₁₋₆ alkoxyl, hydroxy C₁₋₆ alkyl, C₁₋₆ haloalkyl, C₁₋₆ haloalkoxyl, C₃₋₆ cycloalkyl, or 3-6 membered heterocyclyl, the C₁₋₆ alkyl, C₁₋₆ alkoxyl, hydroxy C₁₋₆ alkyl, C₁₋₆ haloalkyl, C₁₋₆ haloalkoxyl, C₃₋₆ cycloalkyl, and 3-6 membered heterocyclyl being optionally further substituted with one or more substituents selected from deuterium, halogen, hydroxyl, C₁₋₃ alkyl, C₁₋₃ alkoxyl, hydroxy C₁₋₃ alkyl, C₁₋₃ haloalkyl, or C₁₋₃ haloalkoxyl;
preferably, the R^{b}, R^{c}, R^{e}, and R^{f} are each independently selected from hydrogen, deuterium, fluoro, chloro, bromo, cyano, oxo, C₁₋₃ alkyl, C₁₋₃ alkoxyl, hydroxy C₁₋₃ alkyl, C₁₋₃ haloalkyl, C₁₋₃ haloalkoxyl, C₃₋₆ cycloalkyl, or 3-6 membered heterocyclyl, the C₁₋₃ alkyl, C₁₋₃ alkoxyl, hydroxy C₁₋₃ alkyl, C₁₋₃ haloalkyl, C₁₋₃ haloalkoxyl, C₃₋₆ cycloalkyl, and 3-6 membered heterocyclyl being optionally further substituted with one or more substituents selected from deuterium, fluoro, chloro, hydroxyl, C₁₋₃ alkyl, C₁₋₃ alkoxyl, hydroxy C₁₋₃ alkyl, C₁₋₃ haloalkyl, or C₁₋₃ haloalkoxyl;
or, R₁, R₂, R₃, R₄, R₅, and R₆ are each independently selected from hydrogen, deuterium, halogen, cyano, oxo, C₁₋₆ alkyl, C₁₋₆ alkoxyl, C₁₋₆ hydroxyalkyl, C₁₋₆ haloalkyl, C₁₋₆ haloalkoxyl, C₃₋₆ cycloalkyl, or 3-6 membered heterocyclyl;
preferably, R₁, R₂, R₃, R₄, R₅, and R₆ are each independently selected from hydrogen, deuterium, fluoro, chloro, cyano, oxo, C₁₋₃ alkyl, C₁₋₃ alkoxyl, C₁₋₃ hydroxyalkyl, C₁₋₃ haloalkyl, C₁₋₃ haloalkoxyl, C₃₋₆ cycloalkyl, or 3-6 membered heterocyclyl;
or, R and R' are each independently selected from hydrogen, deuterium, halogen, C₁₋₆ alkyl, C₁₋₆ alkoxyl, C₁₋₆ haloalkyl, or C₁₋₆ haloalkoxyl; and
preferably, R and R' are each independently selected from hydrogen, deuterium, fluoro, chloro, C₁₋₃ alkyl, C₁₋₃ alkoxyl, C₁₋₃ haloalkyl, or C₁₋₃ haloalkoxyl.

In a preferred embodiment of the present application, the aforementioned L₂ is -Ak1-Cyl-Ak2-Cy2-Ak3-,
the Cy1 and Cy2 are each independently selected from a bond, cycloalkylene, heterocyclylene, arylene, or heteroarylene, the cycloalkylene, heterocyclylene, arylene, and heteroarylene being optionally further substituted with 0 to 4 substituents selected from deuterium, halogen, amino, hydroxyl, cyano, nitro, alkyl, haloalkyl, deuterated alkyl, alkoxyl, hydroxyalkyl, haloalkoxyl, or deuterated alkoxyl;
preferably, the Cy1 and Cy2 are each independently selected from a bond, 5-6 membered monocyclic cycloalkylene, 4-6 membered monocyclic heterocycloalkylene, phenylene, 5-6 membered heteroarylene, 6-12 membered bicyclic cycloalkylene, 6-12 membered bicyclic heterocyclylene, 7-12 membered spirocycloalkylene, or 7-12 membered spiroheterocyclylene, the 5-6 membered monocyclic cycloalkylene, 4-6 membered monocyclic heterocycloalkylene, phenylene, 5-6 membered heteroarylene, 6-12 membered bicyclic cycloalkylene, 6-12 membered bicyclic heterocyclylene, 7-12 membered spirocycloalkylene, and 7-12 membered spiroheterocyclylene being optionally further substituted with 0 to 4 substituents selected from deuterium, fluoro, amino, hydroxyl, cyano, nitro, methyl, ethyl, methoxyl, trifluoromethyl, or difluoromethyl;
further preferably, Cy1 and Cy2 are each independently selected from a bond, cyclohexylene, piperidinylene, phenylene, pyridylene, pyrazolylene, piperazinylene, morpholinylene, 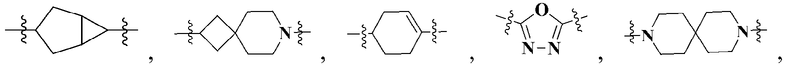 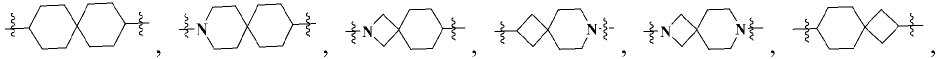 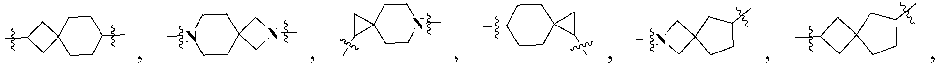 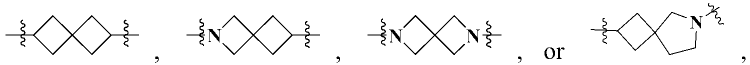 the cyclohexylene, piperidinylene, phenylene, pyridylene, pyrazolylene, piperazinylene, morpholinylene, 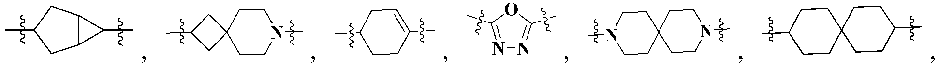 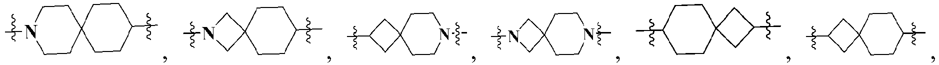 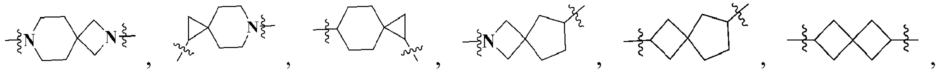 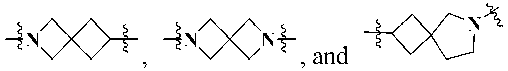 being optionally further substituted with 0 to 4 substituents selected from deuterium, fluoro, amino, hydroxyl, cyano, nitro, methyl, ethyl, methoxyl, ethoxyl, propoxyl, trifluoromethyl, hydroxymethyl, hydroxyethyl, hydroxypropyl, or difluoromethyl.

In a preferred embodiment of the present application, the aforementioned L₂ is -Ak1-Cyl-Ak2-Cy2-Ak3-,
the Ak1, Ak2, and Ak3 are each independently selected from -(CH₂)ₙ₃-, -O-, -C(O)-, - NH-, -NR₇-, -CH₂NR₇-, -(CR₈R₉)ₙ₄-, or a bond;
the Cy1 and Cy2 are each independently selected from a bond, cyclohexylene, piperidinylene, or piperazinylene, the cyclohexylene, piperidinylene, and piperazinylene being optionally further substituted with 1 to 4 substituents selected from deuterium, halogen, hydroxyl, cyano, oxo, C₁₋₃ alkyl, hydroxy C₁₋₃ alkyl, C₁₋₃ alkoxyl, C₃₋₄ cycloalkyl, 3-4 membered heterocyclyl, C₁₋₃ haloalkyl, or C₁₋₃ haloalkoxyl;
the R₇, R₈, and R₉ are each independently selected from hydrogen, deuterium, halogen, C₁₋₃ alkyl, cyano, hydroxyl, C₃₋₄ cycloalkyl, C₁₋₃ haloalkyl, C₁₋₃ deuterated alkyl, C₁₋₃ halocycloalkyl, hydroxy C₁₋₃ alkyl, or C₁₋₃ alkoxyl;
preferably, L₂ is selected from 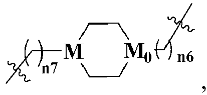 M and M₀ being each independently selected from CR₁₁ or N; R₁₁ is selected from hydrogen, deuterium, halogen, hydroxyl, C₁₋₃ alkyl, C₁₋₃ hydroxyalkyl, C₁₋₃ haloalkyl, or C₁₋₃ haloalkoxyl; and
n6 and n7 are each independently selected from 0, 1, 2, 3, or 4; preferably, n6 is selected from 2, and n7 is selected from 0.

In a preferred embodiment of the present application, the aforementioned compound of general formula (II-A) is further shown in general formula (IV-0): 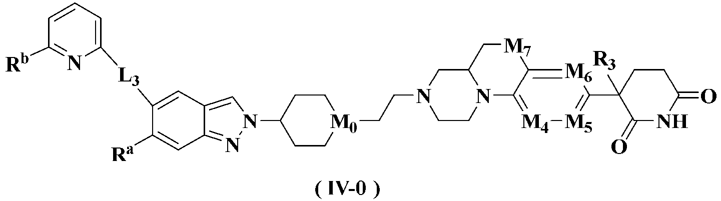 where R^{a}, R^{b}, L₃, R₃, M₀, M₄, M₅, M₆, and M₇ are as defined in the preceding text of the present application.

In a preferred embodiment of the present application, the aforementioned compound of general formula (II-A) is further shown in general formula (IV-1): 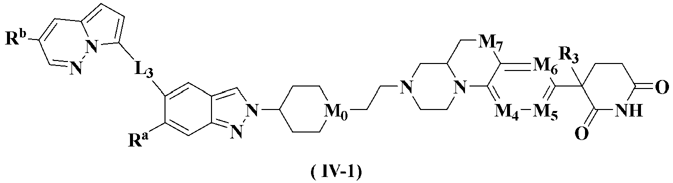 where R^{a}, R^{b}, L₃, R₃, M₀, M₄, M₅, M₆, and M₇ are as defined in the preceding text of the present application.

In a preferred embodiment of the present application, the aforementioned compound of general formula (II-A) is further shown in general formula (IV-2): 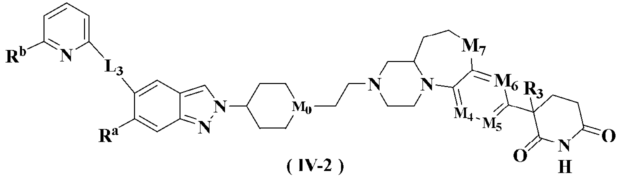 where R^{a}, R^{b}, L₃, R₃, M₀, M₄, M₅, M₆, and M₇ are as defined in the preceding text of the present application.

In a preferred embodiment of the present application, the aforementioned compound of general formula (II-A) is further shown in general formula (IV): 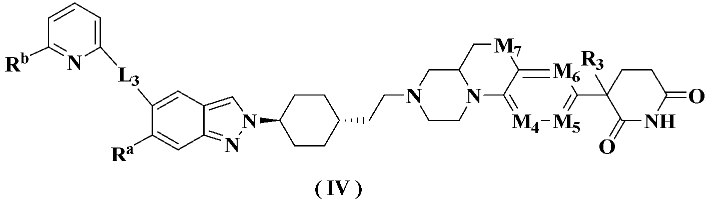 where R^{a}, R^{b}, L₃, R₃, M₄, M₅, M₆, and M₇ are as defined in the preceding text of the present application.

In a preferred embodiment of the present application, the aforementioned compound of general formula (II-A) is further shown in general formula (IV-A) or general formula (IV-B): 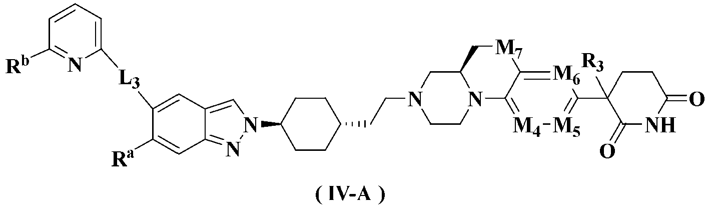 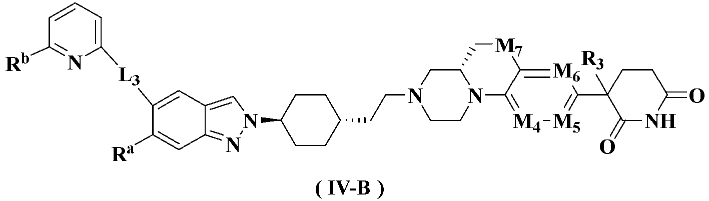 where R^{a}, R^{b}, L₃, R₃, M₄, M₅, M₆, and M₇ are as defined in the preceding text of the present application.

In a preferred embodiment of the present application, the aforementioned compound of general formula (II-A) is further shown in general formula (IV-3): 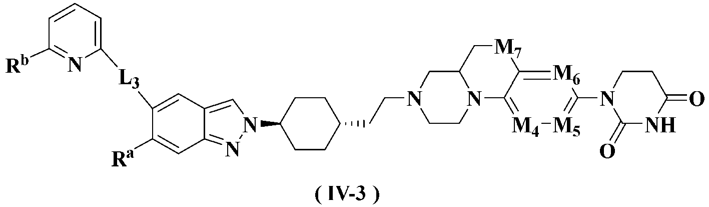 where R^{a}, R^{b}, L₃, M₄, M₅, M₆, and M₇ are as defined in the preceding text of the present application.

In a preferred embodiment of the present application, the aforementioned compound of general formula (II-B) is further shown in general formula (V): 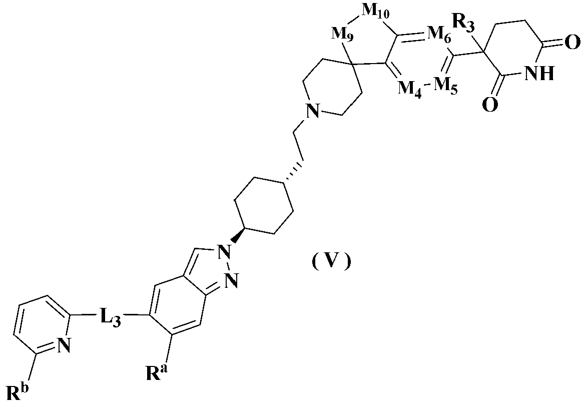 where R^{a}, R^{b}, L₃, R₃, M₄, M₅, M₆, M₉, and M₁₀ are as defined in the preceding text of the present application.

In a preferred embodiment of the present application, the aforementioned R^{a} and R^{b} are each independently selected from hydrogen, deuterium, fluoro, chloro, methyl, ethyl, propyl, methoxyl, ethoxyl, propoxyl, difluoromethyl, trifluoromethyl, trifluoromethoxyl, isopropylmethyl, morpholinyl, cyclopropyl, cyclobutyl,

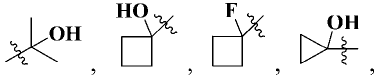

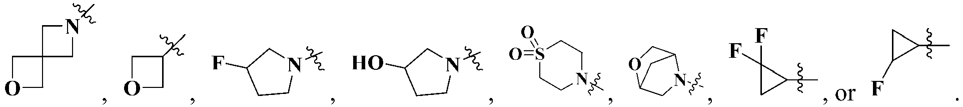

In a preferred embodiment of the present application, the aforementioned R^{c} is each independently selected from hydrogen, deuterium, fluoro, chloro, methyl, ethyl, methoxyl, ethoxyl, trifluoromethyl, difluoromethyl, trifluoromethoxyl, methoxyl, cyclopropyl, or cyclobutyl.

In a preferred embodiment of the present application, the aforementioned R^{d}, R^{d1}, R^{d2}, R^{d3}, and R^{d4} are each independently selected from hydrogen, deuterium, fluoro, chloro, methyl, ethyl, methoxyl, ethoxyl, trifluoromethyl, difluoromethyl, trifluoromethoxyl, methoxyl, cyclopropyl, or cyclobutyl.

In a preferred embodiment of the present application, the aforementioned R^{a} is selected from C₁₋₃ alkyl, C₁₋₃ alkoxyl, hydroxy C₁₋₃ alkyl, C₁₋₃ haloalkyl, or C₁₋₃ haloalkoxyl; preferably C₁₋₃ alkoxyl or hydroxy C₁₋₃ alkyl; more preferably methoxyl, ethoxyl, propoxyl, hydroxymethyl, hydroxyethyl, or hydroxypropyl.

In a preferred embodiment of the present application, the aforementioned R^{b} is selected from C₁₋₃ alkyl, C₁₋₃ alkoxyl, hydroxy C₁₋₃ alkyl, C₁₋₃ haloalkyl, or C₁₋₃ haloalkoxyl; preferably C₁₋₃ haloalkyl or C₁₋₃ haloalkoxyl; more preferably difluoromethyl, trifluoromethyl, or trifluoromethoxyl.

In a preferred embodiment of the present application, the aforementioned L₃ is selected from a bond, -NH-C(O)-, or -C(O)-NH-.

In a preferred embodiment of the present application, the aforementioned R₃ is selected from hydrogen, deuterium, fluoro, chloro, cyano, C₁₋₃ alkyl, C₁₋₃ alkoxyl, C₁₋₃ hydroxyalkyl, C₁₋₃ haloalkyl, or C₁₋₃ haloalkoxyl; preferably hydrogen, deuterium, fluoro, chloro, C₁₋₃ alkyl, C₁₋₃ alkoxyl, or C₁₋₃ haloalkyl; more preferably hydrogen, deuterium, fluoro, methyl, ethyl, propyl, trifluoromethyl, methoxyl, ethoxyl, or propoxyl.

In a preferred embodiment of the present application, the aforementioned M₀ is selected from N or CH.

In a preferred embodiment of the present application, the aforementioned M₄ is N or CR₄, R₄ being selected from hydrogen, deuterium, fluoro, chloro, C₁₋₃ alkyl, C₁₋₃ alkoxyl, C₁₋₃ hydroxyalkyl, C₁₋₃ haloalkyl, or C₁₋₃ haloalkoxyl, preferably hydrogen, deuterium, fluoro, chloro, methyl, ethyl, methoxyl, ethoxyl, hydroxymethyl, or trifluoromethyl.

In a preferred embodiment of the present application, the aforementioned M₅ is N or CR₅, R₅ being selected from hydrogen, deuterium, fluoro, chloro, C₁₋₃ alkyl, C₁₋₃ alkoxyl, C₁₋₃ hydroxyalkyl, C₁₋₃ haloalkyl, or C₁₋₃ haloalkoxyl, preferably hydrogen, deuterium, fluoro, chloro, methyl, ethyl, methoxyl, ethoxyl, hydroxymethyl, or trifluoromethyl.

In a preferred embodiment of the present application, the aforementioned M₆ is N or CR₆, R₆ being selected from hydrogen, deuterium, fluoro, chloro, C₁₋₃ alkyl, C₁₋₃ alkoxyl, C₁₋₃ hydroxyalkyl, C₁₋₃ haloalkyl, or C₁₋₃ haloalkoxyl, preferably hydrogen, deuterium, fluoro, chloro, methyl, ethyl, methoxyl, ethoxyl, hydroxymethyl, or trifluoromethyl.

In a preferred embodiment of the present application, the aforementioned M₇ is O, CH₂, C(O), S, S(O), or S(O)₂; preferably O, CH₂, or S.

In a preferred embodiment of the present application, the aforementioned M₉ is CH₂, C(O), O, or S.

In a preferred embodiment of the present application, the aforementioned M₁₀ is CH₂, C(O), NR₁₀, O, or S, R₁₀ being selected from hydrogen, deuterium, C₁₋₃ alkyl, C₁₋₃ alkoxyl, C₁₋₃ haloalkyl, or C₁₋₃ haloalkoxyl; preferably hydrogen, deuterium, methyl, ethyl, propyl, methoxyl, ethoxyl, hydroxymethyl, or trifluoromethyl.

In a preferred embodiment of the present application, the compound as described in the present application is selected from compound 1, compound 46, compound 120, compound 121, compound 126, compound 128, compound 129, compound 130, compound 131, compound 120-a, compound 120-b, compound 120-a-1, compound 120-a-2, compound 120-b-1, or compound 120-b-2.

In a preferred embodiment of the present application, the aforementioned compound is selected from the compounds in Table 5 below.

**Table 5**

| | |
|---|---|
| 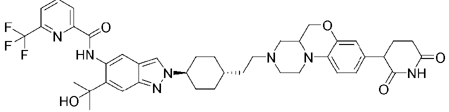 | 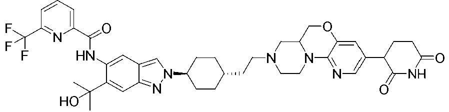 |
| 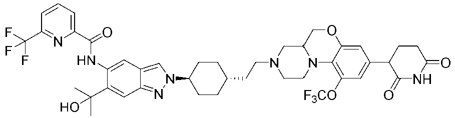 | 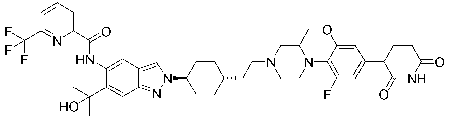 |
| 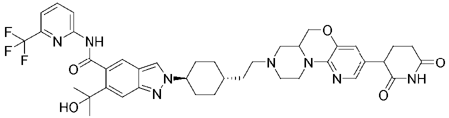 | 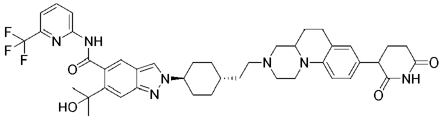 |
| 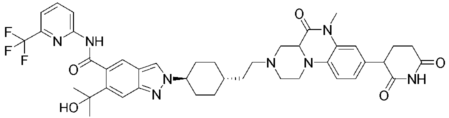 | 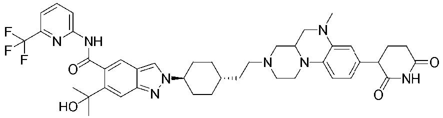 |
| 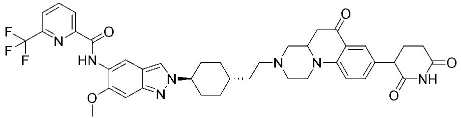 | 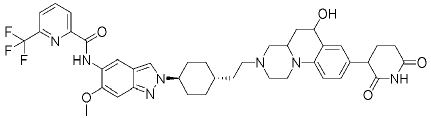 |
| 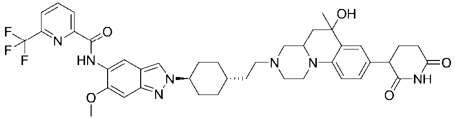 | 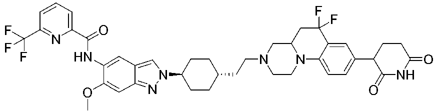 |
| 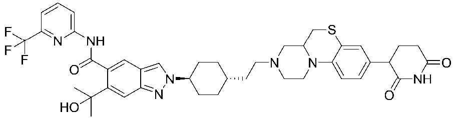 | 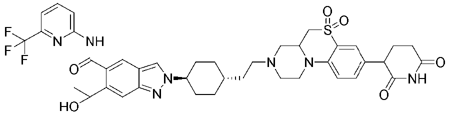 |
| 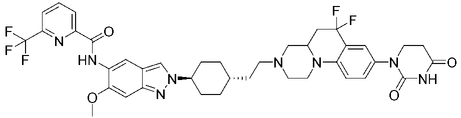 | 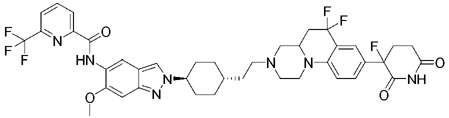 |
| 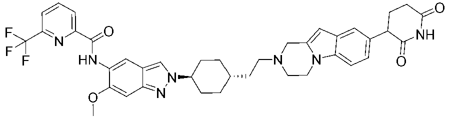 | 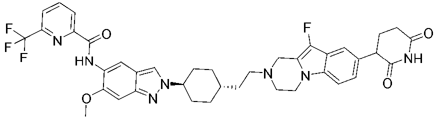 |
| 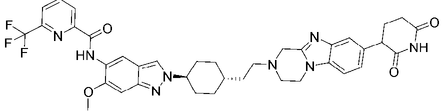 | 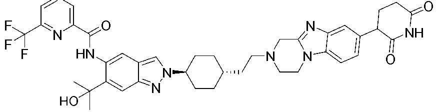 |
| 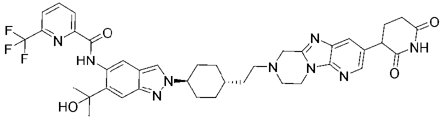 | 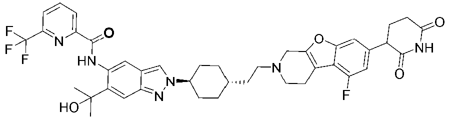 |
| 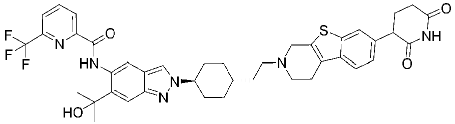 | 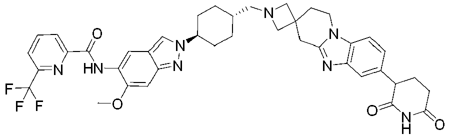 |
| 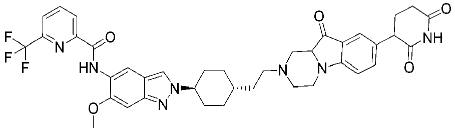 | 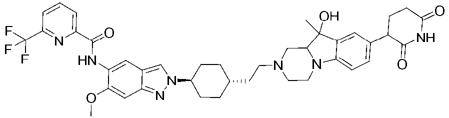 |
| 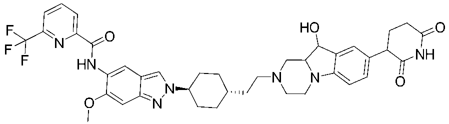 | 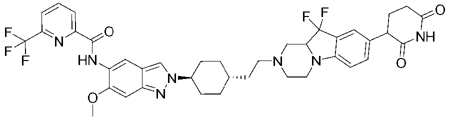 |
| 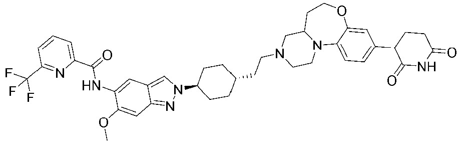 | 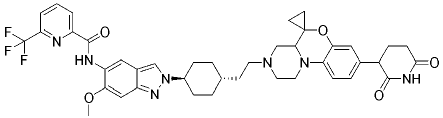 |
| 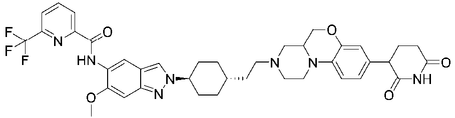 | 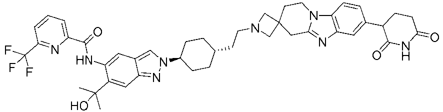 |
| 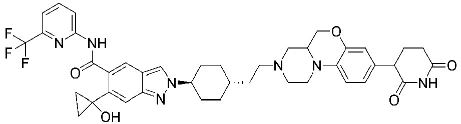 | 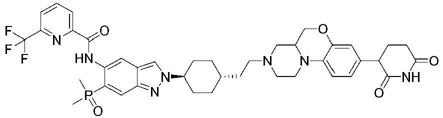 |
| 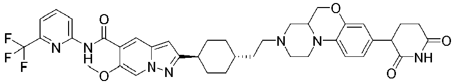 | 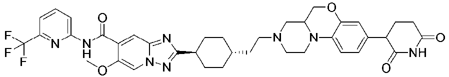 |
| 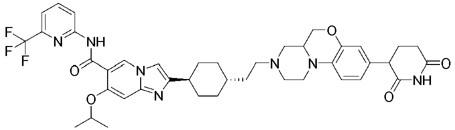 | 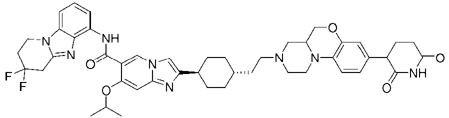 |
| 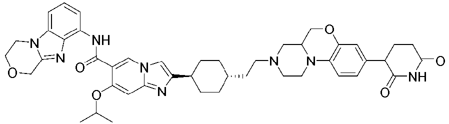 | 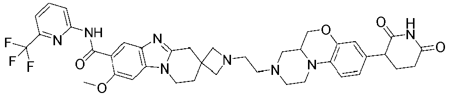 |
| 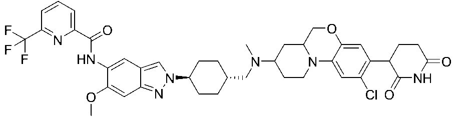 | 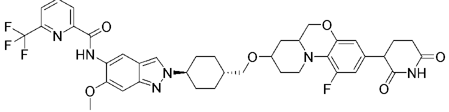 |
| 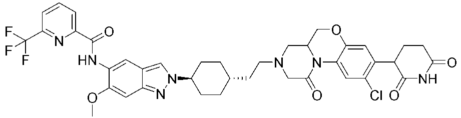 | 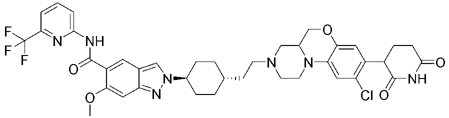 |
| 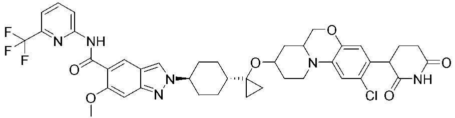 | 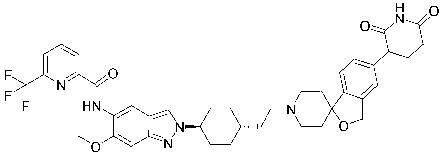 |
| 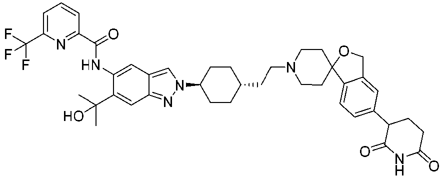 | 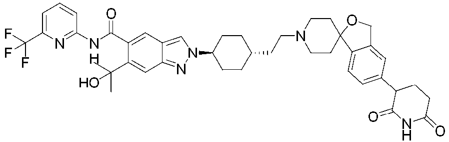 |
| 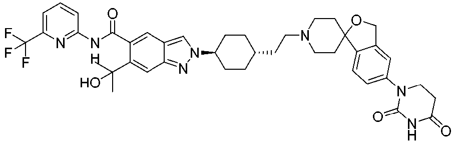 | 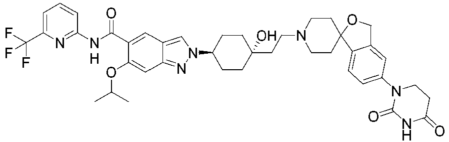 |
| 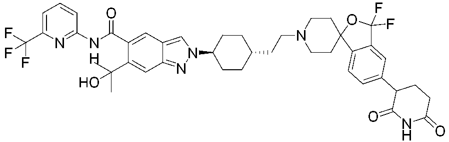 | 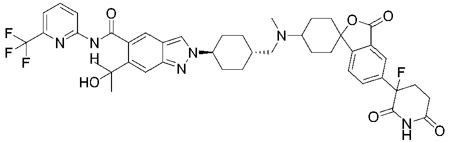 |
| 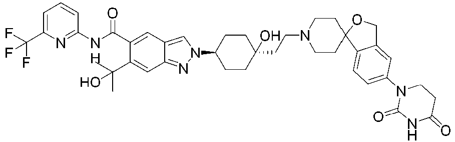 | 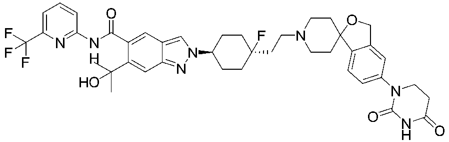 |
| 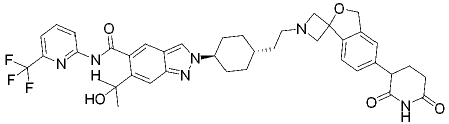 | 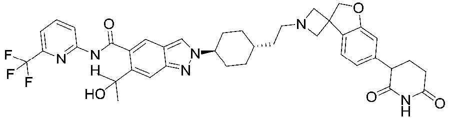 |
| 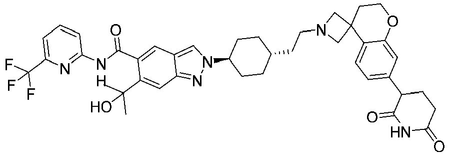 | 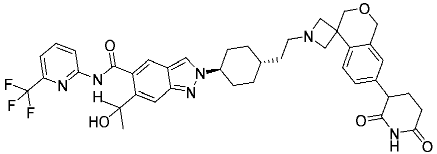 |
| 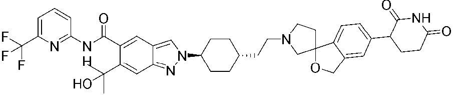 | 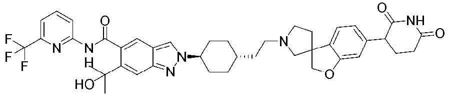 |
| 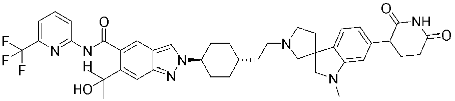 | 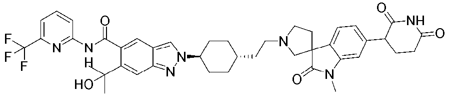 |
| 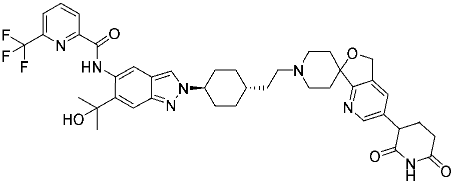 | 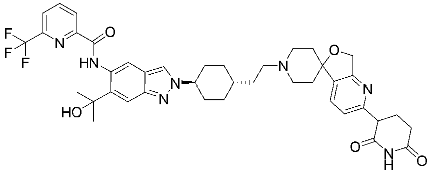 |
| 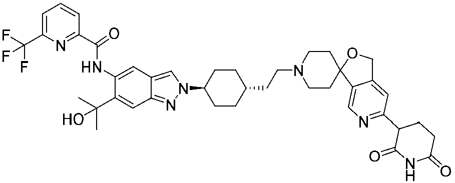 | 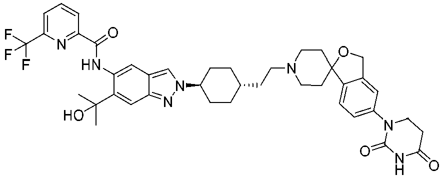 |
| 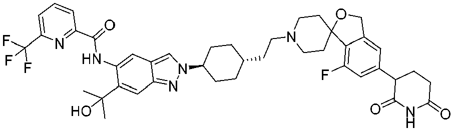 | 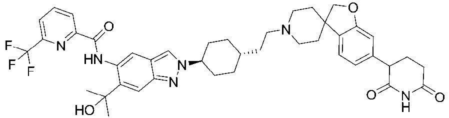 |
| 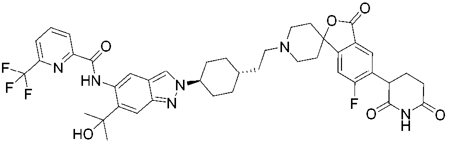 | 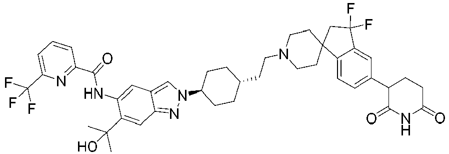 |
| 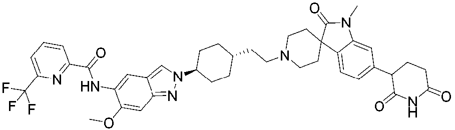 | 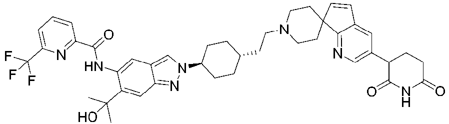 |
| 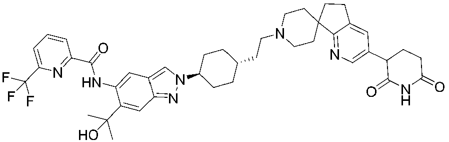 | 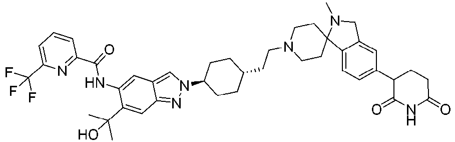 |
| 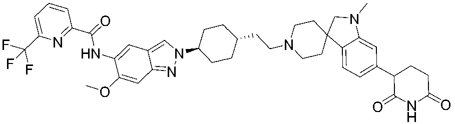 | 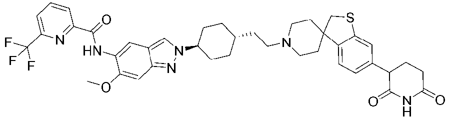 |
| 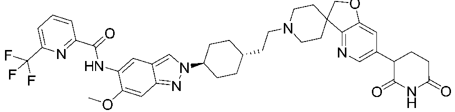 | 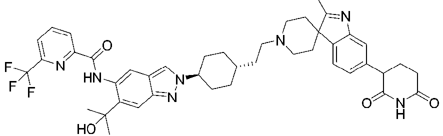 |
| 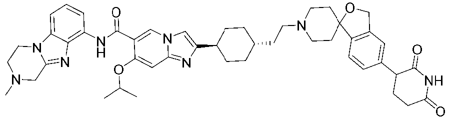 | 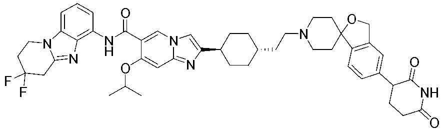 |
| 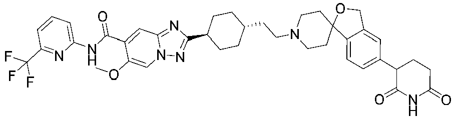 | 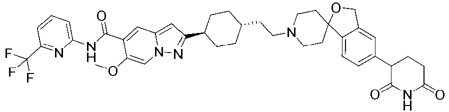 |
| 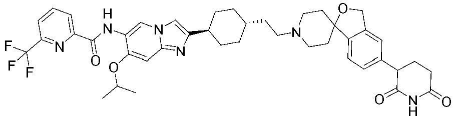 | 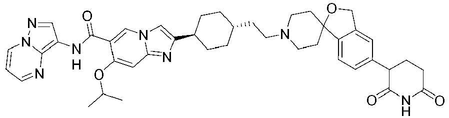 |
| 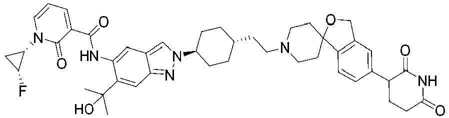 | 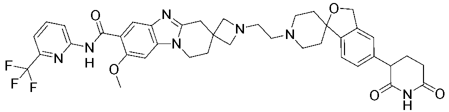 |
| 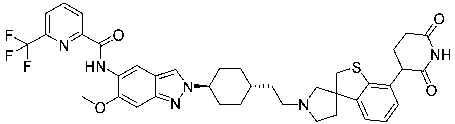 | 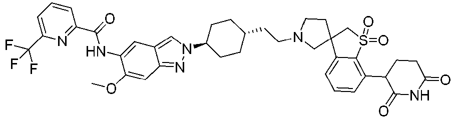 |
| 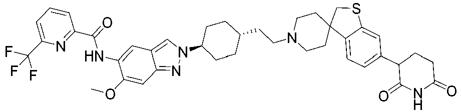 | 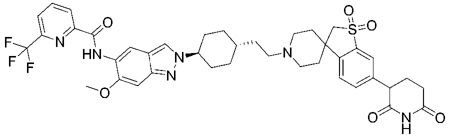 |
| 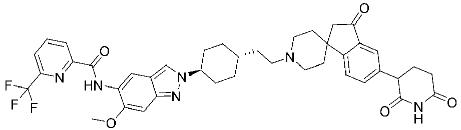 | 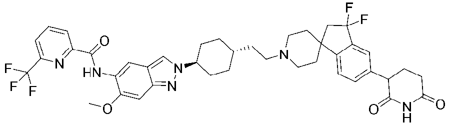 |
| 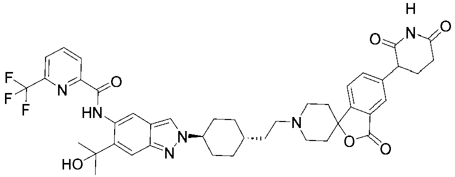 | 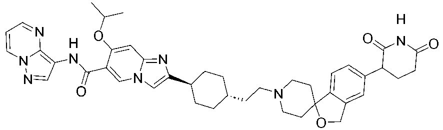 |
| 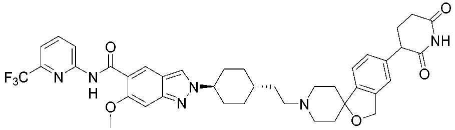 | 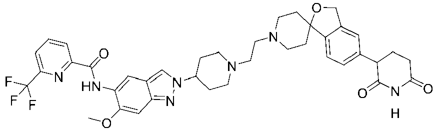 |
| 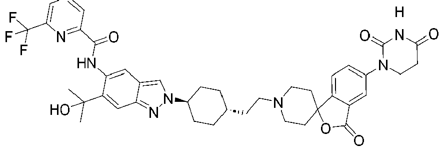 | 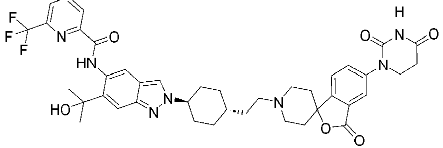 |
| 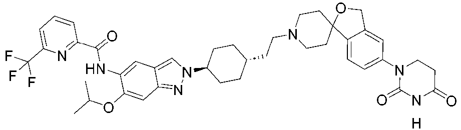 | 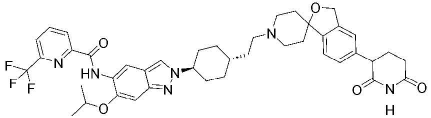 |
| 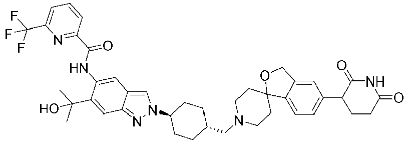 | 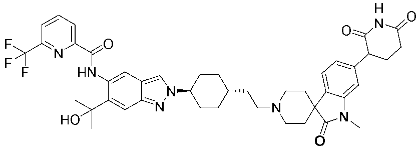 |
| 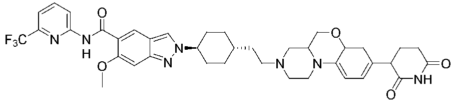 | 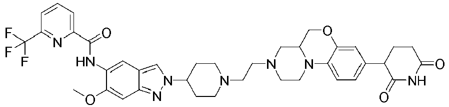 |
| 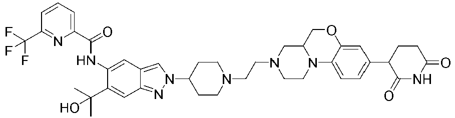 | 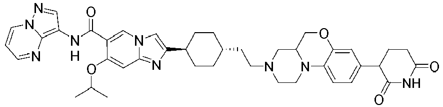 |
| 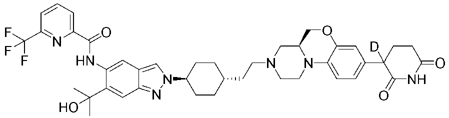 | 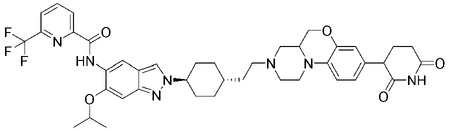 |
| 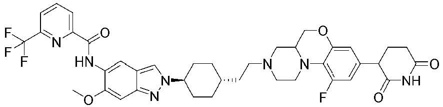 | 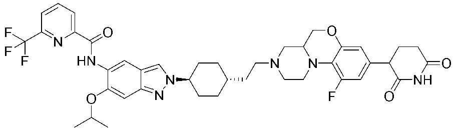 |
| 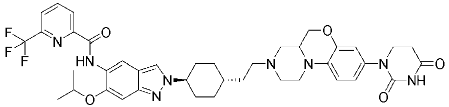 | 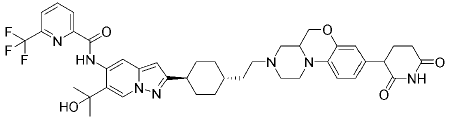 |
| 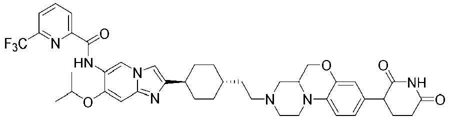 | 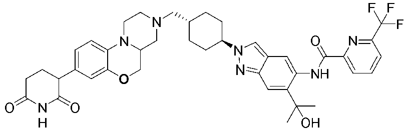 |
| 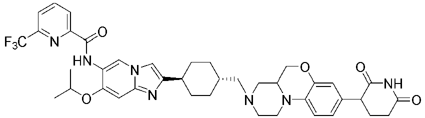 | 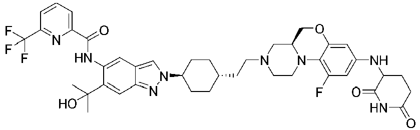 |
| 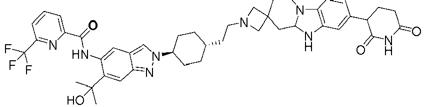 | 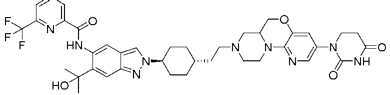 |
| 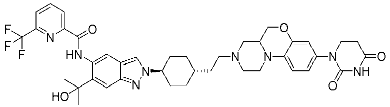 | 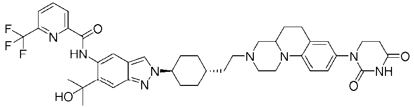 |
| 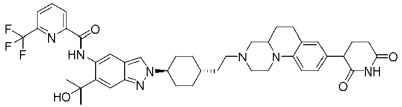 | 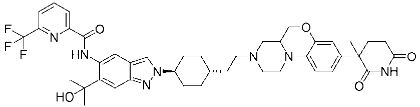 |
| 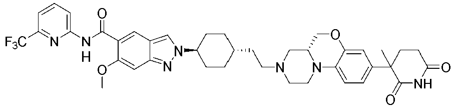 | 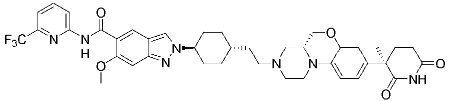 |
| 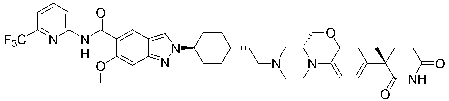 | 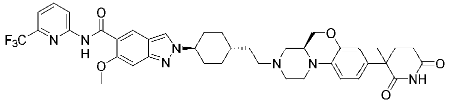 |
| 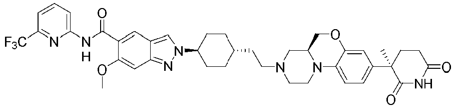 | 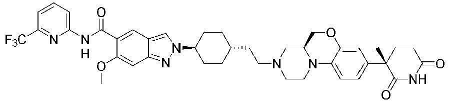 |
| 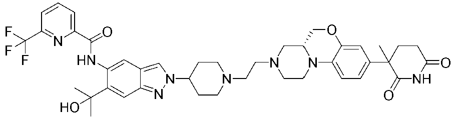 | 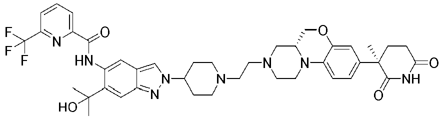 |
| 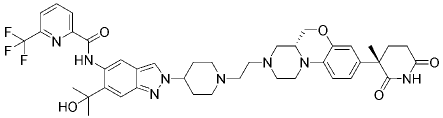 | 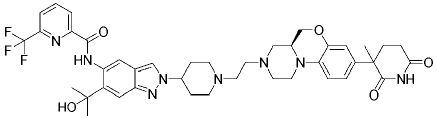 |
| 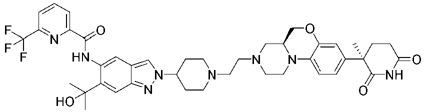 | 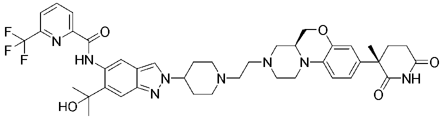 |
| 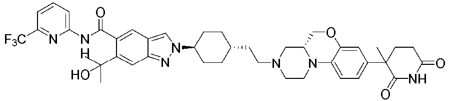 | 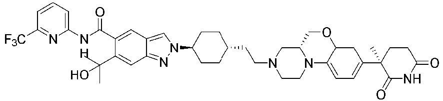 |
| 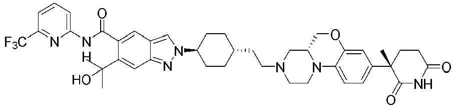 | 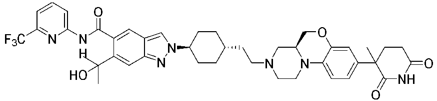 |
| 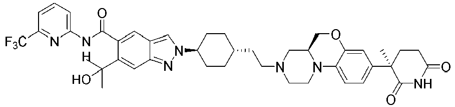 | 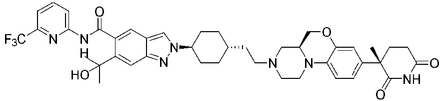 |
| 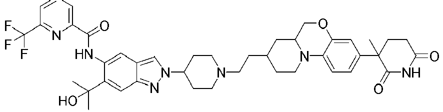 | 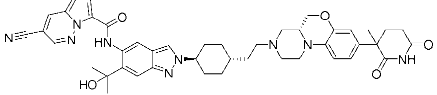 |
| 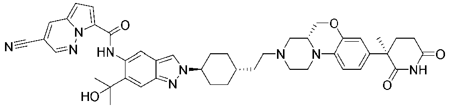 | 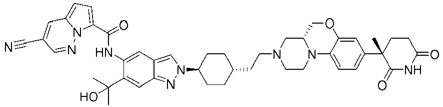 |
| 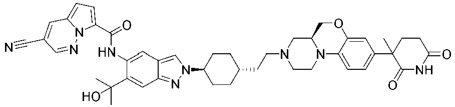 | 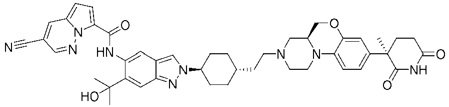 |
| 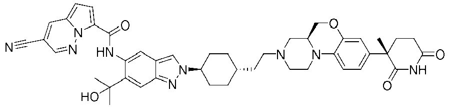 | 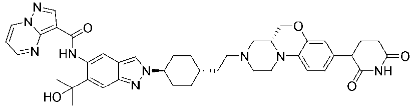 |
| 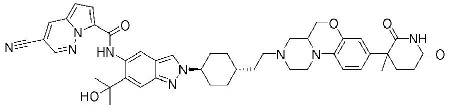 | 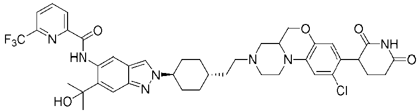 |
| 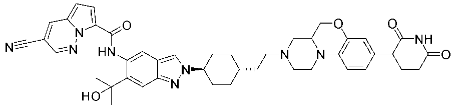 | 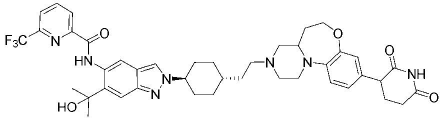 |
| 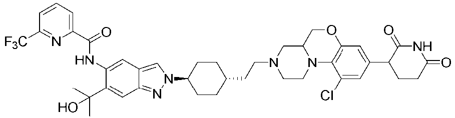 | |

The present application further provides a pharmaceutical composition, including a therapeutic effective dose of the compounds as shown in the aforementioned general formulas, stereoisomers thereof, or pharmaceutically acceptable salts thereof, and one or more pharmaceutically acceptable carriers.

The present application further provides a preferred embodiment, which relates to use of the compounds of the general formulas and stereoisomers or pharmaceutically acceptable salts thereof, or the aforementioned pharmaceutical composition, in the preparation of a drug for treating or preventing IRAK4 mediated diseases.

The present application further provides a preferred embodiment, which relates to use of the compounds of the general formulas and stereoisomers or pharmaceutically acceptable salts thereof, or the aforementioned pharmaceutical composition, in the preparation of a drug for treating or preventing autoimmune diseases, inflammatory diseases, cancers, viral diseases, neurodegenerative diseases, genetic disorders, hormone-related diseases, metabolic disorders, organ-transplantation-related diseases, immunodeficiency disorders, destructive bone diseases, proliferative disorders, infectious diseases, cell-death-related conditions, or cardiovascular diseases.

The present application further relates to a compound of formula (A-a), a stereoisomer thereof, or a pharmaceutically acceptable salt thereof, with the following specific structure: 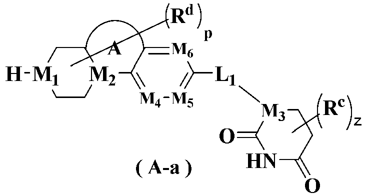 where M₁, M₂, M₃, M₄, M₅, M₆, 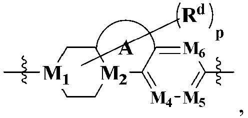 L₁, R^{c}, R^{d}, p, and z are as defined in the preceding text.

The present application further relates to a compound of formula (A-a-a), a stereoisomer thereof, or a pharmaceutically acceptable salt thereof, with the following specific structure: 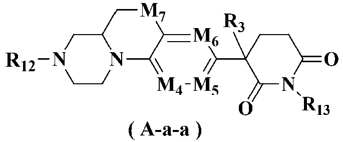 where M₄ is N or CR₄; M₅ is N or CR₅; M₆ is N or CR₆; M₇ is O, S, or CH₂; R₃ is selected from deuterium, halogen, C₁₋₃ alkyl, C₁₋₃ alkoxyl, or C₁₋₃ haloalkyl; R₄ is selected from hydrogen, deuterium, halogen, C₁₋₃ alkyl, C₃₋₅ cycloalkyl, C₁₋₃ alkoxyl, or C₁₋₃ haloalkyl; R₅ is selected from hydrogen, deuterium, fluoro, chloro, C₁₋₃ alkyl, C₁₋₃ alkoxyl, C₁₋₃ hydroxyalkyl, C₁₋₃ haloalkyl, or C₁₋₃ haloalkoxyl; R₆ is selected from hydrogen, deuterium, fluoro, chloro, C₁₋₃ alkyl, C₁₋₃ alkoxyl, C₁₋₃ hydroxyalkyl, C₁₋₃ haloalkyl, or C₁₋₃ haloalkoxyl; R₁₂ and R₁₃ are each independently selected from hydrogen or amino protecting groups.

In a preferred embodiment of the present application, the compound of formula (A-a-a) is further shown in formula (A-a-a-1) and formula (A-a-a-2):

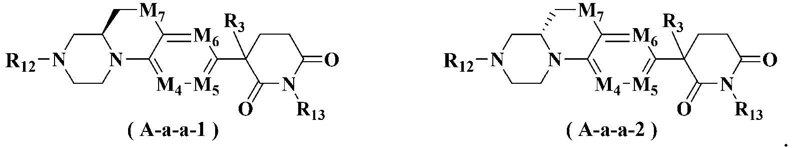

In a preferred embodiment of the present application, the R₃ as described in the present application is selected from deuterium, fluoro, chloro, methyl, ethyl, methoxyl, ethoxyl, or trifluoromethyl.

In a preferred embodiment of the present application, the R₄ as described in the present application is selected from hydrogen, deuterium, fluoro, chloro, methyl, ethyl, methoxyl, ethoxyl, or trifluoromethyl.

In a preferred embodiment of the present application, the R₅ as described in the present application is selected from hydrogen, deuterium, fluoro, chloro, methyl, ethyl, methoxyl, ethoxyl, hydroxymethyl, or trifluoromethyl.

In a preferred embodiment of the present application, the R₆ as described in the present application is selected from hydrogen, deuterium, fluoro, chloro, methyl, ethyl, methoxyl, ethoxyl, hydroxymethyl, or trifluoromethyl.

In a preferred embodiment of the present application, the R₁₂ and R₁₃ as described in the present application are each independently selected from hydrogen, SEM, tert-butoxycarbonyl, - CH₂-tert-butoxycarbonyl, benzyl, M-dimethoxybenzyl, -CH₂COOH, or -COCH₃.

The present application further relates to the following compounds: intermediate A, intermediate B, intermediate C, intermediate D, intermediate E, intermediate E-a, intermediate E-b, intermediate F, intermediate G, intermediate H, intermediate I, intermediate J, intermediate K, intermediate L, intermediate M, intermediate N, intermediate O, intermediate P, intermediate Q, intermediate R, intermediate S, intermediate T, intermediate U, intermediate V, intermediate W, intermediate Y-5, intermediate Y-6, intermediate Y-8, intermediate Y-9, and intermediate Y-10.

The present application further relates to a compound of formula (B-a), a stereoisomer thereof, or a pharmaceutically acceptable salt thereof, with the following specific structure: 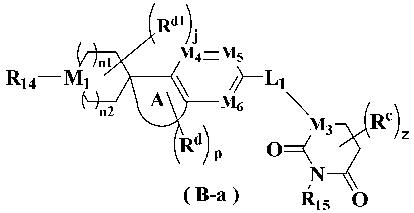 where
R₁₄ and R₁₅ are each independently selected from hydrogen or amino protecting groups; and M₁, M₃, M₄, M₅, M₆, 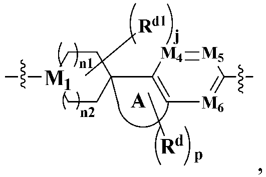 L₁, R^{c}, R^{d}, R^{d1}, p, z, j, n1, and n2 are as defined in the preceding text.

In a preferred embodiment of the present application, R₁₄ and R₁₅ are each independently selected from hydrogen, SEM, tert-butoxycarbonyl, -CH₂-tert-butoxycarbonyl, benzyl, para-methoxybenzyl, -CH₂COOH, or -COCH₃.

In the present application, the preferred amino protecting groups are SEM, tert-butoxycarbonyl, -CH₂-tert-butoxycarbonyl, -CH₂COOH, -COCH₃, ethoxycarbonyl, benzyl, para-methoxybenzyl, benzyloxycarbonyl, fluorenylmethoxycarbonyl, or allyloxycarbonyl.

The present application further relates to use of a structural unit of formula (A-a-1) or formula (B-a-1) as an E3 ubiquitin structural unit for a proteolysis inhibitor: 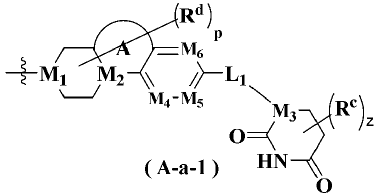 where M₁, M₂, M₃, M₄, M₅, M₆, 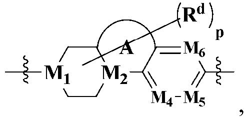 L₁, R^{c}, R^{d}, p, and z are as defined in the preceding text; or, 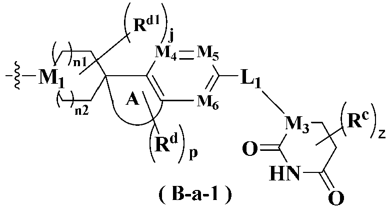 where
M₁, M₃, M₄, M₅, M₆, 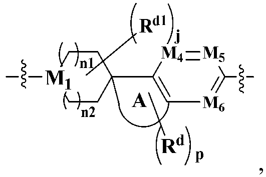 L₁, R^{c}, R^{d}, R^{d1}, p, z, j, n1, and n2 are as defined in the preceding text.

The present application further relates to a method for preparing a compound of general formula (IV-0), including: 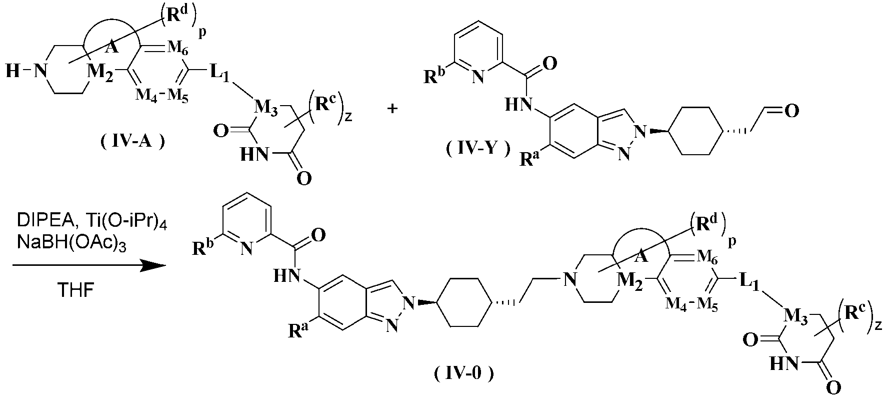
allowing a compound of formula IV-A and a compound of formula IV-Y to undergo reduction amination to yield the compound of formula IV-0;
where ring A, R^{a}, R^{b}, R^{c}, R^{d}, M₂, M₃, M₄, M₅, M₆, L₁, p, and z are as defined in the preceding text of the present application.

The present application further relates to a method for preparing a compound of general formula (V-0), including: 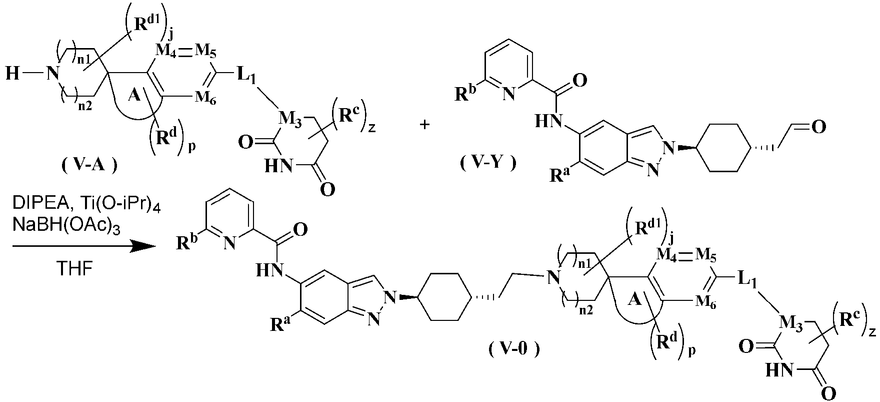
allowing a compound of formula V-A and a compound of formula V-Y to undergo reduction amination to yield the compound of formula V-0;
where ring A, R^{a}, R^{b}, R^{c}, R^{d}, R^{d1}, M₃, M₄, M₅, M₆, L₁, j, n1, n2, p, and z are as defined in the preceding text of the present application.

The present application further relates to a compound of general formula (III-A) or (III-B), a stereoisomer thereof, or a pharmaceutically acceptable salt thereof, with the following specific structure: 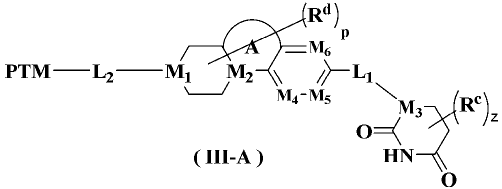 where
PTM is selected from drugs bound to targeted proteins or derivatives thereof;
L₂, M₁, M₂, M₃, M₄, M₅, M₆, 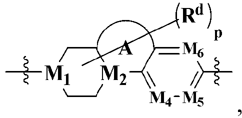 L₁, R^{c}, R^{d}, p, and z are as defined in the preceding text; or, 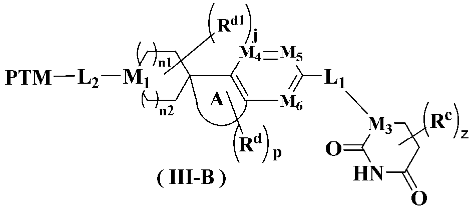
where
PTM is selected from drugs bound to targeted proteins or derivatives thereof;
L₂, M₁, M₃, M₄, M₅, M₆, 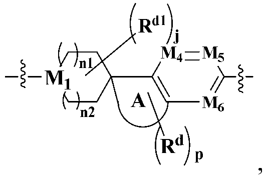 L₁, R^{c}, R^{d}, R^{d1} , p, z, j, n1, and n2 are as defined in the preceding text.

In a preferred embodiment of the present application, the aforementioned PTM is selected from drugs or derivatives that act on AR, ER, kinases, phosphatases, MDM2, proteins with BET Bromo domain in humans, Hsp90, HDAC, human lysine methyltransferases, RAF receptors, FKBP, vascular growth factors, immunosuppressive receptors or proteins, aromatic hydrocarbon receptors, thyroid hormone receptors, HIV proteases, HIV integrases, HCV proteases, HBV proteases, or acyl protein thioesterase 1, or/and acyl protein thioesterase 2; and preferably, the aforementioned PTM is selected from drugs or derivatives thereof that act on ALK, BET, CDK, PARP, EGFR, γ-secretase, CBF β-SMMHC, WEEI, MEK, BCR-ABL, MET, RAS, BTK, VEGFR, JAK, HER2, HDAC, Akt, PI3K, Mtor, AR, ER, PDE, SRC, MDM2, RAF, IRAK4, STAT3, and c-Myc.

The present application further relates to a method for treating or preventing IRAK4 mediated diseases, including applying an effective dose of the compound as described in the present application or a pharmaceutically acceptable salt, ester, prodrug, solvate, or hydrate thereof to mammals. The IRAK4 mediated diseases are selected from autoimmune diseases, inflammatory diseases, cancers, viral diseases, neurodegenerative diseases, genetic disorders, hormone-related diseases, metabolic disorders, organ-transplantation-related diseases, immunodeficiency disorders, destructive bone diseases, proliferative disorders, infectious diseases, cell-death-related conditions, or cardiovascular diseases.

### Detailed description of the application

Unless otherwise stated, the terms used in the specification and claims shall have the following meanings.

The term "alkyl" refers to a saturated aliphatic hydrocarbyl group, which is a linear or branched group containing 1 to 20 carbon atoms, preferably alkyl containing 1 to 8 carbon atoms, more preferably alkyl containing 1 to 6 carbon atoms, and most preferably alkyl containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, and various branched isomers thereof. The present application prefers methyl, ethyl, isopropyl, tert-butyl, haloalkyl, deuterated alkyl, alkoxy substituted alkyl, and hydroxyl substituted alkyl.

The term "alkylene" refers to further substitution of one hydrogen atom in alkyl, for example: "methylene" refers to - CH₂-, "ethylene" refers to -(CH₂)₂-, "propylene" refers to - (CH₂)₃-, and "butylene" refers to -(CH₂)₄-.

The term "alkenyl" refers to alkyl as defined above composed of at least two carbon atoms and at least one carbon-carbon double bond, for example, ethenyl, 1-propenyl, 2-propenyl, and 1-, 2-, or 3- butenyl. The alkenyl may be substituted or unsubstituted. When the alkenyl is substituted, a substituent is preferably one or more of the following groups, independently selected from alkyl, alkenyl, alkynyl, alkoxyl, alkylthio, alkylamino, halogen, sulfhydryl, hydroxyl, nitro, cyano, cycloalkyl, heterocycloalkyl, aryl, heteroaryl, cycloalkoxyl, heterocycloalkoxyl, cycloalkylthio, and heterocycloalkylthio.

The term "alkenylene" refers to further substitution of one hydrogen atom in alkenyl, for example, "ethenylene" refers to -(CH)₂-.

The term "cycloalkyl" refers to a saturated or partially unsaturated monocyclic or polycyclic hydrocarbon substituent, where a cycloalkyl ring contains 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, and cyclooctyl; and polycyclic cycloalkyl includes spirocyclic, fused cyclic, and endocyclic cycloalkyl, with non-limiting examples including

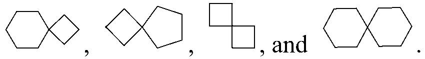

The cycloalkyl ring may be fused to an aryl, heteroaryl, or heterocycloalkyl ring. The ring connected to a parent structure is cycloalkyl, with non-limiting examples including indanyl, tetrahydronaphthyl, and benzocycloheptyl. The cycloalkyl may be optionally substituted or unsubstituted. When the cycloalkyl is substituted, a substituent is preferably one or more of the following groups, independently selected from alkyl, alkenyl, alkynyl, alkoxyl, alkylthio, alkylamino, halogen, sulfhydryl, hydroxyl, nitro, cyano, cycloalkyl, heterocycloalkyl, aryl, heteroaryl, cycloalkoxyl, heterocycloalkoxyl, cycloalkylthio, heterocycloalkylthio, oxo, carboxyl, or carboxylate.

The term "cycloalkylene" refers to further substitution of one hydrogen atom in cycloalkyl, with non-limiting examples including: cyclohexylene, 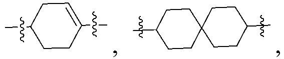 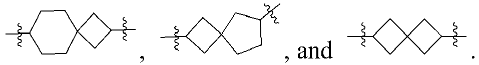 The cycloalkylene may be optionally substituted or unsubstituted. When the cycloalkylene is substituted, a substituent is preferably one or more of the following groups, independently selected from alkyl, alkoxyl, alkylthio, alkylamino, halogen, sulfhydryl, hydroxyl, nitro, cyano, cycloalkyl, heterocycloalkyl, cycloalkoxyl, heterocycloalkoxyl, cycloalkylthio, heterocycloalkylthio, oxo, carboxyl, or carboxylate.

The term "heterocyclyl" refers to a saturated or partially unsaturated monocyclic or polycyclic hydrocarbon substituent, which contains 3 to 20 ring atoms, where one or more ring atoms are heteroatoms selected from nitrogen, oxygen, C(O), S(O)(=NH), or S(O)ₘ (m being an integer from 0 to 2), but does not include a ring portion of -O-O-, -O-S-, or -S-S-, and the remaining ring atoms are carbon. Preferably, 3 to 12 ring atoms are contained, of which 1 to 4 are heteroatoms; more preferably, 3 to 12 ring atoms are contained; and most preferably, 3 to 6 ring atoms are contained. Non-limiting examples of monocyclic heterocyclyl include oxetanyl, thietanyl, azetidinyl, tetrahydropyranyl, azepanyl, pyrrolidinyl, imidazolidinyl, tetrahydrofuranyl, thiolanyl, dihydroimidazolyl, dihydrofuranyl, dihydropyrazolyl, pyrrolinyl, piperidyl, piperazinyl, morpholinyl, thiomorpholinyl, homopiperazinyl, and pyranyl, preferably oxetanyl, thietanyl, azetidinyl, tetrahydrofuranyl, tetrahydropyranyl, 1-aminoyl-1-oxothiopyran, azepanyl, piperidyl, and piperazinyl. Polycyclic heterocyclyl includes spirocyclic, fused cyclic, and endocyclic heterocyclyl, with non-limiting examples including 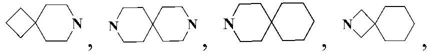 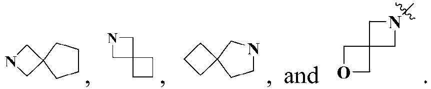 The heterocyclyl may be optionally substituted or unsubstituted. When the heterocyclyl is substituted, a substituent is preferably one or more of the following groups, independently selected from alkyl, alkoxyl, alkylthio, alkylamino, halogen, sulfhydryl, hydroxyl, nitro, cyano, amino, cycloalkyl, heterocycloalkyl, cycloalkoxyl, heterocycloalkoxyl, cycloalkylthio, heterocycloalkylthio, oxo, carboxyl, or carboxylate.

The term "heterocyclylene" refers to further substitution of one hydrogen atom in heterocyclyl, with non-limiting examples including: piperidinylene, piperazinylene, pyrrolopyrrolidinylene, diazaspiro[5.5]undecylene, azaspiro[5.5]undecylene, benzo piperidinylene, azocyclobutylene, diazacyclobutylene, pyrrolidinylene, azaspiro[3.5]nonylene, diazaspiro[3.5]nonylene, azabicyclo[3.1.1]heptylene, azaspiro[2.5]octylene, 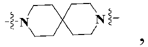 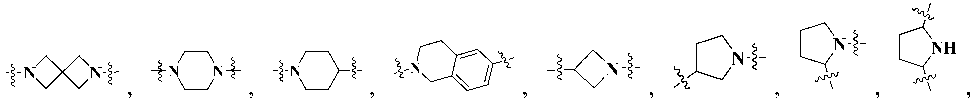 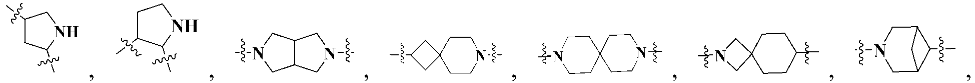 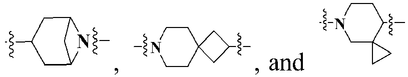 . The heterocyclylene may be optionally substituted or unsubstituted. When the heterocyclylene is substituted, a substituent is preferably one or more of the following groups, independently selected from alkyl, alkoxyl, alkylthio, alkylamino, halogen, sulfhydryl, hydroxyl, nitro, cyano, amino, cycloalkyl, heterocycloalkyl, cycloalkoxyl, heterocycloalkoxyl, cycloalkylthio, heterocycloalkylthio, oxo, carboxyl, or carboxylate.

The term "aryl" refers to a 6 to 14 membered all-carbon monocyclic or fused polycyclic (i.e., ring sharing adjacent carbon atom pairs) group with a conjugated π-electron system, preferably 6 to 10 membered, and more preferably phenyl. The aryl ring may be fused onto a heteroaryl, heterocyclyl, or cycloalkyl ring, where a ring connected to a parent structure is an aryl ring. The aryl may be substituted or unsubstituted. When the aryl is substituted, a substituent is preferably one or more of the following groups, independently selected from alkyl, alkoxyl, alkylthio, alkylamino, halogen, sulfhydryl, hydroxyl, nitro, cyano, cycloalkyl, heterocycloalkyl, cycloalkoxyl, heterocycloalkoxyl, cycloalkylthio, heterocycloalkylthio, carboxyl, or carboxylate.

The term "arylene" refers to further substitution of one hydrogen atom in aryl, with a non-limiting example including: 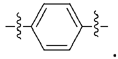 The arylene may be substituted or unsubstituted. When the arylene is substituted, a substituent is preferably one or more of the following groups, independently selected from alkyl, alkoxyl, alkylthio, alkylamino, halogen, sulfhydryl, hydroxyl, nitro, cyano, cycloalkyl, heterocycloalkyl, cycloalkoxyl, heterocycloalkoxyl, cycloalkylthio, heterocycloalkylthio, carboxyl, or carboxylate.

The term "heteroaryl" refers to a heteroaromatic system containing 1 to 4 heteroatoms and 5 to 14 ring atoms, where the heteroatoms are selected from oxygen, sulfur, and nitrogen. The heteroaryl is preferably 5-8 membered monoheteroaryl or 8-14 membered diheteroaryl, and more preferably 5-membered monoheteroaryl, 6-membered monoheteroaryl, or 9-membered diheteroaryl, for example, imidazolyl, furyl, thienyl, thiazolyl, pyrazolyl, pyrrolyl, triazolyl, tetrazyl, pyridyl, pyrimidinyl, pyrazinyl, pyridazinyl, piperazinyl, pyridinoimidazolyl, and pyrimidoimidazolyl, preferably pyridinoimidazolyl and pyrimidoimidazolyl.

The heteroaryl may be optionally substituted or unsubstituted. When the heteroaryl is substituted, a substituent is preferably one or more of the following groups, independently selected from alkyl, alkenyl, alkynyl, alkoxyl, alkylthio, alkylamino, halogen, sulfhydryl, hydroxyl, nitro, cyano, amino, cycloalkyl, heterocycloalkyl, aryl, heteroaryl, cycloalkoxyl, heterocycloalkoxyl, cycloalkylthio, heterocycloalkylthio, carboxyl, or carboxylate.

The term "heteroarylene" refers to further substitution of one hydrogen atom in heteroaryl, with non-limiting examples including:
pyridylene, pyrimidinylene, pyrazolylene, pyridazinylene, pyrazinylene,

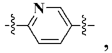

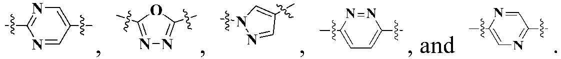

The term "alkoxyl" refers to -O- (alkyl) and -O- (unsubstituted cycloalkyl), where the alkyl is as defined above. Non-limiting examples of the alkoxyl include: methoxyl, ethoxyl, propoxy, butoxyl, cyclopropoxyl, cyclobutoxyl, cyclopentyloxyl, and cyclohexoxyl. The alkoxyl may be optionally substituted or unsubstituted. When the alkoxyl is substituted, a substituent is preferably one or more of the following groups, independently selected from alkyl, alkenyl, alkynyl, alkoxyl, alkylthio, alkylamino, halogen, sulfhydryl, hydroxyl, nitro, cyano, cycloalkyl, heterocycloalkyl, aryl, heteroaryl, cycloalkoxyl, heterocycloalkoxyl, cycloalkylthio, heterocycloalkylthio, carboxyl, or carboxylate. Non-limiting examples include trifluoromethoxyl, trifluoroethoxyl, and difluoromethoxyl.

"Haloalkyl" refers to alkyl substituted with one or more halogens, where the alkyl is as defined above. Non-limiting examples include: trifluoromethyl and difluoromethyl.

"Haloalkoxyl" refers to alkoxyl substituted with one or more halogens, where the alkoxyl is as defined above.

"Hydroxyalkyl" refers to alkyl substituted with hydroxyl, where the alkyl is as defined above. A non-limiting example includes: -C(CH₃)₂(OH).

"Hydroxyl" refers to an -OH group. "Halogens" refer to fluoro, chloro, bromo, or iodo. "Amino" refers to -NH₂. "Cyano" refers to -CN. "Nitro" refers to -NO₂. "Carboxyl" refers to - C(O)OH. "THF" refers to tetrahydrofuran. "EtOAc" refers to ethyl acetate. "MeOH" refers to methanol. "DMF" refers to N,N-dimethylformamide. "TFA" refers to trifluoroacetic acid. "MeCN" refers to acetonitrile. "DMA" refers to N,N-dimethylacetamide. "Et₂O" refers to diethyl ether. "DCE" refers to 1,2-dichloroethane. "DIPEA" refers to N,N-diisopropylethylamine. "NBS" refers to N-bromosuccinimide. "NIS" refers to N-iodosuccinimide. "Cbz-Cl" refers to benzyl chloroformate. "Pd₂(dba)₃" refers to tris(dibenzylideneacetone)dipalladium. "Dppf" refers to 1,1'-bis(diphenylphosphino)ferrocene. "HATU" refers to o-(7-azabenzotriazol-1-yl)-N,N,N',N'-tetramethyluronium hexafluorophosphate. "KHMDS" refers to potassium bis(trimethylsilyl)amide. "LiHMDS" refers to lithium bis(trimethylsilyl)amide. "MeLi" refers to methyl lithium. "n-BuLi" refers to n-butyl lithium. "NMP" refers to N-methyl pyrrolidone. "EDCI" refers to 1-(3-dimethylaminopropyl)-3-ethylcarbodiimide hydrochloride. "TEA" refers to triethylamine. "EA" refers to ethyl acetate. "DCM" refers to dichloromethane. "DMAP" refers to 4-dimethylaminopyridine. "NMO" refers to N-methylmorpholine oxide. "DIBAL-H" refers to diisobutylaluminum hydride. "T₃P" refers to 1-propylphosphonic anhydride. "DMP" refers to dimethyl phthalate. "Dess-Martin" refers to a Dess-Martin oxidant. "Ruphos" refers to 2-dicyclohexylphosphino-2',6'-diisopropoxy-1,1'-biphenyl. "Ruphos Pd G3" refers to methanesulfonato (2-dicyclohexylphosphino-2',6'-diisopropoxy-1,1'-biphenyl)(2'-amino-1,1'-biphenyl-2-yl)palladium(II). "LDA" refers to lithium diisopropylamide. "SEMCl" refers to 2-(trimethylsilyl) ethoxymethyl chloride. "STAB" refers to sodium triacetoxyborohydride. "P-TSA" refers to p-toluenesulfonic acid. "PCC" refers to pyridinium chlorochromate. "Pd-PEPPSI" refers to 1,3-bis[2,6-bis(pentyl-3-yl)phenyl]-4,5-dichloro-2,3-dihydro-1H-imidazol-2-yldichloro(2-methyl-1λ4-pyridin-1-yl)palladium. "LiBH₄" refers to lithium borohydride. "DCE" refers to 1,2-dichloroethane. "SEM" refers to (trimethylsilyl)ethoxymethyl. "TEBAC" refers to benzyl triethylammonium chloride.

"Wt%" refers to mass percent.

Different phrases such as "X is selected from A, B, or C", "X is selected from A, B, and C", "X is A, B, or C", and "X is A, B, and C" all express the same meaning, meaning that X may be any one or several of A, B, and C.

"- - - - - -" in the present application represents a bond or non-existence.

Chiral carbons in compounds as described in the present application, unless otherwise specified, are in an R or S configuration.

An absolute configuration of a chiral compound as described in the present application may be obtained by conventional chiral separation methods in the art or be prepared from chiral raw materials.

The hydrogen as described in the present application may be substituted with its isotope deuterium, and any hydrogen in the example compounds of the present application may also be substituted with a deuterium atom.

"Optional" or "optionally" means that an event or environment described subsequently may, but does not necessarily, occur, and the description includes situations where the event or environment occurs or does not occur. For example, "a heterocyclic group optionally substituted with alkyl" means that alkyl may but do not necessarily exist, and the description includes situations where the heterocyclic group is substituted with alkyl and situations where the heterocyclic group is not substituted with alkyl.

"Substituted" refers to that one or more hydrogen atoms in a group, preferably up to 5, and more preferably 1 to 3 hydrogen atoms, are independently substituted with a corresponding number of substituents. It goes without saying that substituents are only located at their possible chemical positions, and those skilled in the art can determine (through experimentation or theory) possible or impossible substitutions without too much effort. For example, amino or hydroxyl with free hydrogen may be unstable when combined with carbon atoms having unsaturated (e.g., olefinic) bonds.

A "pharmaceutical composition" refers to a mixture of one or more compounds described herein or physiologically/pharmaceutically acceptable salts or prodrugs thereof with other chemical components, as well as other components such as physiologically/pharmaceutically acceptable carriers and excipients. The purpose of a pharmaceutical composition is to promote administration to living organisms, facilitate absorption of active ingredients, and thus exert biological activity.

"Pharmaceutically acceptable salts" refer to the salts of the compounds as described in the present application, which are safe, effective, and have the expected biological activity when used in mammals.

### DETAILED DESCRIPTION

2222 The present application will be further described with reference to the following examples, but these examples do not limit the scope of the present application.

The starting materials and experimental reagents used in the examples of the present application are known and commercially available, or may be synthesized using or according to methods known in the art. For example, compound **1-1** in the present application may be prepared by referring to a method disclosed in WO2020264499A1; and compound **46-1** in the present application may be prepared by referring to a method disclosed in WO2022028547 A1.

### Preparation of intermediate A

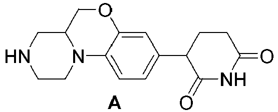

### Step 1: Preparation of compound A-2

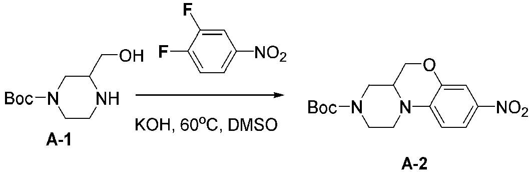

Ground potassium hydroxide (11.6 g, 207 mmol) powder was added to a solution of racemic 1-BOC-3-hydroxymethyl-piperazine **A-1** (15 g, 69 mmol) and 3,4-difluoronitrobenzene (12.8 g, 80.05 mmol) in DMSO (100 mL) at room temperature. The reaction solution was heated to 60°C and allowed to react for 8 h. The reaction was complete, and ice water was added to the reaction solution. The formed solid was collected by filtration, washed with water, and then purified by rapid column chromatography (petroleum ether: ethyl acetate=6:1). Compound **A-2** (16.2 g, yield 70%) was obtained as a yellow solid product.

LCMS: (ESI, *m*/*z):* 336.4 [M+H]⁺.

### Step 2: Preparation of compound A-3

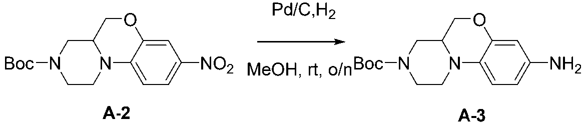

A solution of compound **A-2** (1 g, 2.982 mmol, 1 equiv) in methanol (30 mL) was added to an autoclave, followed by palladium/carbon (10%, 100 mg). Nitrogen replacement was performed and hydrogen gas (5 Mpa) was introduced for reaction at room temperature overnight. Then suction filtration was performed with diatomite and the filtrate was concentrated under reduced pressure. Compound **A-3** (900 mg, 98.8%) was obtained as a brown solid.

LCMS: (ESI, *m*/*z):* 306.5 [M+H]⁺.

### Step 3: Preparation of compound A-4

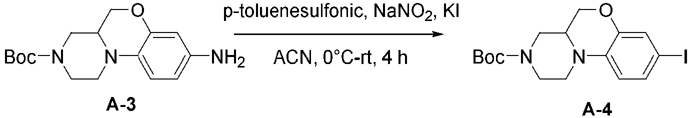

4-methylbenzene-1-sulfonic acid (1.02 g, 5.894 mmol, 2 equiv) was added to a solution of compound **A-3** (900 mg, 2.947 mmol, 1 equiv) in acetonitrile (10 mL) at room temperature. Following addition, the system was stirred at room temperature for 10 min. An aqueous solution of sodium nitrite (406.7 mg, 5.894 mmol, 2 equiv) and an aqueous solution of potassium iodide (1.47 g, 8.841 mmol, 3 equiv) were added to the system at 0°C. Following addition, the system was stirred at room temperature for 4 h. A product desired was found in liquid chromatography-mass spectrometry (LC-MS). The reaction mixture was quenched with a saturated aqueous solution of sodium sulfite at 0°C. The reaction mixture was extracted with dichloromethane (3×100 mL). The organic phases were mixed, backwashed with a saturated solution of sodium bicarbonate (2×100 mL), and dried over anhydrous sodium sulfate. The resulting mixture was filtered and the filtrate was concentrated under reduced pressure. The resulting residue was purified by silica gel column chromatography using ethyl acetate: petroleum ether (0% to 50%), and compound **A-4** (360 mg, 29.4%) was obtained as a yellow solid.

LCMS: (ESI, *m*/*z):* 361.3 [M+H]⁺.

### Step 4: Preparation of compound A-5

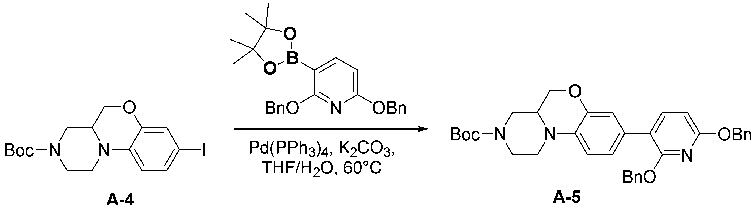

Under nitrogen protection, tetrakis(triphenylphosphine)palladium (299.82 mg, 0.260 mmol, 0.3 equiv) and potassium carbonate (239.05 mg, 1.730 mmol, 2 equiv) were added to a solution of compound **A-4** (360 mg, 0.865 mmol, 1 equiv) and 2,6-dibenzyloxy-3-(4,4,5,5-tetramethyl-1,3,2-dioxaborinane-2-yl) pyridine (433.1 mg, 1.038 mmol, 1.2 equiv) in tetrahydrofuran (6 mL) and water (3 mL) at room temperature. Following addition, the system was stirred at 60°C overnight. A product desired was found in LC-MS. The resulting residue was concentrated in vacuum. The resulting residue was purified by silica gel column chromatography using ethyl acetate: petroleum ether (0% to 50%), and compound **A-5** (160 mg, 31.91%) was obtained as a yellow solid.

LCMS: (ESI, *m*/*z):* 580.6 [M+H]⁺.

### Step 5: Preparation of compound A-6

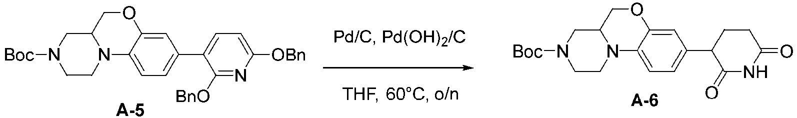

A solution of compound **A-5** (120 mg, 0.207 mmol, 1 equiv) in tetrahydrofuran (10 mL) was added to an autoclave, followed by palladium/carbon (10%, 100 mg) and palladium hydroxide/carbon (20% in palladium content, 100 mg). Nitrogen replacement was performed and hydrogen gas (5 Mpa) was introduced for reaction at 60°C overnight. Then suction filtration was performed with diatomite and the filtrate was concentrated under reduced pressure. Compound **A-6** (58 mg, 69.8%) was obtained as a brown solid.

LCMS: (ESI, *m*/*z):* 402.5 [M+H]⁺.

### Step 6: Preparation of compound A

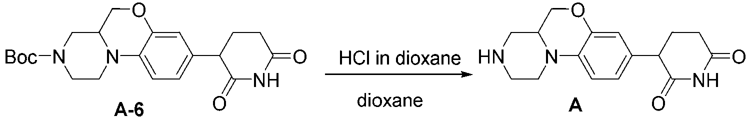

A solution (1 mL, 4 M) of hydrogen chloride in 1,4-dioxane was added to a solution of compound **A-6** (58 mg, 0.144 mmol, 1 equiv) in 1,4-dioxane (1 mL) at room temperature. Following addition, the system was stirred at room temperature overnight. A product desired was found in LC-MS. The resulting residue was concentrated in vacuum. Compound **A** (40 mg, 91.8%) was obtained as a brown solid.

LCMS: (ESI, *m*/*z):* 302.4 [M+H]⁺.

Intermediate A was separated using conventional chiral separation methods in the art to obtain intermediate J and intermediate H.

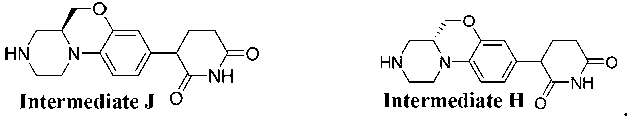

### Preparation of intermediate B

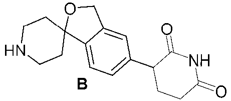

### Step 1: Synthesis of compound B-2

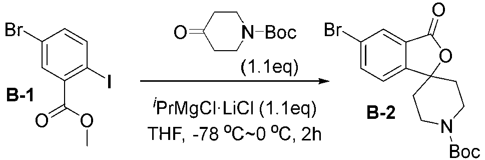

Under nitrogen protection, an isopropyl magnesium chloride-lithium chloride complex (0.47 g, 3.226 mmol, 1.1 equiv) was added to a solution of methyl 5-bromo-2-iodobenzoate (1 g, 2.933 mmol, 1 equiv) and tert-butyl 4-oxopiperidine-1-carboxylate (0.64 g, 3.226 mmol, 1.1 equiv) in tetrahydrofuran (40 mL) at -78°C, and reacted at -78°C for 2 h. After the reaction was complete, water (5 mL) was added at 0°C to quench the reaction. Then extraction was performed with ethyl acetate (3×40 mL), and the organic layers mixed were washed with water (3×20 mL) and dried over anhydrous sodium sulfate. Following filtration, the filtrate was concentrated under reduced pressure, and the residue was purified by silica gel column chromatography and eluted using petroleum ether/ethyl acetate (5:1). Compound **B-2** (900 mg, 80.3%) was obtained as a white solid.

¹H NMR (400 MHz, Chloroform-d, *ppm*) *δ* 8.03 (d, J = 1.8 Hz, 1H), 7.80 (dd, J = 8.1, 1.8 Hz, 1H), 7.28 (s, 1H), 4.20 (d, *J* = 13.7 Hz, 2H), 3.24 (t, *J* = 13.1 Hz, 2H), 2.11 - 2.02 (m, 2H), 1.68 (dd, *J* = 14.4, 2.5 Hz, 2H), 1.68 (s, 9H).

### Step 2: Synthesis of compound B-3

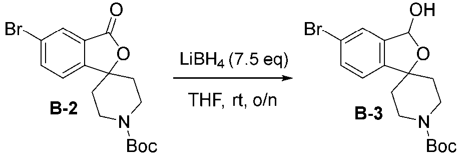

Under nitrogen protection, a solution of lithium borohydride in THF (2 M, 17.7 mmol, 7.5 equiv) was added dropwise to a solution of compound **B-2** (900 mg, 2.354 mmol, 1.0 equiv) in tetrahydrofuran (5 mL) at 0°C. The reaction solution was stirred at room temperature overnight. After the reaction was complete, the reaction was quenched with a saturated solution of water and sodium bicarbonate at 0°C. The reaction mixture was extracted with ethyl acetate (3×100 mL). The organic phases were mixed, backwashed with water (3×30 mL), and dried over anhydrous sodium sulfate. The resulting mixture was filtered, and the filtrate was concentrated under reduced pressure. The resulting residue was purified by silica gel column chromatography using petroleum ether/ethyl acetate (4:1), and compound **B-3** (700 mg, 77.4%) was obtained as white foam.

LCMS: (ESI, *m*/*z*): 384.2[M+H]⁺.

### Step 3: Synthesis of compound B-4

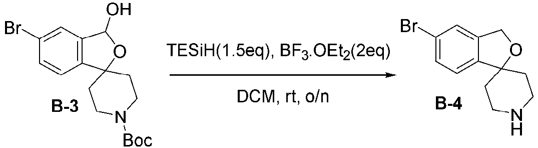

Boron trifluoride diethyl etherate (221.61 mg, 1.562 mmol, 2.0 equiv) was added dropwise to a solution of compound **B-3** (300 mg, 0.781 mmol, 1 equiv) and triethylsilane (136.17 mg, 1.171 mmol, 1.5 equiv) in dichloromethane (3 mL) at 0°C. The reaction solution was stirred at room temperature overnight. After the reaction was complete, the reaction mixture was quenched with water at room temperature. Extraction was performed with dichloromethane (3×10 mL), and the organic phases were mixed, backwashed with water (3×5 mL), and dried over anhydrous sodium sulfate. The resulting mixture was filtered, and the filtrate was concentrated under reduced pressure. The resulting residue was purified by silica gel column chromatography using dichloromethane/methanol (10:1), and compound **B-4** (160 mg, 76.4%) was obtained as a white solid.

LCMS: (ESI, *m*/*z):* 268.1 [M+H]⁺.

### Step 4: Synthesis of compound B-5

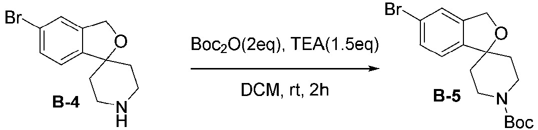

Di-tert-butyl dicarbonate (260.5 mg, 1.194 mmol, 2.0 equiv) was added dropwise to a solution of compound **B-4** (160 mg, 0.597 mmol, 1.0 equiv) and triethylamine (72.5 mg, 0.716 mmol, 1.5 equiv) in dichloromethane (2 mL) at room temperature. The reaction solution was stirred overnight. The resulting residue was concentrated under reduced pressure. The resulting residue was purified by silica gel column chromatography using petroleum ether/ethyl acetate (10:1), and compound **B-5** (215 mg, 97.8%) was obtained as a white solid.

¹H NMR (400 MHz, Chloroform-d, ppm) *δ* 7.42 - 7.38 (d, *J* = 1.6 Hz, 1H), 7.36 (d,*J* = 1.6 Hz, 1H), 6.96 (d, *J =* 8.0 Hz, 1H), 5.04 (s, 2H), 4.12 - 4.03 (m, 2H), 3.15 (td, *J =* 12.9, 3.1 Hz, 2H), 1.82 - 1.67 (m, 4H), 1.49 (s, 9H).

### Step 5: Synthesis of compound B-6

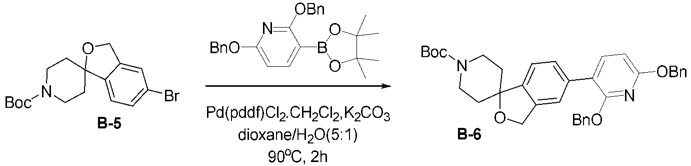

Under nitrogen protection, a [1,1'-bis(diphenylphosphine)ferrocene]palladium chloride dichloromethane complex (46.5 mg, 0.057 mmol, 0.1 equiv) and potassium carbonate (157.6 mg, 1.14 mmol, 2.00 equiv) were added to a solution of compound **B-5** (210 mg, 0.570 mmol, 1.0 equiv) and 2,6-bis(benzyloxy)-3-(4,4,5,5-tetramethyl-1,3,2-dioxoboran-2-yl)pyridine (309.35 mg, 0.741 mmol, 1.3 equiv) in 1,4-dioxane (5 mL) and water (1 mL) at room temperature. The reaction solution was stirred at 90°C for 2 h. After the reaction was complete, extraction was performed with ethyl acetate (3×50 mL). The organic phases were mixed, backwashed with water (3×30 mL), and dried over anhydrous sodium sulfate. The resulting residue was purified by reverse-phase column chromatography (C18 column, mobile phase: water and acetonitrile, 50% to 95%, gradient for 20 min, UV 220 nm), and compound **B-6** (160 mg, 48.5%) was obtained as a white solid.

LCMS: (ESI, *m*/*z):* 579.3 [M+H]⁺.

### Step 6: Synthesis of compound B-7

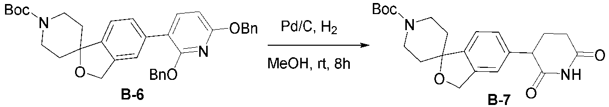

A solution of compound **B-6** (120 mg, 0.207 mmol, 1.0 equiv) in anhydrous methanol (2 mL) was added to a 10 mL autoclave, followed by palladium/carbon (22.07 mg, 0.207 mmol, 1.0 equiv) at room temperature. Under hydrogen protection, the reaction solution was stirred at room temperature for 8 h. After the reaction was complete, filtration was performed, the filter cake was washed with methanol (15 mL), and the filtrate was concentrated under reduced pressure. Compound **B-7** (60 mg, 72.25%) was obtained as a white solid. The crude product was directly used in the next step without being purified.

LCMS: (ESI, *m*/*z):* 401.2 [M+H]⁺.

### Step 7: Synthesis of compound B

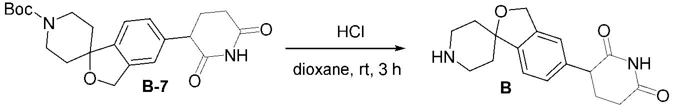

A solution (1 M, 0.08 mL) of hydrogen chloride in 1,4-dioxane was added dropwise to a solution of compound **B-7** (50 mg, 0.125 mmol) in tetrahydrofuran (2 mL) at room temperature. The reaction solution was stirred for 2 h. After the reaction was complete, the resulting residue was concentrated under reduced pressure, and compound **B** (30 mg, 80%) was obtained as a white solid.

LCMS: (ESI, *m*/*z):* 301.2 [M+H]⁺.

### Preparation of intermediate C

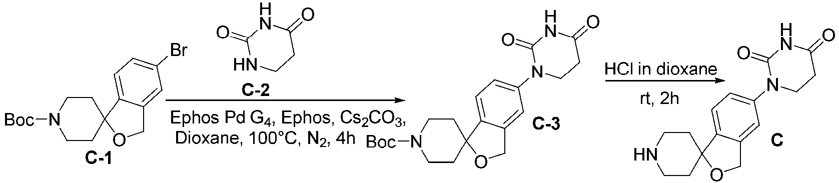

### Step 1: Synthesis of compound C-3

Under nitrogen protection, ({bicyclohexyl(3-isopropoxy-2',4',6'-triisopropyl-[1,1'-biphenyl]-2-yl)phosphine}(2'-methylamino-1,1'-biphenyl-2-yl)methanesulfonate) palladium (II) (124.7 mg, 0.136 mmol) was added to a solution of compound **C-1** (500 mg, 1.36 mmol), compound **C-2** (185.9 mg, 1.63 mmol), cesium carbonate (884.7 mg, 2.72 mmol), and bicyclohexyl(3-isopropoxy-2',4',6'-triisopropyl-[1,1'-biphenyl]-2-yl)phosphine (145.2 mg, 0.27 mmol) in 1,4-dioxane (5 mL). The reaction solution was stirred at 100°C for 4 h. Disappearance of the raw materials was detected by LC-MS, and the resulting residue was concentrated under reduced pressure. The resulting residue was purified by silica gel column chromatography using dichloromethane: methanol (0% to 30%), and compound **C-3** (150 mg, 27.5%) was obtained.

LCMS (ESI, m/z): 346.0 [M-55]⁺.

### Step 2: Synthesis of compound C

Compound **C-3** (150 mg, 0.374 mmol) was dissolved in a solution (4 mol/L) of hydrogen chloride in 1,4-dioxane (3 mL) at room temperature. The reaction solution was stirred for 2 h. Disappearance of the raw materials was detected by LC-MS. The resulting residue was concentrated under reduced pressure, and intermediate C (100 mg, 88.8%) was obtained.

LCMS (ESI, m/z): 302.5 [M+H]⁺.

### Preparation of intermediate D

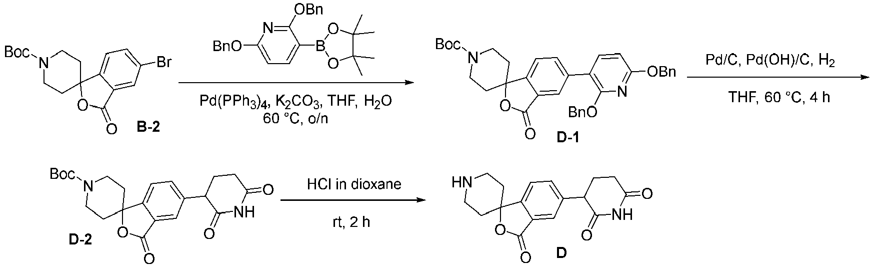

According to the preparation method of intermediate **B,** compound **B-5** was replaced with compound **B-2** to prepare intermediate **D.** LCMS (ESI, m/z): 315.0 [M+H]⁺.

### Preparation of intermediate E

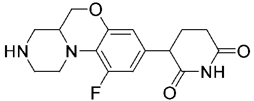

According to the preparation method of intermediate A, compound 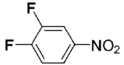 was replaced with compound 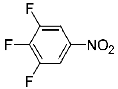 to prepare intermediate E. LCMS (ESI, m/z): 320.5 [M+H]⁺.

### Preparation of intermediate E-a

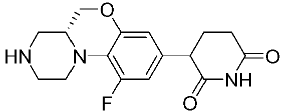

According to the preparation method of intermediate **A,** compound 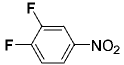 was replaced with compound 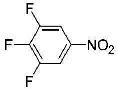 and compound **A-1** was replaced with compound 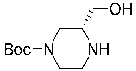 to prepare intermediate **E-a.** LCMS (ESI, *m*/*z):* 320.1 [M+1]⁺.

### Preparation of intermediate E-b

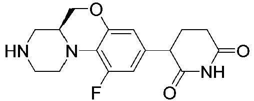

According to the preparation method of intermediate **A,** compound 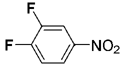 was replaced with compound 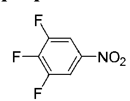 and compound **A-1** was replaced with compound 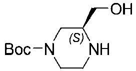 to prepare intermediate **E-b.** LCMS (ESI, *m*/*z):* 320.1 [M+1]⁺.

### Preparation of intermediate F

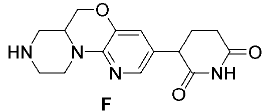

According to the preparation method of intermediate **A,** compound 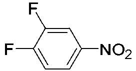 was replaced with compound 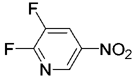 to prepare intermediate **F.** LCMS (ESI, m/z): 303.1 [M+H]⁺.

### Preparation of intermediate G

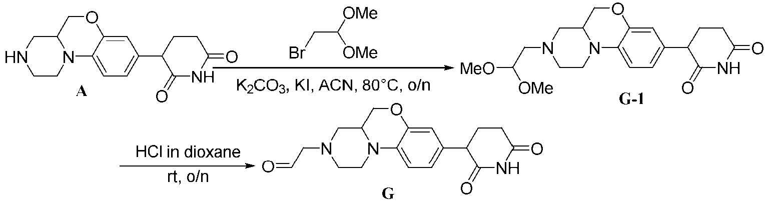

Under nitrogen protection, potassium iodide (110.2 mg, 0.663 mmol, 0.5 eq) was added to a solution of intermediate **A** (400 mg, 1.33 mmol, 1 eq), 2-bromo-1,1-dimethoxyethane (224.4 mg, 1.33 mmol, 1 eq), and potassium carbonate (366.9 mg, 2.66 mmol, 2 eq) in acetonitrile (4 mL) at room temperature. Then, the reaction solution was heated to 80°C and stirred overnight. Disappearance of the raw materials was detected by LC-MS. Filtration was performed, the filter cake was washed with tetrahydrofuran (3×30 mL), and the filtrate was concentrated under reduced pressure. The crude product was purified by high-performance liquid chromatography, and compound **G-1** (200 mg, 38.7%) was obtained under the following conditions (chromatography column specification: YMC Triart C18 ExRs 5 um, 30 mm*150 mm; mobile phase A: water (10 mmol/L ammonium bicarbonate), mobile phase B: acetonitrile; flow rate: 60 mL/min; gradient: 23% B to 38% B in 10 min; detection wavelength: UV 254 nm; retention time (min): 9.97. LCMS (ESI, m/z): 390.1 [M+H]⁺.

Compound **G-1** (70 mg, 0.18 mmol, 1 eq) was added to a solution (4 M, 3.6 mL) of hydrogen chloride in 1,4-dioxane at room temperature and the reaction solution was stirred at room temperature overnight. A product desired was found in LC-MS. The resulting residue was concentrated in vacuum, and intermediate **G** (80 mg, crude) was obtained as a white solid. LCMS (ESI, m/z): 344.2 [M+H]⁺.

### Alternative preparation method of intermediate H

According to the preparation method of intermediate **A,** compound **A-1** was replaced with compound 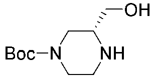 to prepare intermediate **H.** LCMS (ESI, m/z): 301.9 [M+H]⁺.

### Preparation of intermediate I

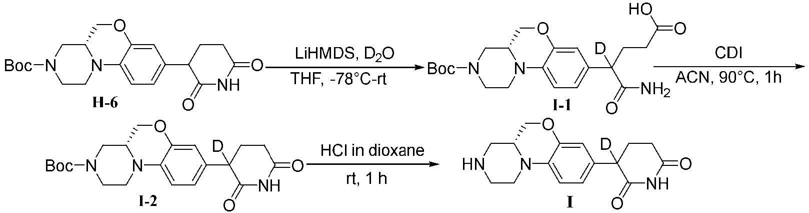

According to the preparation method of compound **A-6,** compound **A-1** was replaced with compound 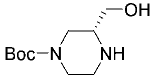 to prepare compound **H-6.** Under nitrogen protection, lithium bis(trimethylsilyl)amide (1.9 mL, 1 M) was added dropwise to a solution of compound **H-6** (300 mg, 0.747 mmol, 1 eq) in tetrahydrofuran (7.5 mL) at -78°C. Following addition, the system was stirred at -78°C for 1 h. Deuterium water (1.2 mL) was added to the system at -78°C. Following addition, the system was stirred at room temperature overnight. A product desired was found in LC-MS. The reaction mixture was quenched with an aqueous solution of citric acid at room temperature. The reaction mixture was extracted with ethyl acetate (2×50 mL). The organic phases were mixed, backwashed with water (1×50 mL), and dried over anhydrous sodium sulfate. The resulting mixture was filtered and the filtrate was concentrated under reduced pressure. **I-1** (140 mg, 44.6%) was obtained as a white solid. LCMS (ESI, m/z): 421.2 [M+H]⁺.

1,1'-carbonyldiimidazole (154.3 mg, 0.96 mmol, 4 eq) was added to a solution of **I-1** (100 mg, 0.238 mmol, 1 eq) in acetonitrile (2.35 mL) at room temperature. Following addition, the system was stirred at 90°C for 1 h. Complete reaction was detected by LC-MS. The resulting residue was concentrated under reduced pressure. The crude product was purified by silica gel column chromatography using ethyl acetate/petroleum ether (0% to 60%), and **I-2** (61 mg, 63.7%) was obtained as a white solid. LCMS (ESI, m/z): 402.9 [M+H]⁺.

**I-2** (73 mg, 0.181 mmol, 1 eq) was added to a solution (1.8 mL, 4 M) of hydrogen chloride in 1,4-dioxane at room temperature. Following addition, the system was stirred at room temperature for 1 h. Complete reaction was detected by LC-MS. The resulting residue was concentrated in vacuum. Intermediate **I** (65 mg) was obtained as a white-like solid. LCMS (ESI, m/z): 303.4 [M+H]⁺.

### Alternative preparation method of intermediate J

According to the preparation method of intermediate **A,** compound **A-1** was replaced with 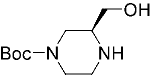 to prepare intermediate **J.** LCMS (ESI, m/z): 302.4 [M+H]⁺.

### Preparation of intermediate K

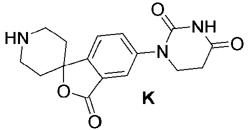

According to the preparation method of intermediate **C,** compound **C-1** was replaced with compound **B-2** to prepare intermediate **K.** LCMS (ESI, m/z): 316.1 [M+H]⁺.

### Preparation of intermediate L

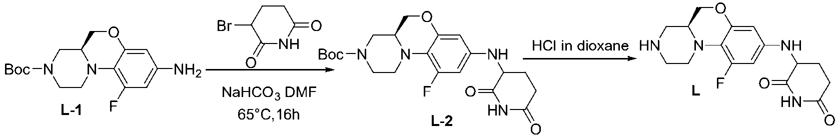

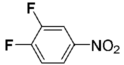

was replaced with compound 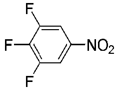 and **A-1** was replaced with 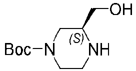 to prepare compound **L-1.** Under nitrogen protection, sodium bicarbonate (779.4 mg, 9.3 mmol, 3 eq) was added to a solution of **L-1** (1 g, 3.1 mmol, 1 eq) and 3-bromopiperidine-2,6-dione (890.7 mg, 4.64 mmol, 1.5 eq) in dimethylformamide (10 mL). Following complete addition, the system was stirred at 65°C for 16 h. Complete reaction was detected by LC-MS. The reaction mixture was extracted with ethyl acetate (3×100 mL), and the organic phases were mixed, backwashed with saturated brine (2×100 mL), and dried over anhydrous sodium sulfate. The resulting mixture was filtered and the filtrate was concentrated under reduced pressure. The resulting residue was purified by silica gel column chromatography using petroleum ether/ethyl acetate (0% to 50%), and **L-2** (600 mg, 44.7%) was obtained as a light green solid. LCMS (ESI, m/z): 435.2 [M+H]⁺.

A solution (2 mL, 4 M) of **L-2** (200 mg, 0.46 mmol, 1 eq) and hydrogen chloride in 1,4-dioxane was stirred for reaction for 1 h at room temperature. Complete reaction was detected by LC-MS, and the resulting residue was concentrated under reduced pressure. Intermediate **L** (150 mg, 97.5%) was obtained as a white-like solid. LCMS (ESI, m/z): 334.9 [M+H]⁺.

### Preparation of intermediate M

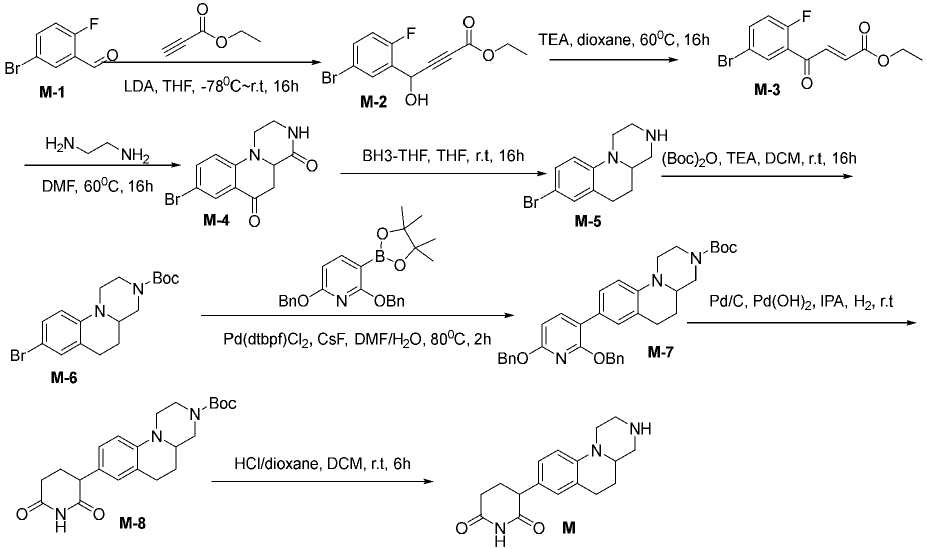

**M-1** (15 g, 73.9 mmol, 1.0 eq) was added to a reaction flask, nitrogen replacement was performed, and then THF (130 mL) was added. A solution (20 mL) of ethyl propiolate (7.97 g, 81.3 mmol, 1.1 eq) in THF was added dropwise at -78°C. Following addition, LDA (2 M, 40.6 mL, 81.3 mmol, 1.1 eq) was added dropwise at -78°C and stirred at -78°C for 2 h. Complete reaction of the raw materials was detected by TLC. After the reaction solution returned to room temperature, an aqueous solution of ammonium chloride was added for quenching the reaction. Following complete quenching, EA was added for extraction and liquid separation. The organic phase was dried over sodium sulfate and spin dried, and the resulting oily substance **M-2** crude product (12.0 g, yield: 53.8%) was directly used in the next step. LCMS (ESI, m/z): 300.8 [M+H]⁺.

The resulting **M-2** crude product (12.0 g, 1.0 eq) was dissolved in 1,4-dioxane (100 mL), and then triethylamine (14 mL, 121.7 mmol, 2.5 eq) was added and reacted at 60°C for 3 h. Complete reaction of the raw materials was detected by TLC. The reaction solution was cooled to room temperature and was spin dried, and 7 g of **M-3** was obtained as an oily substance with a yield of 58.3%. LCMS (ESI, m/z): 300.8 [M+H]⁺.

The **M-3** (5.1 g, 17 mmol, 1.0 eq) was dissolved in DMF (60 mL) in a sealed tube, and ethylenediamine (2.0 g, 34 mmol, 2.0 eq) and triethylamine (7 mL, 51 mmol, 3.0 eq) were added, heated to 60°C, and stirred for 16 h. Complete reaction of the raw materials was detected by TLC. After the reaction solution was cooled to room temperature, water (10 mL) and EA were added for extraction. Liquid separation, drying, concentration, and purification by chromatography were performed, and 3.7 g **of M-4** was obtained as a yellow solid with a yield of 74.03%. LCMS (ESI, m/z): 295.0 [M+H]⁺.

The **M-4** (300 mg, 1.02 mmol, 1.0 eq) was dissolved in THF (5 mL), nitrogen replacement was performed, and a solution (4.08 mL, 4.08 mmol, 4 eq) of borane in tetrahydrofuran was added dropwise in an ice bath. Following addition, the temperature was increased to 60°C overnight. Complete reaction was detected by TLC. Methanol was slowly added dropwise at low temperature until no bubbles were generated, and then stirred at 60°C for 2 h. 0.5 mL of a 1 M aqueous solution of hydrochloric acid was added and stirred at 60°C overnight. After complete quenching was detected and confirmed by LC-MS, the reaction solution was cooled to room temperature, and triethylamine (412 mg, 4.08 mmol, 4.0 eq) was added and stirred for 0.5 h. After the solvent was spin dried, DCM (10 mL) and (BOC)₂O (445 mg, 2.04 mmol, 2.0 eq) were added and the reaction solution was stirred at room temperature for 5 h. Complete reaction was detected by TLC. Water (10 mL) and EA were added for extraction, and drying over anhydrous sodium sulfate, concentration, and purification by column chromatography were performed. **M-6** (360 mg, yield: 96.4%) was obtained as a colorless oil substance. LCMS (ESI, m/z): 367.1 [M+H]⁺.

**M-6** (140 mg, 0.38 mmol, 1.0 eq) was dissolved in 5 mL of DMF, and 2,6-bis(benzyloxy)-3-(4,4,5,5-tetramethyl-1,3,2-dioxoboran-2-yl)pyridine (318 mg, 0.76 mmol, 2.0 eq), [1,1'-bis(di-tert-butylphosphine)ferrocene]palladium chloride (50.4 mg, 0.076 mmol, 0.2 eq), cesium fluoride (174 mg, 1.15 mmol, 3.0 eq), and water (1 mL) were added and the reaction solution was stirred at 80°C for 3 h following nitrogen replacement. The reaction was stopped after complete reaction was detected by TLC. After the reaction solution was cooled to room temperature, water (10 mL) and EA were added for extraction. Liquid separation, drying, concentration, and purification by column chromatography were performed, and **M-7** (120 mg, yield: 54.4%) was obtained as a yellowish oily substance. LCMS (ESI, m/z): 578.3 [M+H]⁺.

**M-7** (120 mg, 0.21 mmol, 1.0 eq) was dissolved in 2 mL of isopropanol, and Pd/C (12 mg) and palladium hydroxide on carbon (12 mg) were added and the reaction solution was stirred at room temperature for 16 h following hydrogen replacement. Complete reaction was detected by TLC. The reaction solution was filtered with diatomite, and the mother liquor was concentrated. **M-8** (70 mg, yield: 84.4%) was obtained as a yellowish oily substance. LCMS (ESI, m/z): 400.2 [M+H]⁺.

**M-8** (70 mg, 0.18 mmol, 1.0 eq) was dissolved in 2 mL of DCM, and HCl-dioxane (4 M, 0.44 mL, 1.8 mmol, 10 eq) was added and the reaction solution was stirred at room temperature for 2 h. Complete reaction was detected by TLC. The reaction solution was concentrated, dispersed by adding DCM, and spin dried. **M** (40 mg, yield: 76.9%) was obtained as a yellowish oily substance. LCMS (ESI, m/z): 300.2 [M+H]⁺.

### Preparation of intermediate N

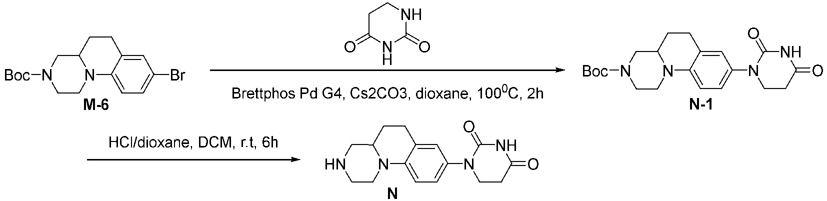

**M-6** (90 mg, 0.25 mmol, 1.0 eq), dihydrouracil (42 mg, 0.37 mmol, 1.5 eq), Brettphos Pd G4 (45 mg, 0.05 mmol, 0.2 eq), cesium carbonate (240 mg, 0.74 mmol, 3.0 eq), and dioxane (2 mL) were added to a reaction flask, and stirred at 100°C for 4 h following nitrogen displacement. Complete reaction of the raw materials was detected by TLC. After the reaction solution was cooled to room temperature, water and EA were added for extraction and liquid separation, and the organic phase was dried over sodium sulfate, spin dried, and purified by column chromatography. **N-1** (45 mg, yield: 45.8%) was obtained as an oily substance. LCMS (ESI, m/z): 400.8 [M+H]⁺.

**N-1** (45 mg, 0.11 mmol, 1.0 eq) was dissolved in 2 mL of DCM, and HCl-dioxane (4 M, 0.28 mL, 1.1 mmol, 10 eq) was added and the reaction solution was stirred at room temperature for 2 h. Complete reaction was detected by TLC. The reaction solution was directly spin dried, and 35 mg of intermediate N was obtained as a yellowish oily substance with a crude yield of 103%. LCMS (ESI, m/z): 301.1 [M+H]⁺.

### Preparation of intermediate O

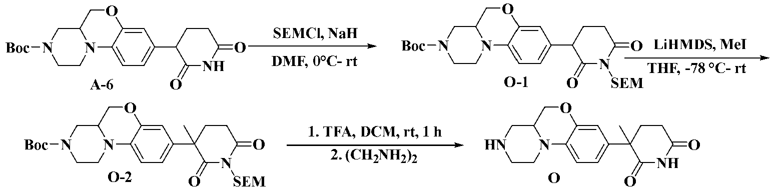

Sodium hydride (110 mg, 60%) was added to a solution of compound **A-6** (1 g, 2.491 mmol, 1 eq) in *N,N*-dimethylformamide (20 mL) at 0°C. Following addition, the system was stirred at 0°C for 0.5 h. 2-(trimethylsilyl)ethoxymethyl chloride (458 mg, 2.747 mmol, 1.10 eq) was added to the system at 0°C. Following addition, the system was stirred at room temperature for 1 h. Complete reaction was detected by LC-MS. The reaction mixture was quenched with water at 0°C. The reaction mixture was extracted with ethyl acetate (2×100 mL). The organic phases were mixed, backwashed with a saturated solution of sodium chloride (1×100 mL), and dried over anhydrous sodium sulfate. The resulting mixture was filtered and the filtrate was concentrated under reduced pressure. The resulting residue was purified by silica gel column chromatography using ethyl acetate: petroleum ether (0% to 30%), and compound **O-1** (770 mg, 58.14%) was obtained as a yellow oil. LCMS: (ESI, *m*/*z):* 532.5 [M+H]⁺.

Under nitrogen protection, a solution (0.72 mL, 1 M) of lithium bis(trimethylsilyl)amide in tetrahydrofuran was added to a solution of **O-1** (320 mg, 0.602 mmol, 1 eq) in tetrahydrofuran (5 mL) at -78°C. Following addition, the system was stirred at -78°C for 1 h. Iodomethane (171 mg, 1.205 mmol, 2.00 eq) was added to the system at -78°C. Following addition, the system was stirred at -78°C for 1 h. Then, the system was stirred at room temperature for 0.5 h. Complete reaction was detected by LC-MS. The reaction mixture was quenched with water at 0°C. The reaction mixture was extracted with ethyl acetate (2×100 mL). The organic phases were mixed, backwashed with a saturated solution of sodium chloride (1×100 mL), and dried over anhydrous sodium sulfate. The resulting mixture was filtered and the filtrate was concentrated under reduced pressure. The resulting residue was purified by silica gel column chromatography using ethyl acetate: petroleum ether (0% to 50%), and compound **O-2** (137 mg, 41.71%) was obtained as a brown oil. LCMS (ESI, *m*/*z):* 546.2 [M+H]⁺.

Trifluoroacetic acid (0.6 mL, 8.078 mmol, 35.55 eq) was added to a solution of compound **O-2** (124 mg, 0.227 mmol, 1 eq) in dichloromethane (4 mL) at room temperature. Following addition, the system was stirred at room temperature for 1 h. Complete reaction of the raw materials was detected by LC-MS, and ethylenediamine (0.8 mL) was added to the system at 0°C. Following addition, the system was stirred at room temperature for 1 h. Complete reaction was detected by LC-MS. A product desired was found in LC-MS. The reaction mixture was neutralized to pH=7 with citric acid. 10 mL of water was added to the reaction mixture, and the reaction mixture was extracted with dichloromethane ( 2×20 mL). The organic phases were mixed, backwashed with a saturated solution of sodium chloride (1×30 mL), and dried over anhydrous sodium sulfate. The resulting mixture was filtered and the filtrate was concentrated under reduced pressure. Intermediate **O** (98 mg, 94.37%) crude product was obtained as a yellow solid. LCMS: (ESI, *m*/*z):* 316.1 [M+H]⁺.

### Preparation of intermediate P

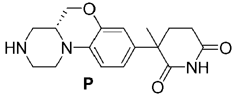

According to the preparation method of compound **A-6,** compound **A-1** was replaced with 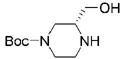 to prepare compound **H-6,** and then intermediate **P** was obtained according to the preparation method of intermediate O. LCMS: (ESI, *m*/*z):* 316.2 [M+H]⁺.

### Preparation of intermediate Q

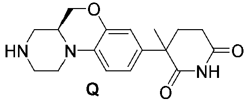

According to the preparation method of compound **A-6,** compound **A-1** was replaced with compound 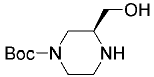 to prepare compound **J-6,** and then intermediate **Q** was obtained according to the preparation method of intermediate O. LCMS: (ESI, *m*/*z):* 316.2 [M+H]⁺.

### Preparation of intermediate R

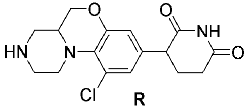

According to the preparation method of intermediate **A,** 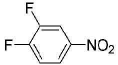 was replaced with compound 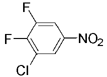 to prepare intermediate **R.** LCMS (ESI, *m*/*z):* 336.1 [M+1]⁺.

### Preparation of intermediate S

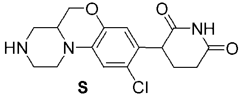

According to the preparation method of intermediate **A,** 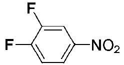 was replaced with compound 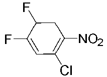 to prepare intermediate **S.** LCMS (ESI, *m*/*z):* 336.1 [M+1]⁺.

### Preparation of intermediate T

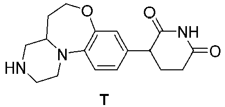

According to the preparation method of intermediate **A, A-1** was replaced with compound 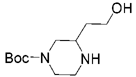 to prepare intermediate **T.** LCMS (ESI, *m*/*z):* 316.4 [M+1]⁺.

### Preparation of intermediate U

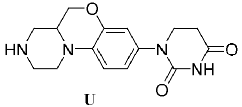

According to the preparation method of intermediate **N, M-6** was replaced with compound 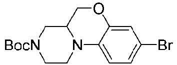 to prepare intermediate U. LCMS (ESI, *m*/*z):* 303.1 [M+1]⁺.

### Preparation of intermediate V

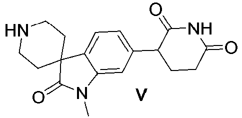

According to the preparation method of intermediate **A, A-4** was replaced with compound 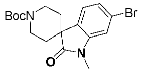 to prepare intermediate V. LCMS (ESI, *m*/*z):* 328.2 [M+1]⁺.

### Preparation of intermediate W

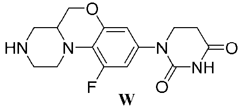

According to the preparation method of intermediate **N, M-6** was replaced with compound 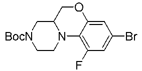 to prepare intermediate **W.** LCMS (ESI, m/z): 321.1 [M+1]⁺.

### Preparation of intermediate Y-1

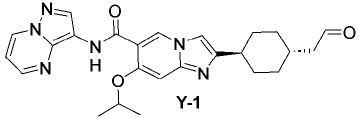

Intermediate **Y-1** was synthesized according to the prior art WO2023283610 A1.

### Preparation of intermediate Y-2

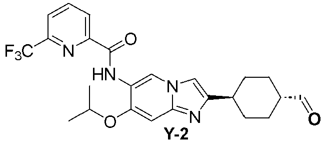

Intermediate **Y-2** was synthesized according to the prior art WO02023283372A1.

### Preparation of intermediate Y-3

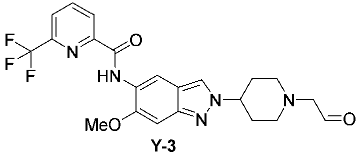

Intermediate **Y-3** was synthesized according to the prior art WO2022028547A1.

### Preparation of intermediate Y-4

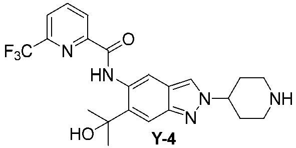

Intermediate **Y-4** was synthesized according to the prior art WO2022028547A1.

### Preparation of intermediate Y-5-1

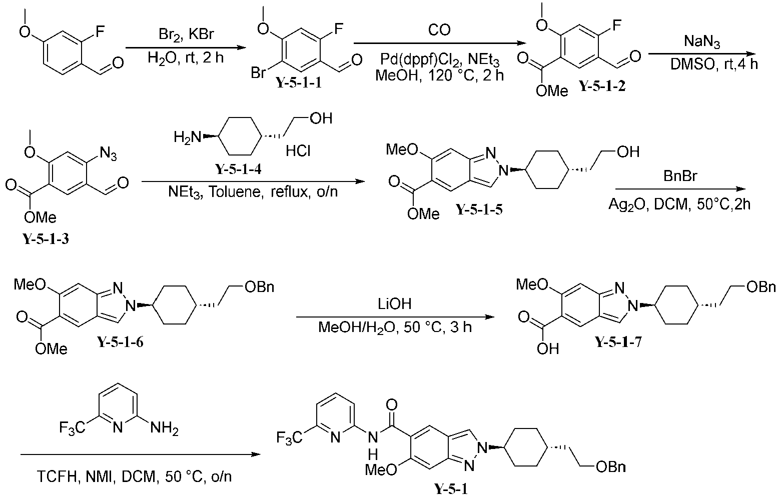

2-fluoro-4-methoxybenzaldehyde (24 g, 155.7 mmol, 1 eq) was added to a solution of bromine (49.77 g, 311.4 mmol, 2 eq) and potassium bromide (92.6 g, 778.5 mmol, 5 eq) in water (310 mL) at 5°C, and the reaction solution was stirred at room temperature for 2 h. Following complete reaction, water (500 mL) was added to the system, and a large amount of solid was observed to precipitate. The solid was filtered, and the filter cake was collected and washed with a saturated solution of sodium bicarbonate (3×200 mL). After freeze-drying, 5-bromo-2-fluoro-4-methoxybenzaldehyde **(Y-5-1-1,** 30 g, 82.7%) was obtained as a yellow solid.

In an autoclave, 1,1-bis(diphenylphosphino)ferrocene palladium dichloride (3.50 g, 4.3 mmol, 0.05 eq) and triethylamine (17.37 g, 171.6 mmol, 2 eq) were added to a solution of compound **Y-5-1-1** (20 g, 85.8 mmol, 1 eq) in methanol (300 mL), nitrogen replacement was performed for 10 min, and carbon monoxide was introduced to 5 MPa for reaction at 120°C for 2 h. Complete reaction was detected by LC-MS. The system was cooled to room temperature, and insoluble substances were removed by filtration. The resulting residue was concentrated under reduced pressure. The resulting residue was purified by silica gel column chromatography using ethyl acetate/petroleum ether (0% to 60%), and methyl 4-fluoro-5-formyl-2-methoxybenzoate **Y-**5-1-2 (8.7 g, 47%) was obtained as a white-like solid.

¹H NMR (400 MHz, DMSO-d₆) δ 10.06 (s, 1H), 8.17 (d, J = 8.3 Hz, 1H), 7.25 (d, J = 13.1 Hz, 1H), 3.94 (s, 3H), 3.81 (s, 3H).

Under nitrogen protection, sodium azide (5.81 g, 89.4 mmol, 2 eq) was added in batches to a solution of compound **Y-5-1-2** (8.9 g, 42.9 mmol, 1 eq) in dimethyl sulfoxide (150 mL) at room temperature. After the reaction solution was stirred for 4 h, complete reaction was detected. The reaction mixture was quenched with ice water at 0°C. The reaction mixture was extracted with ethyl acetate (3×300 mL). The organic phases were mixed, backwashed with a saturated aqueous solution of sodium bicarbonate (2×300 mL), and dried over sodium sulfate. The resulting mixture was filtered, the filtrate was concentrated under reduced pressure, and **Y-5-1-3** (7.9 g, 75.2%) was obtained as a brown solid.

¹H NMR (400 MHz, DMSO-d₆) δ 10.08 (s, 1H), 8.12 (s, 1H), 7.10 (s, 1H), 4.00 (s, 3H), 3.80 (s, 3H).

Under nitrogen protection, triethylamine (10.2 g, 100.8 mmol, 3 eq) was added to a solution of methyl 4-azido-5-formyl-2-methoxybenzoate **Y-5-1-3** (7.9 g, 33.6 mmol, 1 eq) and **Y-5-1-4** (5.77 g, 40.3 mmol, 1.2 eq) in toluene (200 mL) at 110°C, and the reaction solution was stirred overnight. Complete reaction was detected by LC-MS. The resulting residue was concentrated under reduced pressure. The reaction mixture was extracted with ethyl acetate (3×200 mL). The organic phases were mixed and dried over sodium sulfate. The resulting mixture was filtered and the filtrate was concentrated under reduced pressure. The resulting residue was purified by silica gel column chromatography using petroleum ether/ethyl acetate (0% to 50%), and **Y-5-1-5** (3.5 g, 31.4%) was obtained as a yellow solid. LCMS: (ES,m/z): 333.4 [M+1]⁺.

Under nitrogen protection, silver oxide (4.88 g, 21.1 mmol, 2 eq) was added to a solution of **Y-5-1-5** (3.5 g, 10.529 mmol, 1 eq) and benzyl bromide (18.01 g, 105.3 mmol, 10 eq) in dichloromethane (50 mL) at 50°C, and the reaction solution was stirred for 2 h. Complete reaction was detected by LC-MS. The resulting residue was concentrated in vacuum. The resulting residue was purified by silica gel column chromatography using petroleum ether/dichloromethane (0% to 50%), and **Y-5-1-6** (3.1 g, 69.7%) was obtained as a brown oil. LCMS: (ES,m/z): 423.5 [M+1]⁺.

Lithium hydroxide (476.14 mg, 19.9 mmol) was added to a solution of compound **Y-5-**1-6 (2.8 g, 6.6 mmol) and water (3 mL) in methanol (30 mL), and the reaction solution was stirred at 50°C for 3 h. Complete reaction was detected by LC-MS. The reaction mixture was quenched with ice water at 0°C. The reaction mixture was acidified to pH=4 with citric acid. The reaction mixture was extracted with ethyl acetate (3×100 mL). The organic phases were mixed and dried over sodium sulfate. The resulting mixture was filtered and the filtrate was concentrated under reduced pressure. **Y-5-1-7** (2 g, 73.8%) was obtained as a brown solid. LCMS: (ES,m/z): 409.5 [M+1]⁺.

Under nitrogen protection, N,N,N',N'-tetramethylchloroformamidinium hexafluorophosphate (5.22 g, 18.6 mmol) and N-methylimidazole (3.82 g, 46.5 mmol) were added in batches to a solution of compound **Y-5-1-7** (1.9 g, 4.6 mmol, 1 eq) and 6-(trifluoromethyl)pyridin-2-amine (754.03 mg, 4.65 mmol) in dichloromethane (30 mL) at 50°C, and the reaction solution was stirred overnight. Complete reaction was detected by LC-MS. The resulting residue was concentrated under reduced pressure. The resulting residue was purified by silica gel column chromatography using petroleum ether/ethyl acetate (0% to 50%), and **Y-5-1** (1.5 g, 58.4%) was obtained as a yellow solid. LCMS: (ES, m/z): 553.5 [M+1]⁺.

### Preparation of intermediate Y-5

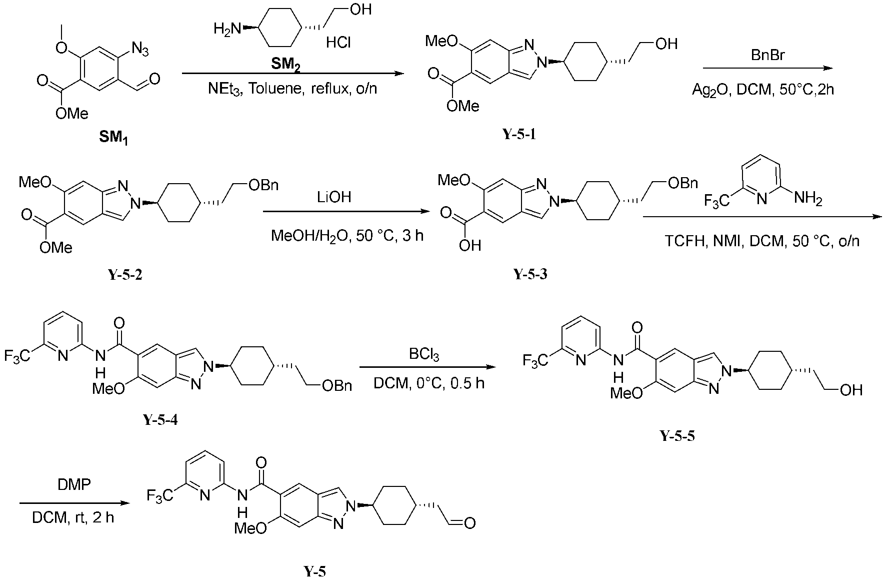

Under nitrogen protection, triethylamine (10.2 g, 100.9 mmol) was added to a solution of methyl 4-azido-5-formyl-2-methoxybenzoate (7.9 g, 33.62 mmol) and 2-[(1r,4r)-4-aminocyclohexyl]ethanol (5.77 g, 40.3 mmol) in toluene (200 mL) at 110°C, and the reaction solution was stirred overnight. Complete reaction was detected by LC-MS. The resulting residue was concentrated under reduced pressure. The reaction mixture was extracted with ethyl acetate (3×200 mL). The organic phases were mixed and dried over sodium sulfate. The resulting mixture was filtered and the filtrate was concentrated under reduced pressure. The resulting residue was purified by silica gel column chromatography using petroleum ether/ethyl acetate (0% to 50%), and **Y-5-1** (3.5 g, 31.4%) was obtained as a yellow solid. LCMS: (ES, m/z): 333.5 [M+1]⁺.

Under nitrogen protection, silver oxide (4.88 g, 21.1 mmol) was added to a solution of **Y-5-1** (3.5 g, 10.5 mmol) and benzyl bromide (18 g, 105.3 mmol) in dichloromethane (50 mL) at 50°C, and the reaction solution was stirred for 2 h. Complete reaction was detected by LC-MS. The resulting residue was concentrated in vacuum. The resulting residue was purified by silica gel column chromatography using petroleum ether/dichloromethane (0% to 50%), and **Y-5-2** (3.1 g, 69.7%) was obtained as a brown oil. LCMS: (ES, m/z): 423.5 [M+1]⁺.

Lithium hydroxide (476.2 mg, 19.9 mmol) was added to a solution of compound **Y-5-2** (2.8 g, 6.6 mmol) and water (3 mL) in methanol (30 mL), and the reaction solution was stirred at 50°C for 3 h. Complete reaction was detected by LC-MS. The reaction mixture was quenched with ice water at 0°C. The reaction mixture was acidified to pH=4 with citric acid. The reaction mixture was extracted with ethyl acetate (3×100 mL). The organic phases were mixed and dried over sodium sulfate. The resulting mixture was filtered and the filtrate was concentrated under reduced pressure. **Y-5-3** (2 g, 73.88%) was obtained as a brown solid. LCMS: (ES, m/z): 409.5 [M+1]⁺.

Under nitrogen protection, N,N,N',N'-tetramethylchloroformamidinium hexafluorophosphate (5.22 g, 18.6 mmol) and N-methylimidazole (3.82 g, 46.5 mmol) were added in batches to a solution of **Y-5-3** (1.9 g, 4.65 mmol) and 6-(trifluoromethyl)pyridin-2-amine (754.03 mg, 4.65 mmol) in dichloromethane (30 mL) at 50°C, and the reaction solution was stirred overnight. Complete reaction was detected by LC-MS. The resulting residue was concentrated under reduced pressure. The resulting residue was purified by silica gel column chromatography using petroleum ether/ethyl acetate (0% to 50%), and **Y-5-4** (1.5 g, 58.36%) was obtained as a yellow solid. LCMS: (ES, m/z): 553.5 [M+1]⁺.

Under nitrogen protection, boron trichloride (424.03 mg, 3.620 mmol, 5 equiv) was added to a solution of Y-5-4 (400 mg, 0.724 mmol, 1 equiv) in dichloromethane (5 mL) at 0°C. After the reaction solution was stirred for 30 min, complete reaction was detected by LC-MS. The reaction mixture was quenched with a saturated aqueous solution of sodium bicarbonate at 0°C. The reaction mixture was extracted with dichloromethane (3×10 mL). The organic phases were mixed and dried over anhydrous sodium sulfate. The resulting mixture was filtered and the filtrate was concentrated under reduced pressure. **Y-5-5** (300 mg, 89.6%) was obtained as a yellowish solid. LCMS (ESI, m/z): 463.1 [M+H]⁺.

Under nitrogen protection, Dess-Martin periodinane (275.14 mg, 0.649 mmol, 1.2 equiv) was added to a solution of **Y-5-5** (250 mg, 0.54 mmol, 1 equiv) in dichloromethane (5 mL) at room temperature. After the reaction solution was stirred for 2 h, complete reaction was detected by LC-MS. The reaction mixture was quenched with water at 0°C. The reaction mixture was extracted with dichloromethane (3×10 mL). The organic phases were mixed and dried over anhydrous sodium sulfate. The resulting mixture was filtered and the filtrate was concentrated under reduced pressure. Intermediate Y-5 (200 mg, 80.35%) was obtained as a yellow solid. LCMS (ESI, m/z): 461.5 [M+H]⁺.

### Preparation of intermediate Y-6

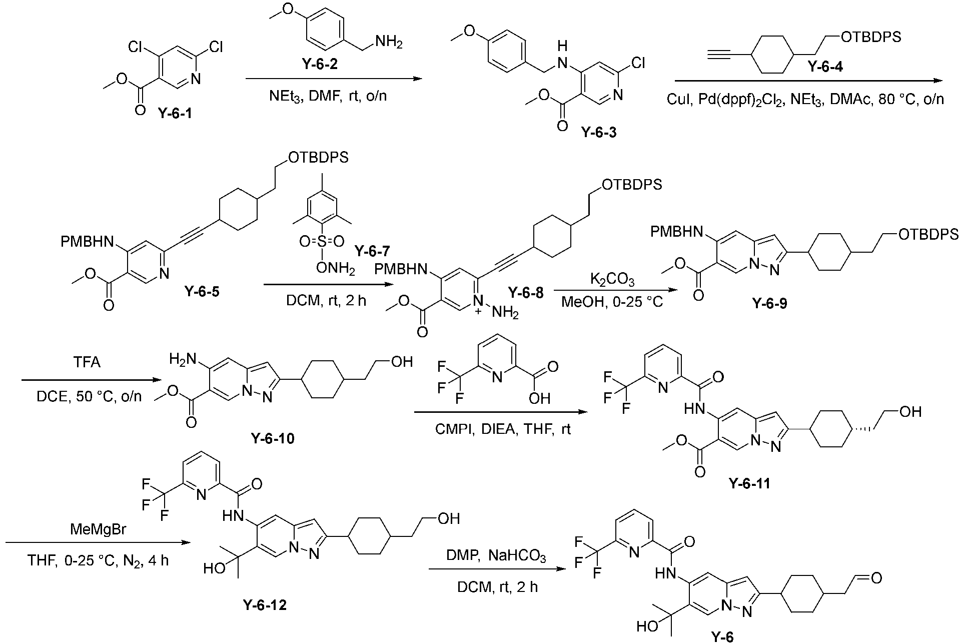

Triethylamine (20.85 mL, 149.98 mmol, 3 eq) was added to a solution **of Y-6-1** (10.3 g, 49.995 mmol, 1 eq) in dimethylformamide (100 mL) in an ice bath, then **Y-6-2** (8.23 g, 59.99 mmol, 1.2 eq) was slowly added dropwise, and the reaction solution was heated to room temperature and stirred for 2 h. Disappearance of the raw materials was detected by LC-MS. The resulting residue was concentrated under reduced pressure. The resulting residue was purified by silica gel column chromatography using ethyl acetate/petroleum ether (0% to 30%), and **Y-6-3** (14.3 g, 93.2%) was obtained as a white solid. LCMS (ESI, m/z): 307.0 [M+H]⁺.

Under nitrogen protection, **Y-6-4** (2.29 g, 5.868 mmol, 1.5 eq), [1,1-bis(diphenylphosphine)ferrocene]palladium chloride (286.24 mg, 0.391 mmol, 0.1 eq), cuprous iodide (149.01 mg, 0.782 mmol, 0.2 eq), and triethylamine (5.44 mL, 39.120 mmol, 10 eq) were added to a solution of **Y-6-3** (1.2 g, 3.91 mmol, 1 eq) in N,N-dimethylacetamide (19.6 mL) at room temperature, and then the reaction solution was heated to 80°C and stirred overnight. Complete reaction was detected by LC-MS. The reaction mixture was extracted with ethyl acetate (3×200 mL) and water (1×100 mL). The organic phases were mixed, backwashed with a saturated solution of sodium chloride (1×100 mL), and dried over anhydrous sodium sulfate. The resulting mixture was filtered and the filtrate was concentrated under reduced pressure. The resulting residue was purified by silica gel column chromatography using ethyl acetate/petroleum ether (0% to 50%), and **Y-6-5** (1.5 g, 58.0%) was obtained as a colorless oil. LCMS (ESI, m/z): 661.6 [M+H]⁺.

Amino 2,4,6-trimethylbenzenesulfonate (1.03 g, 4.767 mmol, 3 eq) was added to a solution **of Y-6-5** (1.05 g, 1.589 mmol, 1 eq) in dichloromethane (15 mL) at room temperature. After the reaction solution was stirred for 2 h, a product desired was found in LC-MS. The resulting residue was concentrated under reduced pressure. **Y-6-8** (1.3 g, crude) was obtained as a yellow oil. The crude product was directly used in the next step without being purified. LCMS (ESI, m/z): 676.6 [M+H]⁺.

Potassium carbonate (530.81 mg, 3.840 mmol, 2 eq) was added to a solution **of Y-6-8** (1.3 g, 1.920 mmol, 1 eq) in methanol (8 mL) at room temperature, and the reaction solution was stirred for 2 h. A product desired was found in LC-MS. The resulting residue was concentrated under reduced pressure. The resulting residue was purified by silica gel column chromatography using ethyl acetate/petroleum ether (0% to 30%), and **Y-6-9** (450 mg, 34.67%) was obtained as a yellowish oil. LCMS (ESI, m/z): 676.3 [M+H]⁺.

Trifluoroacetic acid (1.8 mL) was added to a solution **of Y-6-9** (360 mg, 0.533 mmol, 1 eq) in 1,2-dichloroethane (5.4 mL) at room temperature. The reaction solution was heated to 50°C and stirred overnight. A product desired was found in LC-MS. The resulting residue was concentrated under reduced pressure. Compound **Y-6-10** (220 mg, crude) was obtained as a light brown oil. LCMS (ESI, m/z): 318.5 [M+H]⁺.

N,N-diisopropylethylamine (0.25 mL, 1.452 mmol, 3 eq) and 2-chloro-1-methylpyridine-1-onium iodide (135.96 mg, 0.532 mmol, 1.1 eq) were added to a solution of compound **Y-6-10** (200 mg, 0.484 mmol, 1 eq) and 6-(trifluoromethyl)pyridine-2-carboxylic acid (73.97 mg, 0.387 mmol, 0.8 eq) in tetrahydrofuran (4.8 mL) in an ice bath. Following addition, the system was stirred at room temperature for 4 h. A product desired was found in LC-MS, and the raw materials substantially disappeared. The resulting residue was concentrated in vacuum and purified by silica gel column chromatography using ethyl acetate/petroleum ether (0% to 40%), and **Y-6-11** (150 mg, 63.21%) was obtained as a yellow solid. LCMS (ESI, m/z): 491.1 [M+H]⁺.

Under nitrogen protection, methyl magnesium bromide (1.1 mL, 25.83 mmol, 10 eq) was added dropwise to a solution of **Y-6-11** (150 mg, 0.306 mmol, 1 eq) in tetrahydrofuran (3 mL) at 0°C. Following addition, the system was stirred at 0°C for 0.5 h. Then, the system was heated to room temperature and stirred for 3 h. A product desired was found in LC-MS. The reaction mixture was poured into a saturated solution of ammonium chloride and quenched. Extraction was performed with ethyl acetate (3×30 mL). The organic phases were mixed and dried over anhydrous sodium sulfate. The resulting mixture was filtered and the filtrate was concentrated under reduced pressure. The resulting residue was purified by silica gel column chromatography using ethyl acetate: dichloromethane (0% to 50%), and **Y-6-12** (53 mg, 35.3%) was obtained as a yellow solid. LCMS (ESI, m/z): 491.5 [M+H]⁺.

Dess-Martin periodinane (64.85 mg, 0.153 mmol, 1.50 eq) was added to a solution of **Y-6-12** (50 mg, 0.102 mmol, 1 eq) in dichloromethane (5.0 mL) at room temperature. After reaction for 2 h, a product desired was found in LC-MS, and the raw materials disappeared. A saturated aqueous solution of sodium bicarbonate (20 mL) was added. The reaction mixture was extracted with dichloromethane (3×20 mL). The organic phases were mixed and dried over anhydrous sodium sulfate. The resulting mixture was filtered and the filtrate was concentrated under reduced pressure. Intermediate **Y-6** (35 mg, 70.3%) was obtained as a yellow solid. LCMS (ESI, m/z): 489.5 [M+H]⁺.

### Preparation of intermediate Y-7

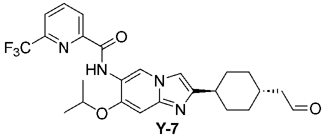

Intermediate **Y-7** was synthesized according to the prior art WO02023283372A1.

### Preparation of intermediate Y-8

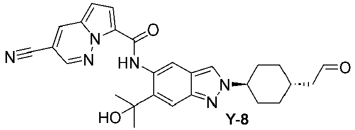

Compound 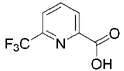 was replaced with 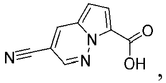 and intermediate **Y-8** was prepared by referring to compound **1-1** using a method disclosed in WO2020264499 A1.

### Preparation of intermediate Y-9

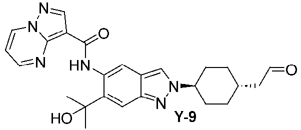

Compound 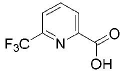 was replaced with 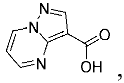 and intermediate **Y-9** was prepared by referring to compound **1-1** using a method disclosed in WO2020264499 A1.

### Preparation of intermediate Y-10

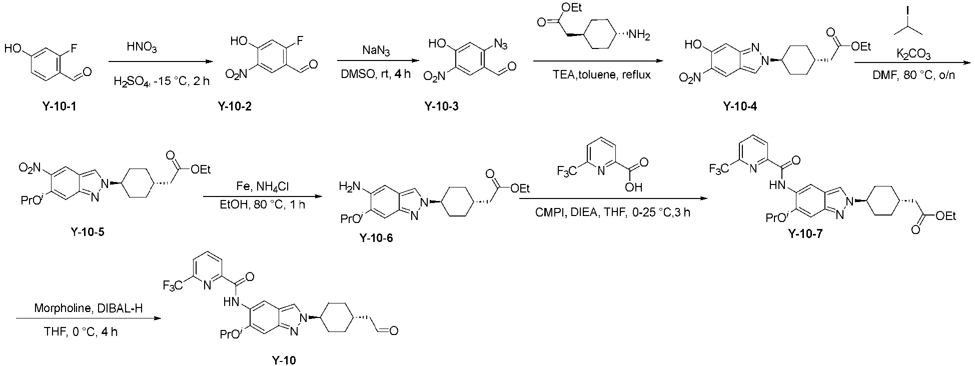

### Step 1: Synthesis of compound Y-10-2

A mixed solution of nitric acid (12 mL, 267.568 mmol, 1.50 eq) and nitric acid (12 mL, 267.568 mmol, 1.50 eq) was added dropwise to a solution of 2-fluoro-4-hydroxybenzaldehyde (25 g, 178.427 mmol, 1 eq) in sulfuric acid (150 mL) at 0°C. After the reaction solution was stirred for 2 h, complete reaction was detected by gas chromatography-mass spectrometry (GC-MS). The reaction solution was poured into ice water. A solid was precipitated and filtered, and the filter cake was collected and washed with water (4×100 mL). 2-fluoro-4-hydroxy-5-nitrobenzaldehyde compound **Y-10-2** (18 g, 54.50%) was obtained as a white-like solid. GCMS: 185.0 [M-1]⁺.

### Step 2: Synthesis of compound Y-10-3

Under nitrogen protection, sodium azide (10.54 g, 162.066 mmol, 2 eq) was added in batches to a solution of compound **Y-10-2** (15 g, 81.033 mmol, 1 eq) in dimethyl sulfoxide (80 mL) at room temperature. After the reaction solution was stirred for 4 h, complete reaction was detected by LC-MS. The reaction mixture was quenched with ice water (200 mL) at 0°C. The reaction mixture was extracted with ethyl acetate (4×200 mL). The organic phases were mixed, backwashed with saturated brine (2×100 mL), and dried over anhydrous sodium sulfate. The resulting mixture was filtered and the filtrate was concentrated under reduced pressure. 2-azido-4-hydroxy-5-nitrobenzaldehyde compound **Y-10-3** (8 g, 47.43%) was obtained as a yellow liquid. LCMS: (ESI, *m*/*z):* 206.9 [M-1]⁻.

### Step 3: Synthesis of compound Y-10-4

Under nitrogen protection, triethylamine (5.48 g, 48.046 mmol, 2 eq) was added dropwise to a solution of compound **Y-10-3** (5 g, 24.023 mmol, 1 eq) and ethyl 2-[(1r,4r)-4-aminocyclohexyl]acetate (4.45 g, 24.023 mmol, 1 eq) in toluene (50 mL). Following addition, the system was stirred at 110°C for 3 h. Complete reaction was detected by LC-MS. The resulting residue was concentrated under reduced pressure. The resulting residue was purified by silica gel column chromatography using petroleum ether/ethyl acetate (0% to 50%), and ethyl 2-[(1r,4r)-4-(6-hydroxy-5-nitroindazol-2-yl)cyclohexyl]acetate compound **Y-10-4** (2.5 g, 29.96%) was obtained as a yellow solid. LCMS: (ESI, *m*/*z*): 348.0 [M+1]⁺.

### Step 4: Synthesis of compound Y-10-5

Under nitrogen protection, potassium carbonate (1.99 g, 14.394 mmol, 2 eq) was added to a solution of compound **Y-10-4** (2.5 g, 7.197 mmol, 1 eq) and 2-iodopropane (2.45 g, 14.394 mmol, 2 eq) in N,N-dimethylformamide (30 mL) at 80°C, and the reaction solution was stirred overnight. Complete reaction was detected by LC-MS. The reaction mixture was poured into ice water. A solid was precipitated and filtered, and the filter cake was collected and washed with water (3×10 mL). Ethyl 2-[(1r,4r)-4-(6-isopropoxy-5-nitroindazol-2-yl)cyclohexyl]acetate compound **Y-10-5** (3 g, 107.03%) was obtained as a yellow solid. LCMS: (ESI, *m*/*z*): 390.1 [M+1]⁺.

### Step 5: Synthesis of compound Y-10-6

Under nitrogen protection, iron powder (1.20 g, 21.570 mmol, 3 eq) and water (7 mL, 388.565 mmol, 54.05 eq) were added to a solution of compound **Y-10-5** (2.8 g, 7.190 mmol, 1 eq) and ammonium chloride (0.38 g, 7.190 mmol, 1 eq) in ethanol (35 mL) at 80°C and the reaction solution was stirred for 1 h. Complete reaction was detected by LC-MS. The resulting mixture was filtered, the filter cake was washed with ethanol (3×30 mL), and the filtrate was concentrated under reduced pressure. The reaction mixture was extracted with ethyl acetate (3×50 mL). The organic phases were mixed, backwashed with saturated brine (1×100 mL), and dried over anhydrous sodium sulfate. The resulting mixture was filtered and the filtrate was concentrated under reduced pressure. Ethyl 2-[(1r,4r)-4-(5-amino-6-isopropoxyindol-2-yl)cyclohexyl]acetate compound **Y-10-6** (2.4 g, 92.86%) was obtained as a yellowish solid. LCMS: (ESI, *m*/*z*): 360.1 [M+1]⁺.

### Step 6: Synthesis of compound Y-10-7

Under nitrogen protection, N,N-diisopropylethylamine (1.73 g, 13.352 mmol, 2 eq) was added dropwise to a solution of compound **Y-10-6** (2.4 g, 6.676 mmol, 1 eq) and 6-(trifluoromethyl)pyridine-2-carboxylic acid (1.28 g, 6.676 mmol, 1 eq) in tetrahydrofuran (20 mL). After the reaction solution was stirred for 10 min, 2-chloro-1-methylpyridine-1-iodonium (1.88 g, 7.344 mmol, 1.1 eq) was added dropwise at 0°C. Following addition, the system was stirred at room temperature for 3 h. Complete reaction was detected by LC-MS. The resulting residue was concentrated under reduced pressure. The resulting residue was purified by silica gel column chromatography using petroleum ether/ethyl acetate (0% to 50%), and ethyl 2-[(1r,4r)-4-(6-isopropoxy-5-[6-(trifluoromethyl)pyridin-2-amino]indazol-2-ylcyclohexyl]acetate compound **Y-10-7** (2.1 g, 59.06%) was obtained as a yellow solid.

LCMS: (ESI, *m*/*z*): 533.10 [M+1]⁺.

### Step 7: Synthesis of compound Y-10

Under nitrogen protection, DIBAL-H (2.1 mL, 10.348 mmol, 5.51 eq) was added to a solution of morpholine (687.06 mg, 7.888 mmol, 4.2 eq) in tetrahydrofuran (20.0 mL) at 0°C. After the reaction solution was stirred for 1 h, compound **Y-10-7** (1 g, 1.878 mmol, 1 eq) was added dropwise at 0°C. After the reaction solution was stirred for 1 h, complete reaction was detected by LC-MS. The resulting residue was concentrated under reduced pressure. The resulting residue was purified by silica gel column chromatography using petroleum ether/ethyl acetate (0% to 50%), and N-(6-isopropoxy-2-[(1r,4r)-4-(2-oxyethyl)cyclohexyl]indazol-5-yl-6-(trifluoromethyl)pyridine-2-formamide compound **Y-10** (300 mg, 32.71%) was obtained as a white-like solid.

### LCMS: (ESI, m/z): 487.05 [M+1]⁺.

General preparation method I of the compounds as described in the present application:

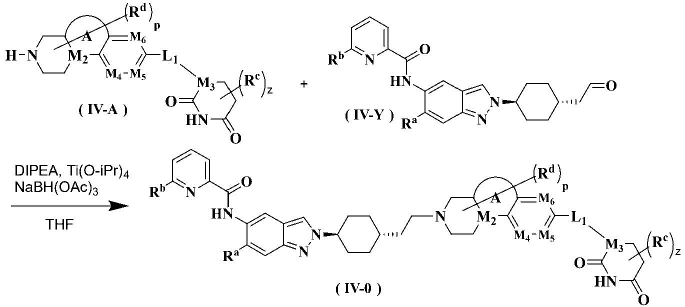

A compound of formula IV-A and a compound of formula IV-Y were allowed to undergo reduction amination to yield a compound of formula IV-0.

General preparation method II of the compounds as described in the present application:

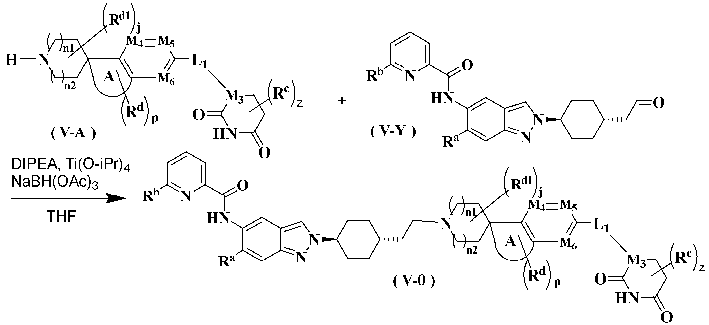

A compound of formula V-A and a compound of formula V-Y were allowed to undergo reduction amination to yield a compound of formula V-0.

Chiral compounds in the present application may be separated under conventional chiral separation conditions in the art (e.g., chromatography column specification: CHIRALPAK-IA 2*25 cm, 5 µm; mobile phase A: n-hexane (0.1% diethylamine), mobile phase B: methanol: dichloromethane=1: 1; flow rate: 18 mL/min; elution gradient: isocratic 70; detection wavelength: UV 254/220 nm. The specification of the chromatography column may be selected as needed, and the elution gradient, the flow rate, and the like may be adjusted as needed. As an alternative preparation method, chiral synthesis may also be conducted using chiral raw materials.

### Example 1 Preparation of compound 1

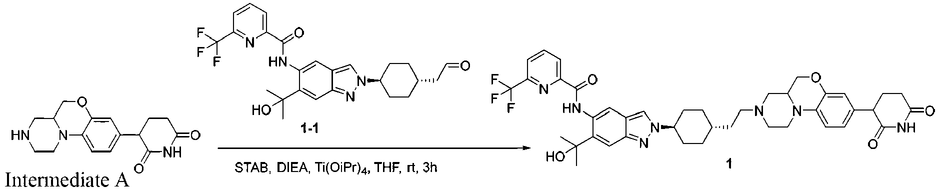

*N,N*-diisopropylethylamine (27 mg, 0.209 mmol, 2.10 equiv), compound **1-1** (35 mg, 0.072 mmol, 0.72 equiv), and tetraisopropyl titanate (85 mg, 0.299 mmol, 3.00 equiv) were added to a solution of intermediate **A** (30 mg, 0.100 mmol, 1 equiv) in tetrahydrofuran (1 mL) at room temperature. Following addition, the system was stirred at room temperature for 1 h. Sodium triacetoxyborohydride (30 mg, 0.142 mmol, 1.42 equiv) was added to the system at 0°C. Following addition, the system was stirred at room temperature for 2 h. A product desired was found in LC-MS. The crude product was purified by high-performance liquid chromatography to obtain compound **1** (7.6 mg, 9.68%) under the following conditions (chromatography column specification: XBridge BEH Shield RP18 5 um, 30 mm *150 mm; mobile phase A: water (10 mmol/L ammonium bicarbonate), mobile phase B: acetonitrile; flow rate: 60 mL/min; elution gradient: 38% B to 62% B in 10 min; detection wavelength: UV 254 nm/220 nm; retention time (min): 9.43).

LCMS: (ESI, *m*/*z*): 774.4 [M+H]⁺.

¹H NMR: (400 MHz, DMSO-d₆) δ 12.36 (s, 1H), 10.76 (s, 1H), 8.71 (s, 1H), 8.45 (d, J = 7.7 Hz, 1H), 8.36 (t, J = 7.8 Hz, 2H), 8.16 (d, J = 8.2 Hz, 1H), 7.57 (s, 1H), 6.80 (d, J = 8.6 Hz, 1H), 6.63 (q, J = 3.4 Hz, 1H), 6.55 (d, J = 1.8 Hz, 1H), 5.94 (s, 1H), 4.42 (t, J = 11.6 Hz, 1H), 4.24 (q, J = 4.3 Hz, 1H), 3.88 (t, J = 9.8 Hz, 1H), 3.68 (m, J = 5.3 Hz, 2H), 2.96 (q, J = 10.8 Hz, 3H), 2.61 (m, J = 5.4 Hz, 2H), 2.46 (d, J = 4.7 Hz, 1H), 2.41 (m, J = 5.3 Hz, 2H), 2.10 (q, J = 6.0 Hz, 1H), 1.95 (m, J = 7.6 Hz, 5H), 1.68 (t, J = 10.7 Hz, 1H), 1.62 (s, 6H), 1.45 (s, 3H), 1.21 (t, J = 10.4 Hz, 2H).

### Example 2 Synthesis of compounds 120-a, 120-a-1, and 120-a-2

Intermediate **1-1** and intermediate **Q** were selected, and compound **120-a** was prepared according to general preparation method I.

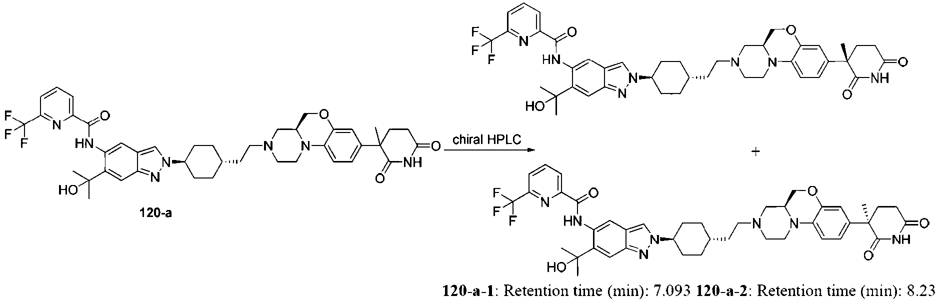

20.7 mg of compound **12-a** was chirally separated, and 6.1 mg of **120-a-1** and 6.5 mg of **120-a-2** were obtained under the following conditions (chromatography column specification: CHIRAL ART Cellulose-SB 3*25 cm, 5 um; mobile phase A: n-hexane (0.1% diethylamine), mobile phase B: ethanol: dichloromethane=1: 1; flow rate: 40 mL/min; elution gradient: isocratic 50; detection wavelength: UV 254/220 nm; retention time (min) of **120-a-1:** 7.093; retention time (min) of postpeak **120-a-2:** 8.23; sample solvent: ethanol; single injection volume: 1.0 mL; number of injections: 3). **LCMS-120-a-1** (ESI, *m*/*z*): 788.1 [M+H]⁺. **LCMS-120-a-2** (ESI, *m*/*z*): 787.9 [M+H]⁺.

¹HNMR **120-a-1:** (400 MHz, DMSO-*d*₆, *ppm*) *δ* 12.54 (s, 1H), 10.82 (s, 1H), 9.06 (s, 1H), 8.58 (d, *J =* 0.7 Hz, 1H), 8.50-8.44(m, 2H), 8.39 (t, *J =* 7.9 Hz, 1H), 8.21 (dd, *J =* 0.9, 7.8 Hz, 1H), 6.83 (d, *J =* 8.7 Hz, 1H), 6.68 (dd, *J =* 2.2, 8.5 Hz, 1H), 6.60 (d, *J =* 2.3 Hz, 1H), 4.59-4.48 (m, 1H), 4.24 (dd, *J =* 2.6, 10.5 Hz, 1H), 3.89 (dd, *J =* 9.3, 10.3 Hz, 3H), 3.68 (d, *J =* 11.1 Hz, 1H), 3.30-3.27 (m, 1H), 3.07-2.95 (m, 3H), 2.92 (d, *J* = 10.2 Hz, 1H), 2.65-2.59 (m, 2H), 2.48- 2.41 (m, 2H), 2.41-2.34 (d, *J* = 18.2 Hz, 2H), 2.31-2.24 (m, 1H), 2.22-2.14 (m, 2H), 2.13-2.00 (m, 3H), 2.00-1.87 (m, 4H), 1.71-1.64 (m, 1H), 1.52-1.41 (m, 3H), 1.36 (s, 3H), 1.31-1.13(m, *J =* 7.5 Hz, 3H).

¹HNMR **120-a-2:** (400 MHz, DMSO-*d*₆, *ppm*) δ 12.54 (s, 1H), 10.83 (s, 1H), 9.06 (s, 1H), 8.58 (s, 1H), 8.49-8.44 (m, 2H), 8.39 (t, *J =* 7.8 Hz, 1H), 8.21 (d, *J =* 7.7 Hz, 1H), 6.83 (d, *J* = 8.7 Hz, 1H), 6.68 (dd, *J =* 2.2, 8.5 Hz, 1H), 6.60 (d, *J =* 2.3 Hz, 1H), 4.58-4.48 (m, 1H), 4.24 (dd, *J* = 2.0, 10.6 Hz, 1H), 3.96 (s, 3H), 3.89 (t, *J =* 9.9 Hz, 1H), 3.68 (d, *J* = 10.2 Hz, 1H), 3.30-3.28 (m, 1H), 3.06-2.90 (m, 3H), 2.64-2.59 (m, 1H), 2.49-2.45 (m, 2H), 2.45-2.41 (m, 1H), 2.41-2.36 (m, 2H), 2.31-2.23 (m, 1H), 2.21-2.13 (m, 2H), 2.13-2.08 (m, 1H), 2.080-2.00(m, 2H), 2.00-1.88 (m, 4H), 1.74-1.61 (m, 1H), 1.51-1.40 (m, 3H), 1.36 (s, 3H), 1.29-1.14 (m, *J =* 5.2 Hz, 3H).

### Example 3 Preparation of compounds 120-b, 120-b-1, and 120-b-2

Intermediate **1-1** and intermediate **P** were selected, and compound **120-b** was prepared according to general preparation method I. 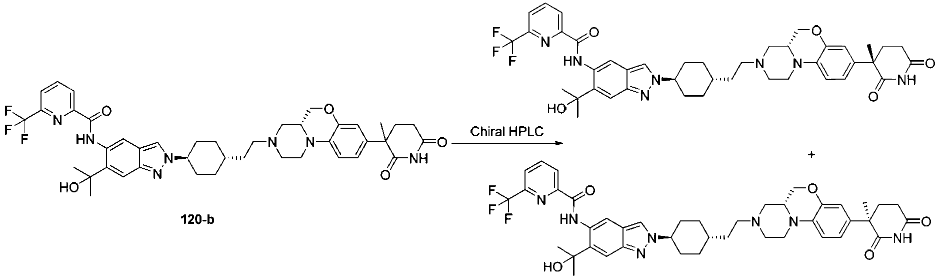 **120-b-1:** Retention time (min): 8.716 **120-b-2:** Retention time (min): 11.828

65 mg of compound **120-b** was chirally separated, and 21.7 mg of **120-b-1** and 22.5 mg of **120-b-2** were obtained under the following conditions (chromatography column specification: CHIRAL ART Cellulose-SB 2*25 cm, 5 um; mobile phase A: n-hexane (0.1% diethylamine), mobile phase B: ethanol: dichloromethane=1: 1; flow rate: 20 mL/min; elution gradient: isocratic 40; detection wavelength: UV 254/220 nm; retention time 1 (min) of **120-b-1:** 8.716; retention time 2 (min) of **120-b-2:** 11.828; sample solvent: ethanol + dichloromethane; injection volume: 1.0 mL; number of runs: 7).

**LCMS-120-b-1:** (ESI, *m*/*z*): 788.25 [M+H]⁺.

**LCMS-120-b-2:** (ESI, *m*/*z*): 788.25 [M+H]⁺.

¹HNMR-**120-b-1:** (400 MHz, DMSO-*d*₆, *ppm*) δ 12.36 (s, 1H), 10.82 (s, 1H), 8.71 (s, 1H), 8.45 (d, J = 7.7 Hz, 1H), 8.39-8.31(m, 2H), 8.16 (q, J = 2.8 Hz, 1H), 7.57 (s, 1H), 6.83 (d, J = 8.7 Hz, 1H), 6.68 (q, J = 3.6 Hz, 1H), 6.64-6.46 (m, 1H), 5.93 (s, 1H), 4.51-4.35 (m, 1H), 4.24 (q, J = 4.4 Hz, 1H), 3.92-3.87 (m, 1H), 3.68 (d, J = 11.4 Hz, 1H), 3.04-2.96 (m, 3H), 2.66-2.62 (m, 1H), 2.44-2.36 (m, 2H), 2.32-2.26 (m, 1H), 2.17-2.01 (m, 5H), 1.95-1.86 (m, 4H), 1.66 (t, J = 10.7 Hz, 1H), 1.62 (s, 6H), 1.46-1.42 (m, 3H), 1.36 (s, 3H), 1.22-1.18 (m, 3H).

¹H NMR**-120-b-2:** (400 MHz, DMSO-*d*₆, ppm) δ 12.36 (s, 1H), 10.83 (s, 1H), 8.71 (s, 1H), 8.45 (d, J = 7.8 Hz, 1H), 8.40-8.33(m, 2H), 8.16 (d, J = 8.0 Hz, 1H), 7.57 (s, 1H), 6.83 (d, J = 8.6 Hz, 1H), 6.69-6.56 (m, 2H), 5.93 (s, 1H), 4.49-4.37(m, 1H), 4.24 (dd, J = 2.5, 10.6 Hz, 1H), 3.89 (t, J = 9.8 Hz, 1H), 3.68 (d, J = 11.1 Hz, 1H), 3.06-2.90 (m, 3H), 2.66-2.57(m, 1H), 2.41-2.36 (m, 2H), 2.30-2.23 (m, 1H), 2.17-2.01 (m, 5H), 1.960-1.86 (m, 4H), 1.66 (t, J = 10.6 Hz, 1H), 1.62 (s, 6H), 1.45 (t, J = 6.2 Hz, 3H), 1.36 (s, 3H), 1.19 (m, J = 6.3 Hz, 3H).

### Example 4 Synthesis of compound 126

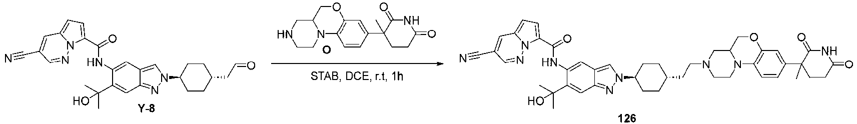

Intermediate **Y-8** and intermediate **O** were selected, and compound **126** (16.6mg, yield 22.8%) was prepared according to general preparation method I. LCMS: (ESI, *m*/*z*): 784.4 [M+1]⁺.

¹H NMR (400 MHz, DMSO-d6) δ 12.03 (s, 1H), 10.84 (s, 1H), 8.94 (d, J = 2.1 Hz, 1H), 8.75 (d, J = 2.1 Hz, 1H), 8.55 (s, 1H), 8.34 (s, 1H), 7.72 (d, J = 4.8 Hz, 1H), 7.57 (s, 1H), 7.09 (d, J = 4.8 Hz, 1H), 6.83 (s, 1H), 6.75 - 6.57 (m, 2H), 5.74 (s, 1H), 4.50 - 4.35 (m, 1H), 4.25 (d, J = 8.7 Hz, 1H), 3.89 (t, J = 9.5 Hz, 1H), 3.77 - 3.64 (m, 1H), 3.01 (s, 3H), 2.71 - 2.60 (m, 1H), 2.45 - 2.36 (m, 3H), 2.28 (d, J = 8.3 Hz, 1H), 2.17 - 2.00 (m, 5H), 1.94 - 1.88 (m, 4H), 1.63 (s, 6H), 1.45 (s, 3H), 1.35 (s, 3H), 1.28 - 1.14 (m, 3H).

### Example 5 Synthesis of compound 128

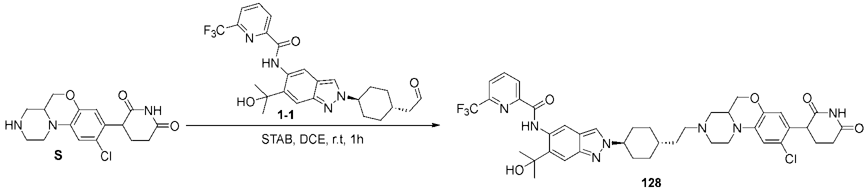

Intermediate **1-1** and intermediate S were selected, and compound **128** (71 mg, yield 39.9%) was prepared according to general preparation method I. LCMS: (ESI, *m*/*z*): 708.3 [M+1]⁺.

¹H NMR (400 MHz, DMSO-d6) δ 12.36 (s, 1H), 10.82 (d, J = 2.7 Hz, 1H), 8.71 (s, 1H), 8.45 (d, J = 7.8 Hz, 1H), 8.40 - 8.32 (m, 2H), 8.16 (d, J = 7.8 Hz, 1H), 7.57 (s, 1H), 6.89 (s, 1H), 6.63 (s, 1H), 5.94 (s, 1H), 4.42 (s, 1H), 4.27 (dd, J = 10.6, 2.1 Hz, 1H), 3.99 (dd, J = 12.2, 4.9 Hz, 1H), 3.93 - 3.82 (m, 1H), 3.72 (d, J = 11.6 Hz, 1H), 3.05 (s, 1H), 2.95 (t, J = 12.3 Hz, 2H), 2.77 - 2.61 (m, 2H), 2.39 (d, J = 7.7 Hz, 2H), 2.29 - 2.17 (m, 1H), 2.10 (dd, J = 18.7, 5.7 Hz, 3H), 1.91 (d, J = 11.0 Hz, 4H), 1.74 - 1.54 (m, 8H), 1.44 (s, 3H), 1.19 (dd, J = 20.7, 8.8 Hz, 3H).

### Example 6 Synthesis of compound 129

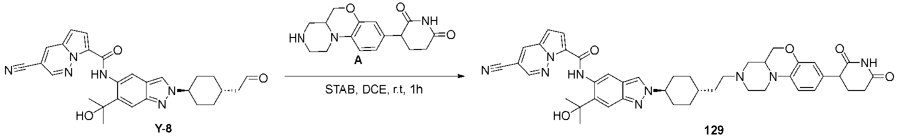

Intermediate **Y-8** and intermediate **A** were selected, and compound **129** (12 mg, yield 16.8%) was prepared according to general preparation method I. LCMS: (ESI, *m*/*z*): 770.4 [M+1]⁺.

¹H NMR (400 MHz, DMSO-d6) δ 12.02 (s, 1H), 10.76 (s, 1H), 8.93 (d, J = 2.2 Hz, 1H), 8.74 (d, J = 2.2 Hz, 1H), 8.55 (s, 1H), 8.34 (s, 1H), 7.72 (d, J = 4.8 Hz, 1H), 7.57 (s, 1H), 7.09 (d, J = 4.8 Hz, 1H), 6.80 (d, J = 8.5 Hz, 1H), 6.63 (dd, J = 8.4, 1.8 Hz, 1H), 6.55 (d, J = 1.8 Hz, 1H), 5.72 (s, 1H), 4.42 (t, J = 11.6 Hz, 1H), 4.24 (dd, J = 10.6, 2.5 Hz, 1H), 3.92 - 3.83 (m, 1H), 3.74 - 3.59 (m, 2H), 3.08 - 2.87 (m, 3H), 2.68 - 2.55 (m, 2H), 2.48 - 2.35 (m, 3H), 2.19 - 1.85 (m, 9H), 1.68 (s, 1H), 1.63 (s, 6H), 1.45 (s, 3H), 1.29 - 1.11 (m, 2H).

### Example 7 Synthesis of compound 130

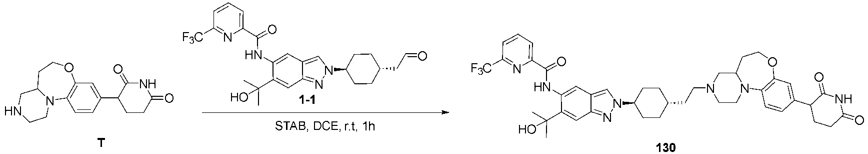

Intermediate **1-1** and intermediate **T** were selected, and compound **130** (9.2 mg, yield 14.7%) was prepared according to general preparation method I. LCMS: (ESI, m/z): 788.4 [M+1]⁺.

¹H NMR (400 MHz, DMSO-d6) δ 12.42 (s, 1H), 10.84 (s, 1H), 8.77 (s, 1H), 8.58 - 8.37 (m, 3H), 8.22 (d, J = 7.8 Hz, 1H), 7.64 (s, 1H), 6.97 (d, J = 8.3 Hz, 1H), 6.81 (d, J = 7.9 Hz, 1H), 6.68 (s, 1H), 6.01 (s, 1H), 4.47 (d, J = 14.1 Hz, 2H), 4.23 - 4.11 (m, 1H), 3.77 (dd, J = 10.9, 4.8 Hz, 1H), 3.19 (s, 3H), 2.82 - 2.62 (m, 4H), 2.49 (s, 1H), 2.39 (s, 1H), 2.19 (d, J = 10.1 Hz, 3H), 2.11 - 1.93 (m, 8H), 1.68 (s, 6H), 1.51 (s, 3H), 1.30 (s, 3H).

### Example 8 Synthesis of compound 131

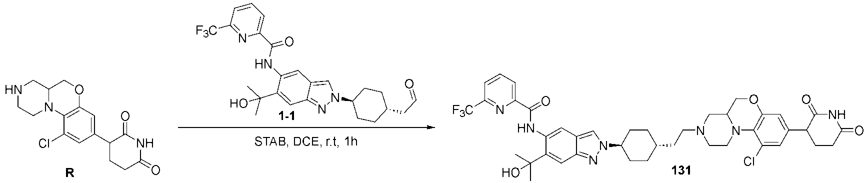

Intermediate **1-1** and intermediate **R** were selected, and compound **131** (29.4 mg, yield 25.0%) was prepared according to general preparation method I. LCMS: (ESI, *m*/*z*): 708.3 [M+1]⁺.

¹H NMR (400 MHz, DMSO-d6) δ 12.37 (s, 1H), 10.82 (s, 1H), 8.72 (s, 1H), 8.45 (d, J = 7.7 Hz, 1H), 8.42 - 8.30 (m, 2H), 8.16 (d, J = 7.8 Hz, 1H), 7.58 (s, 1H), 6.84 (s, 1H), 6.71 (d, J = 1.7 Hz, 1H), 5.94 (s, 1H), 4.41 (t, J = 10.4 Hz, 2H), 4.16 (dd, J = 10.5, 2.3 Hz, 1H), 3.78 (dd, J = 11.9, 4.7 Hz, 1H), 3.41 (d, J = 11.4 Hz, 1H), 3.11 (d, J = 9.8 Hz, 1H), 2.95 (d, J = 11.5 Hz, 1H), 2.92 - 2.77 (m, 2H), 2.70 - 2.56 (m, 1H), 2.43 (d, J = 8.4 Hz, 1H), 2.33 (s, 2H), 2.22 (t, J = 10.6 Hz, 2H), 2.12 (d, J = 9.5 Hz, 2H), 2.04 - 1.85 (m, 5H), 1.62 (s, 6H), 1.42 (s, 3H), 1.28 - 1.13 (m, 3H).

According to the preparation method of Example 1 (selecting corresponding intermediates and according to general preparation method I), the following compounds were prepared:

| Compound | Structure | MS m/z (ESI) |
|---|---|---|
| 1-a | 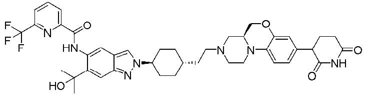 | 774.2 [M+H]⁺ |
| 1-b | 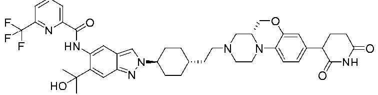 | 774.2 [M+H]⁺ |
| 2 | 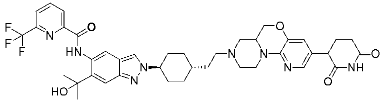 | 775.4 [M+H]⁺ |
| 3 | 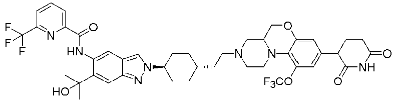 | 858.4 [M+H]⁺ |
| 4 | 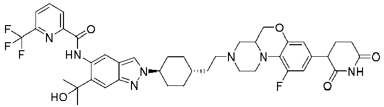 | 792.3 [M+H]⁺ |
| 4-a | 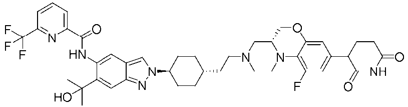 | 792.2 [M+H]⁺ |
| 4-b | 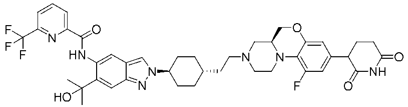 | 792.2 [M+H]⁺ |
| 5 | 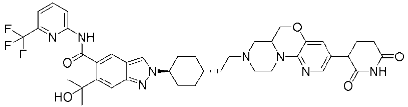 | 775.4 [M+H]⁺ |
| 6 | 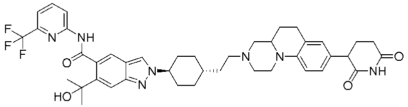 | 772.4 [M+H]⁺ |
| 7 | 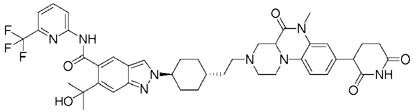 | 801.4 [M+H]⁺ |
| 8 | 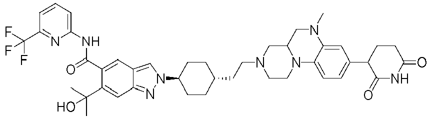 | 787.4 [M+H]⁺ |
| 9 | 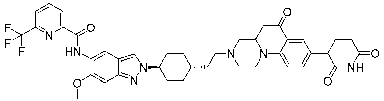 | 758.3 [M+H]⁺ |
| 10 | 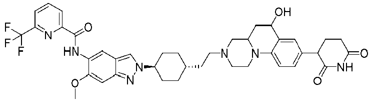 | 760.3 [M+H]⁺ |
| 11 | 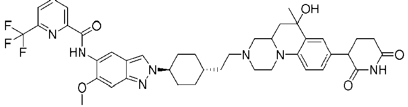 | 774.4 [M+H]⁺ |
| 12 | 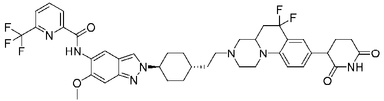 | 780.3 [M+H]⁺ |
| 13 | 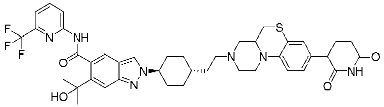 | 790.3 [M+H]⁺ |
| 14 | 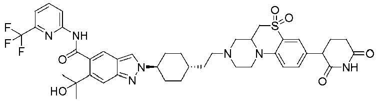 | 822.3 [M+H]⁺ |
| 15 | 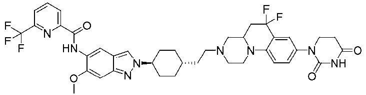 | 781.3 [M+H]⁺ |
| 16 | 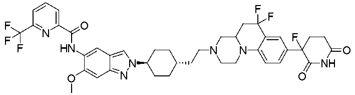 | 798.3 [M+H]⁺ |
| 17 | 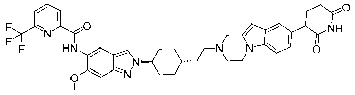 | 728.3 [M+H]⁺ |
| 18 | 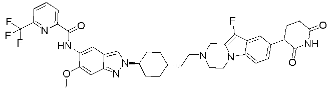 | 746.3 [M+H]⁺ |
| 19 | 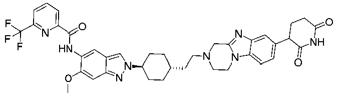 | 729.3 [M+H]⁺ |
| 20 | 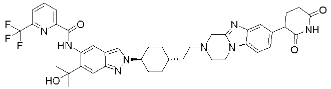 | 757.3 [M+H]⁺ |
| 21 | 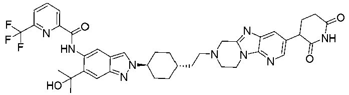 | 758.3 [M+H]⁺ |
| 22 | 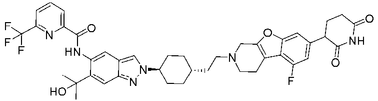 | 775.3 [M+H]⁺ |
| 23 | 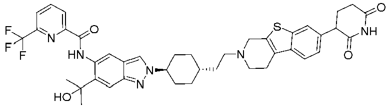 | 773.3 [M+H]⁺ |
| 24 | 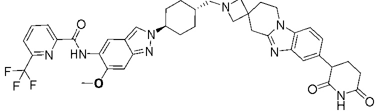 | 755.3 [M+H]⁺ |
| 25 | 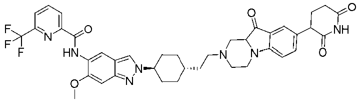 | 744.3 [M+H]⁺ |
| 26 | 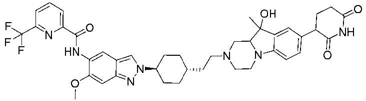 | 760.3 [M+H]⁺ |
| 27 | 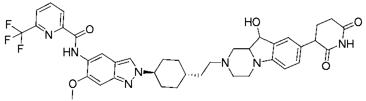 | 746.3 [M+H]⁺ |
| 28 | 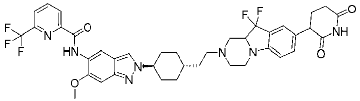 | 766.3 [M+H]⁺ |
| 29 | 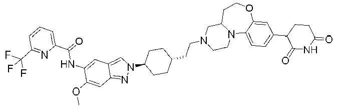 | 760.3 [M+H]⁺ |
| 30 | 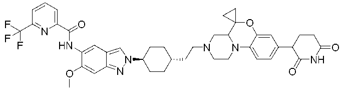 | 772.3 [M+H]⁺ |
| 31 | 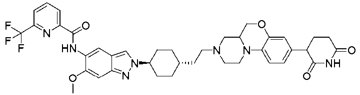 | 746.3 [M+H]⁺ |
| 32 | 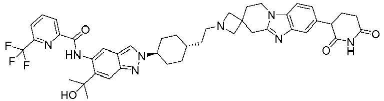 | 797.4 [M+H]⁺ |
| 33 | 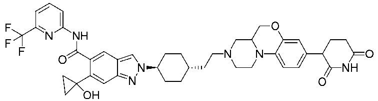 | 772.3 [M+H]⁺ |
| 34 | 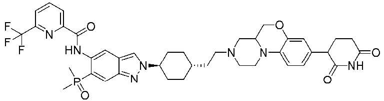 | 792.3 [M+H]⁺ |
| 35 | 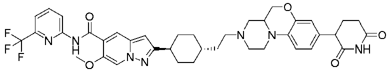 | 746.3 [M+H]⁺ |
| 36 | 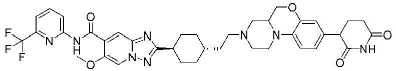 | 747.3 [M+H]⁺ |
| 37 | 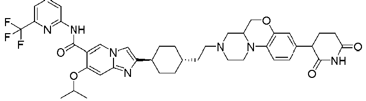 | 774.4 [M+H]⁺ |
| 38 | 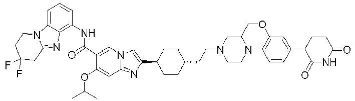 | 835.4 [M+H]⁺ |
| 39 | 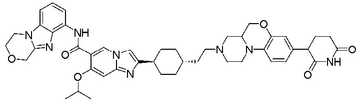 | 801.4 [M+H]⁺ |
| 40 | 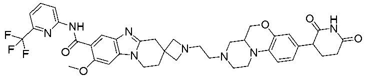 | 759.4 [M+H]⁺ |
| 41 | 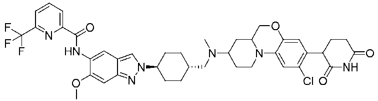 | 794.3 [M+H]⁺ |
| 42 | 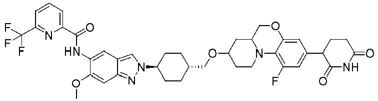 | 765.3 [M+H]⁺ |
| 43 | 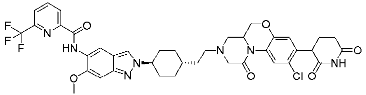 | 794.3 [M+H]⁺ |
| 44 | 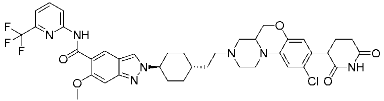 | 780.3 [M+H]⁺ |
| 45 | 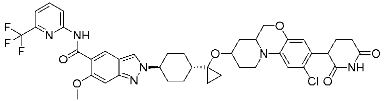 | 807.3 [M+H]⁺ |
| 95 | 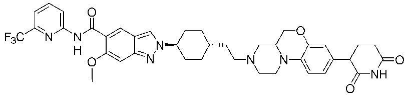 | 746.3 [M+H]⁺ |
| 98 | 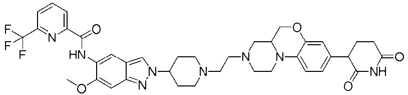 | 746.9 [M+H]⁺ |
| 99 | 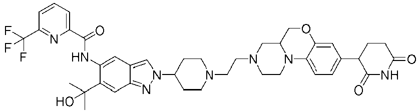 | 775.2 [M+H]⁺ |
| 100 | 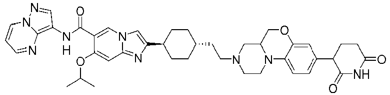 | 745.9 [M+H]⁺ |
| 101 | 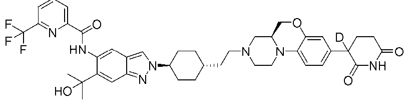 | 774.7 [M+H]⁺ |
| 102 | 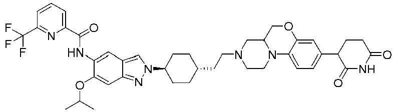 | 774.4 [M+H]⁺ |
| 104 | 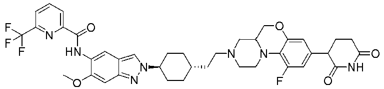 | 764.2 [M+H]⁺ |
| 105 | 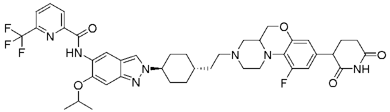 | 773.1 [M+H]⁺ |
| 107 | 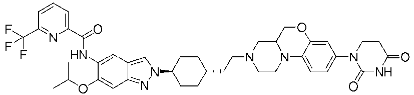 | 746.3 [M+H]⁺ |
| 108 | 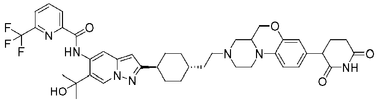 | 774.2 [M+H]⁺ |
| 109 | 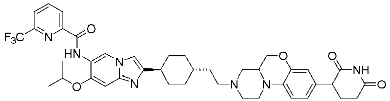 | 774.3 [M+H]⁺ |
| 111 | 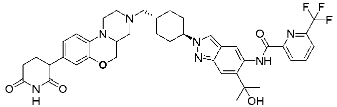 | 760.0 [M+H]⁺ |
| 112 | 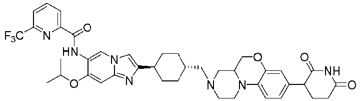 | 760.9 [M+H]⁺ |
| 113 | 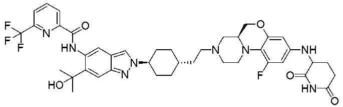 | 807.7 [M+H]⁺ |
| 115 | 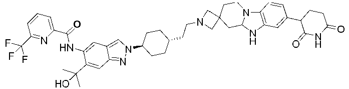 | 799.4 [M+H]⁺ |
| 116 | 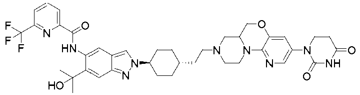 | 776.3 [M+H]⁺ |
| 117 | 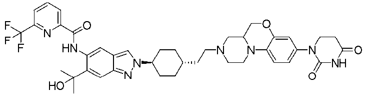 | 775.4 [M+H]⁺ |
| 118 | 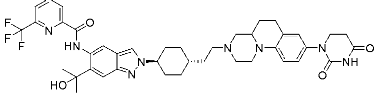 | 773.2 [M+H]⁺ |
| 119 | 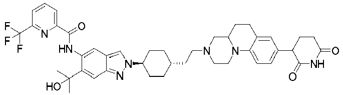 | 772.4 [M+H]⁺ |
| 120 | 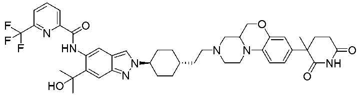 | 788.4 [M+H]⁺ |
| 122-a | 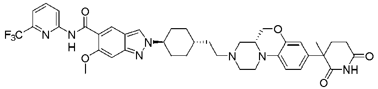 | 760.3 [M+H]⁺ |
| 122-a-1 | 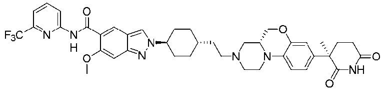 | 760.3 [M+H]⁺ |
| 122-a-2 | 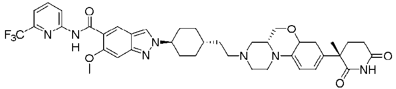 | 760.3 [M+H]⁺ |
| 122-b | 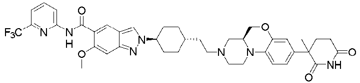 | 760.3 [M+H]⁺ |
| 122-b-1 | 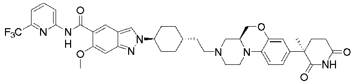 | 760.3 [M+H]⁺ |
| 122-b-2 | 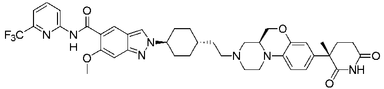 | 760.3 [M+H]⁺ |
| 123-a | 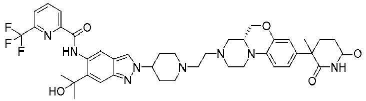 | 789.4 [M+H]⁺ |
| 123-a-1 | 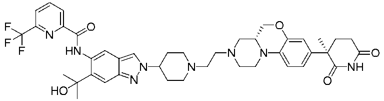 | 789.4 [M+H]⁺ |
| 123-a-1 | 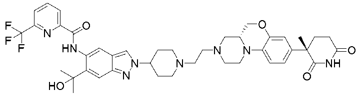 | 789.4 [M+H]⁺ |
| 123-b | 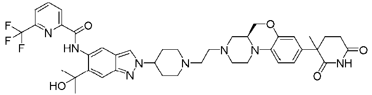 | 789.4 [M+H]⁺ |
| 123-b-1 | 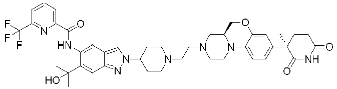 | 789.4 [M+H]⁺ |
| 123-b-2 | 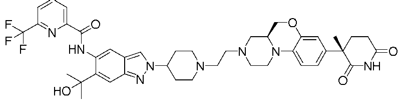 | 789.4 [M+H]⁺ |
| 124-a | 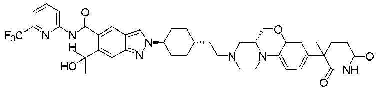 | 788.4 [M+H]⁺ |
| 124-a-1 | 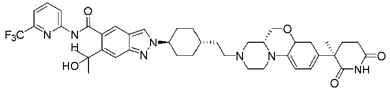 | 788.4 [M+H]⁺ |
| 124-a-2 | 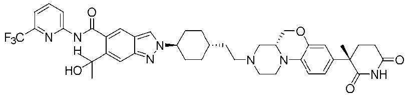 | 788.4 [M+H]⁺ |
| 124-b | 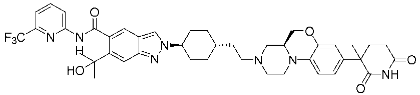 | 788.4 [M+H]⁺ |
| 124-b-1 | 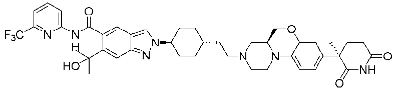 | 788.4 [M+H]⁺ |
| 124-b-2 | 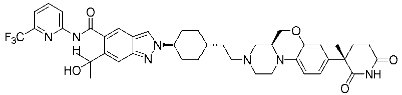 | 788.4 [M+H]⁺ |
| 125 | 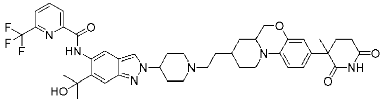 | 788.4 [M+H]⁺ |
| 126-a | 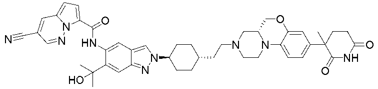 | 784.9 [M+H]⁺ |
| 126-a-1 | 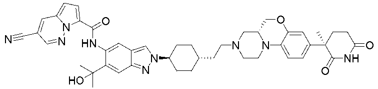 | 784.9 [M+H]⁺ |
| 126-a-2 | 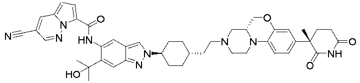 | 784.9 [M+H]⁺ |
| 126-b | 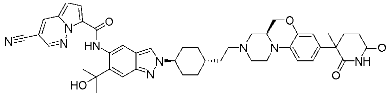 | 784.9 [M+H]⁺ |
| 126-b-1 | 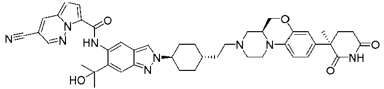 | 784.9 [M+H]⁺ |
| 126-b-2 | 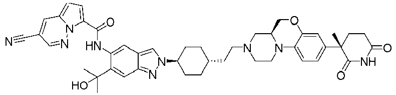 | 784.9 [M+H]⁺ |
| 127 | 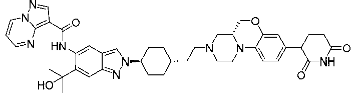 | 746.9 [M+H]⁺ |

### Example 9 Preparation of compound 46

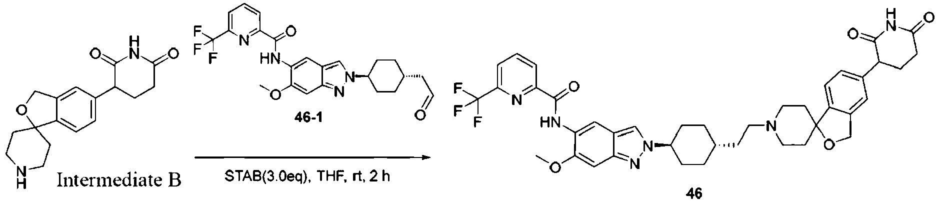

Sodium triacetoxyborohydride (74.09 mg, 0.351 mmol, 3 equiv) was added in batches to a solution of intermediate **B** (35 mg, 0.117 mmol, 1 equiv) and compound **46-1** (53.66 mg, 0.117 mmol, 1.0 equiv) in tetrahydrofuran (1.0 mL) at 0°C. The resulting residue was stirred for reaction for 2 h at room temperature. Following complete reaction, the reaction mixture was quenched with water at 0°C. The resulting residue was concentrated under reduced pressure. The reaction mixture was extracted with ethyl acetate (3×10 mL). The organic phases were mixed, backwashed with water (3×5 mL), and dried over sodium sulfate. The resulting mixture was filtered, and the filtrate was concentrated under reduced pressure. The resulting residue was prepared by high performance liquid chromatography under the following conditions (chromatography column specification: XBridge BEH C18 OBD Prep Column 130, 5 um, 30 mm *150 mm; mobile phase A: water (10 mmol/L ammonium bicarbonate), mobile phase B: acetonitrile; flow rate: 60 mL/min; elution gradient: 38% B to 60% B in 10 min; detection wavelength: UV 254 nm/220 nm; retention time (min): 8.47). Compound **46** was obtained.

LCMS: (ESI, *m*/*z):* 745.3 [M+H]⁺.

¹H NMR: (400 MHz, Chloroform-d, ppm) δ 12.58 (s, 1H), δ 10.70 (s, 1H), 8.82 (s, 1H), 8.49 (d, *J =* 7.8 Hz, 1H), 8.12 (t, *J =* 7.8 Hz, 1H), 7.90 (s, 1H), 7.88 - 7.84 (m, 2H), 7.15 (s, 1H), 7.07 (d, *J =* 6.9 Hz, 2H), 5.07 (s, 2H), 4.32 (t, *J =* 11.9 Hz, 1H), 4.03 (s, 3H), 3.81 - 3.77 (m, 1H), 3.07 (s, 2H), 2.85 (m, 2H), 2.75 (t, *J* = 5.0 Hz, 1H), 2.72 - 2.56 (m, 2H), 2.43 (m, 2H), 2.34 - 2.17 (m, 5H), 1.99 (t, *J =* 13.4 Hz, 6H), 1.83 (s, 2H), 1.26 (s, 2H).

### Example 10 Synthesis of compound 121

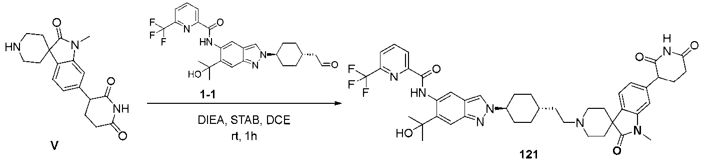

Intermediate **1-1** and intermediate **V** were selected, and compound **121** (27.2 mg, 37.00%) was prepared according to general preparation method II. LCMS: (ESI, m/z): 799.95 [M+1]⁺.

¹H NMR (400 MHz, DMSO-*d*₆, *ppm)* δ 12.36 (s, 1H), 10.85 (s, 1H), 8.71 (s, 1H), 8.45 (d, J= 8.1 Hz, 1H), 8.41-8.31(m, 2H), 8.16 (q, *J =* 2.9 Hz, 1H), 7.58 (s, 1H), 7.48 (d, *J =* 7.6 Hz, 1H), 6.92 (s, 1H), 6.89 (d, *J =* 7.8 Hz, 1H), 5.94 (s, 1H), 4.44 (t, *J =* 11.5 Hz, 1H), 3.87 (dd, *J =* 4.9, 11.8 Hz, 1H), 3.11 (s, 3H), 2.90-2.80 (m, 2H), 2.71-2.67 (m, 1H), 2.63-2.53 (m, 4H), 2.30-2.22 (m, 1H), 2.21-2.12 (m, 2H), 2.12-2.05 (m, 1H), 2.05-1.86 (m, 5H), 1.86-1.72 (m, 2H), 1.710-1.58 (m, 8H), 1.55-1.39 (m, 3H), 1.28-1.16 (m, 2H).

According to the preparation method of Example 9 (selecting corresponding intermediates and according to general preparation method II), the following compounds were prepared:

| Compound | Structure | MS m/z (ESI) |
|---|---|---|
| 47 | 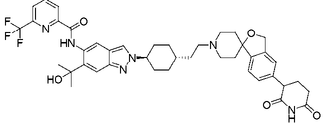 | 773.4 [M+H]⁺ |
| 48 | 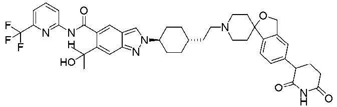 | 773.4 [M+H]⁺ |
| 49 | 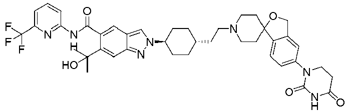 | 774.4 [M+H]⁺ |
| 50 | 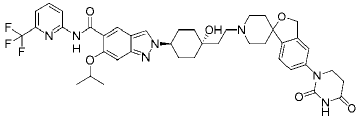 | 790.4 [M+H]⁺ |
| 51 | 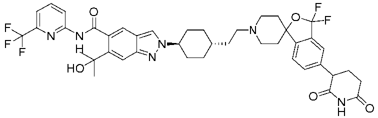 | 809.3 [M+H]⁺ |
| 52 | 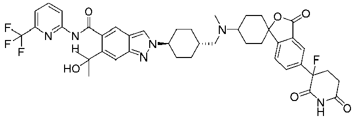 | 819.3 [M+H]⁺ |
| 53 | 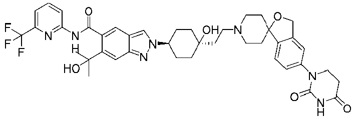 | 890.4 [M+H]⁺ |
| 54 | 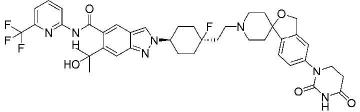 | 792.3 [M+H]⁺ |
| 55 | 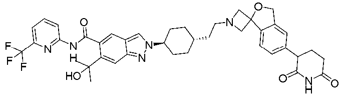 | 745.3 [M+H]⁺ |
| 56 | 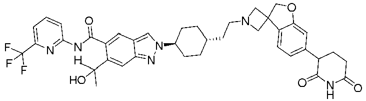 | 745.3 [M+H]⁺ |
| 57 | 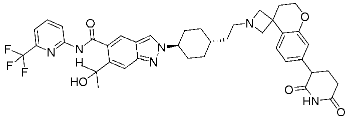 | 759.3 [M+H]⁺ |
| 58 | 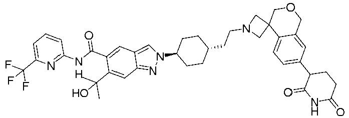 | 759.3 [M+H]⁺ |
| 59 | 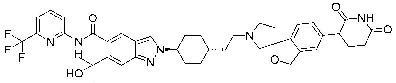 | 759.3 [M+H]⁺ |
| 60 | 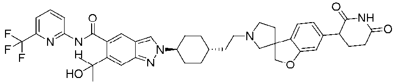 | 759.3 [M+H]⁺ |
| 61 | 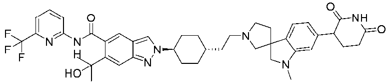 | 772.4 [M+H]⁺ |
| 62 | 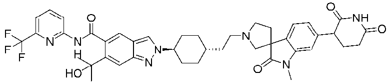 | 786.4 [M+H]⁺ |
| 63 | 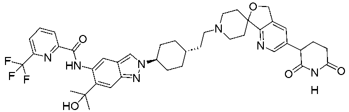 | 774.4 [M+H]⁺ |
| 64 | 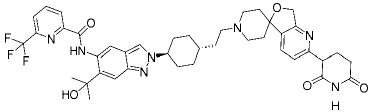 | 774.4 [M+H]⁺ |
| 65 | 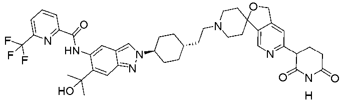 | 774.4 [M+H]⁺ |
| 66 | 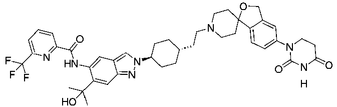 | 774.4 [M+H]⁺ |
| 67 | 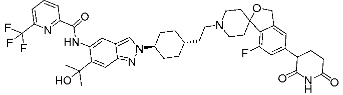 | 791.4 [M+H]⁺ |
| 69 | 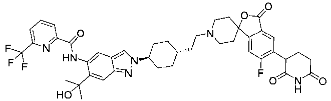 | 805.3 [M+H]⁺ |
| 70 | 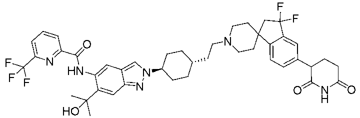 | 807.4 [M+H]⁺ |
| 71 | 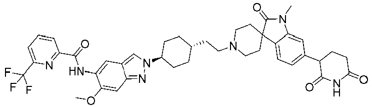 | 772.4 [M+H]⁺ |
| 72 | 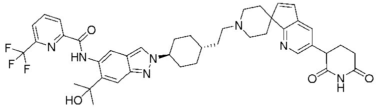 | 770.4 [M+H]⁺ |
| 73 | 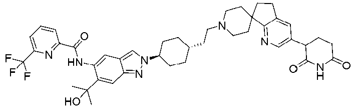 | 772.4 [M+H]⁺ |
| 74 | 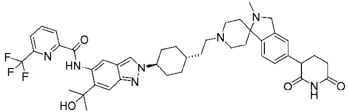 | 786.4 [M+H]⁺ |
| 75 | 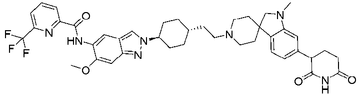 | 758.4 [M+H]⁺ |
| 76 | 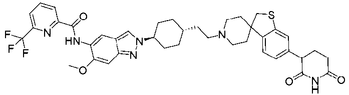 | 761.3 [M+H]⁺ |
| 77 | 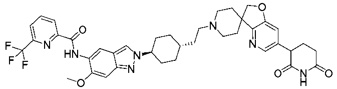 | 746.3 [M+H]⁺ |
| 78 | 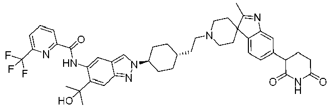 | 784.4 [M+H]⁺ |
| 79 | 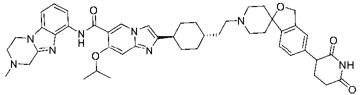 | 813.4 [M+H]⁺ |
| 80 | 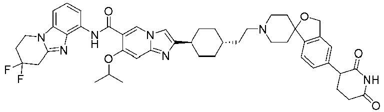 | 834.4 [M+H]⁺ |
| 81 | 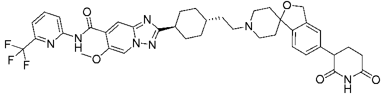 | 746.3 [M+H]⁺ |
| 82 | 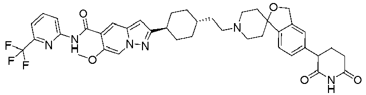 | 745.3 [M+H]⁺ |
| 83 | 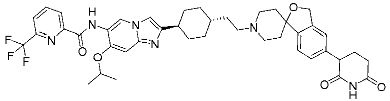 | 773.4 [M+H]⁺ |
| 84 | 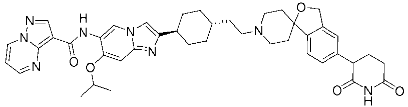 | 745.4 [M+H]⁺ |
| 85 | 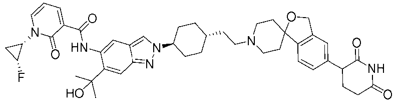 | 779.4 [M+H]⁺ |
| 86 | 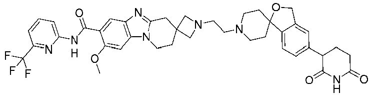 | 758.3 [M+H]⁺ |
| 87 | 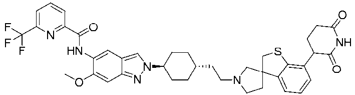 | 747.3 [M+H]⁺ |
| 88 | 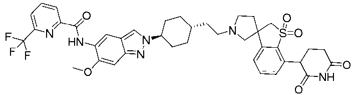 | 779.3 [M+H]⁺ |
| 89 | 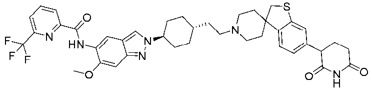 | 761.3 [M+H]⁺ |
| 90 | 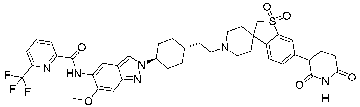 | 793.3 [M+H]⁺ |
| 91 | 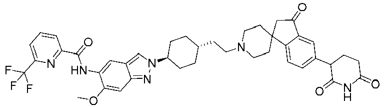 | 757.3 [M+H]⁺ |
| 92 | 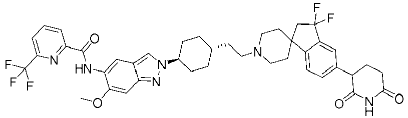 | 779.3 [M+H]⁺ |
| 93 | 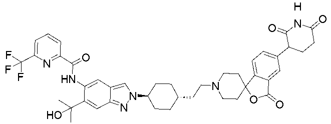 | 787.2 [M+H]⁺ |
| 94 | 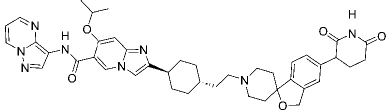 | 745.3 [M+H]⁺ |
| 96 | 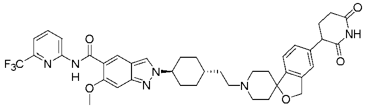 | 744.9 [M+H]⁺ |
| 97 | 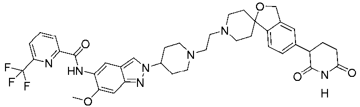 | 745.9 [M+H]⁺ |
| 103 | 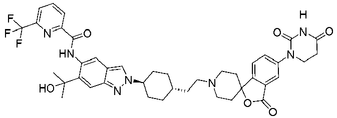 | 788.3 [M+H]⁺ |
| 106 | 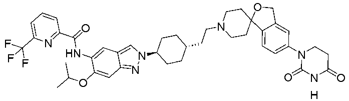 | 774.3 [M+ H]⁺ |
| 110 | 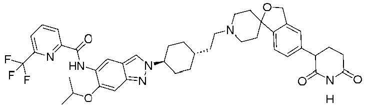 | 773.1 [M+H]⁺ |
| 114 | 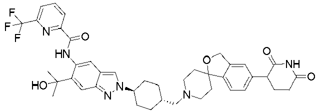 | 759.3 [M+H]⁺ |

NMR data of the compounds of the present application is as follows:

| Compound | NMR data |
|---|---|
| 1-a | ¹H NMR (400 MHz, DMSO-*d*₆, *ppm*) δ 12.36 (s, 1H), 10.75 (s, 1H), 8.71 (s, 1H), 8.45 (d, J = 7.3 Hz, 1H), 8.41-8.25(m, 2H), 8.16 (d, J = 7.4 Hz, 1H), 7.57 (s, 1H), 6.80 (d, J = 7.8 Hz, 1H), 6.63 (d, J = 7.4 Hz, 1H), 6.55 (s, 1H), 5.93 (s, 1H), 4.42 (s, 1H), 4.24 (d, J = 10.0 Hz, 1H), 3.88 (t, J = 9.3 Hz, 1H), 3.68 (s, 2H), 3.11-2.88 (m, 3H), 2.75-2.61 (m, 2H), 2.43-2.28(m, 3H), 2.22-2.04 (m, 4H), 2.04-1.78 (m, 5H), 1.780-1.53 (m, 7H), 1.45 (s, 3H), 1.32-1.044 (m, 2H). |
| 1-b | ¹HNMR (400 MHz, DMSO-*d*₆, ppm) δ 12.36 (s, 1H), 10.76 (s, 1H), 8.71 (s, 1H), 8.45 (d, *J =* 7.7 Hz, 1H), 8.36 (t, *J =* 7.8 Hz, 2H), 8.16 (d, *J =* 8.2 Hz, 1H), 7.57 (s, 1H), 6.80 (d, *J =* 8.6 Hz, 1H), 6.63 (q, *J =* 3.4 Hz, 1H), 6.55 (d, *J =* 1.8 Hz, 1H), 5.94 (s, 1H), 4.42 (t, *J =* 11.6 Hz, 1H), 4.24 (q, *J =* 4.3 Hz, 1H), 3.88 (t, *J* = 9.8 Hz, 1H), 3.68 (m, *J =* 5.3 Hz, 2H), 2.96 (q, *J =* 10.8 Hz, 3H), 2.61 (m, *J =* 5.4 Hz, 2H), 2.46 (d, *J =* 4.7 Hz, 1H), 2.41 (m, *J =* 5.3 Hz, 2H), 2.10 (q, *J =* 6.0 Hz, 4H), 1.95 (m, *J =* 7.6 Hz, 5H), 1.68 (t, *J =* 10.7 Hz, 1H), 1.62 (s, 6H), 1.45 (s, 3H), 1.21 (t, *J =* 10.4 Hz, 2H). |
| 2 | ¹HNMR (400 MHz, DMSO-*d*₆, ppm) δ 12.36 (s, 1H), 10.79 (s, 1H), 8.71 (s, 1H), 8.45 (d, *J* = 7.9 Hz, 1H), 8.37 (d, *J* = 7.5 Hz, 2H), 8.16 (d, *J* = 7.7 Hz, 1H), 7.57 (d, *J* = 2.1 Hz, 2H), 6.88 (s, 1H), 5.93 (s, 1H), 4.43 (s, 1H), 4.30 *(d, J* = 11.5 Hz, 2H), 3.90 (t, *J* = 9.7 Hz, 1H), 3.72 (dd, *J* = 12.1, 4.9 Hz, 1H), 2.97 (t, *J* = 10.5 Hz, 2H), 2.78 - 2.63 (m, 2H), 2.13 (d, *J* = 11.5 Hz, 4H), 1.91 (d, *J* = 12.2 Hz, 6H), 1.73 (s, 1H), 1.62 (s, 6H), 1.45 (s, 3H), 1.20 *(d, J =* 11.9 Hz, 3H). |
| 4 | ¹H NMR (400 MHz, DMSO-*d*₆, *ppm*): δ 12.36 (s, 1H), 10.80 (s, 1H), 8.71 (s, 1H), 8.45 (d, *J* = 7.8 Hz, 1H), 8.37 (t, *J* = 7.8 Hz, 2H), 8.15 (t, *J* = 7.2 Hz, 1H), 7.57 (s, 1H), 6.60 (t, *J =* 33.5 Hz, 2H), 5.93 (s, 1H), 4.45 (s, 1H), 4.24 (d, *J =* 14.9 Hz, 2H), 4.02 (t, *J* = 9.0 Hz, 1H), 3.73 (d, *J* = 7.8 Hz, 1H), 3.63 (s, 3H), 3.14 (s, 1H), 2.92 (d, *J* = 23.2 Hz, 1H), 2.17 (t, *J* = 16.2 Hz, 4H), 1.94 (q, *J =* 10.8 Hz, 6H), 1.62 (s, 8H), 1.45 (s, 2H), 1.24 (s, 3H). |
| 4-a | ¹HNMR (400 MHz, DMSO-*d*₆, ppm) δ 12.36 (s, 1H), 10.79 (s, 1H), 8.71 (s, 1H), 8.45 (d, J = 7.8 Hz, 1H), 8.40-8.32(m, 2H), 8.16 (d, J = 7.8 Hz, 1H), 7.57 (s, 1H), 6.60 (d, J = 14.0 Hz, 1H), 6.51 (s, 1H), 5.93 (s, 1H), 4.42 (t, J = 11.7 Hz, 1H), 4.20 (dd, J = 2.2, 10.8 Hz, 1H), 4.02 (t, J = 9.8 Hz, 1H), 3.71 (dd, J = 4.7, 11.7 Hz, 1H), 3.67-3.57 (m, 1H), 3.14 (s, 1H), 3.01 (t, J = 8.9 Hz, 1H), 2.74 (d, J = 8.9 Hz, 1H), 2.67-2.58(m, 2H), 2.47-2.42 (m, 1H), 2.39-2.32(m, 3H), 2.25-2.09 (m,4H), 2.02-1.960(m, 1H), 1.94-1.84 (m, 4H), 1.62 (s, 6H), 1.49-1.38 (m, 3H), 1.24-1.14 (m, 2H). |
| 4-b | ¹H NMR (400 MHz, DMSO-*d*₆, *ppm):* δ 12.36 (s, 1H), 10.79 (s, 1H), 8.71 (s, 1H), 8.45 (d, *J =* 7.8 Hz, 1H), 8.41-8.31 (m, 2H), 8.16 (d, *J =* 7.7 Hz, 1H), 7.57 (s, 1H), 4.42 (t, *J* = 11.2 Hz, 1H), 4.20 (d, *J* = 9.6 Hz, 1H), 4.03 (t, *J* = 9.7 Hz, 1H), 3.80-3.68 (m, 1H), 3.68-3.58 (m, 1H), 3.17-3.11 (m, 1H), 3.01 (t, *J* = 8.8 Hz, 1H), 2.74 (d, *J =* 9.4 Hz, 1H), 2.67-2.58 (m, 2H), 2.42-2.30 (m, 4H), 2.28-2.17 (m, 2H), 2.16-2.11(m, 2H), 1.93-1.86 (m, 2H), 1.91 (d, *J* = 11.1 Hz, 3H), 1.62 (s, 6H), 1.48-1.38(m, 3H), 1.26-1.15(m, 2H). |
| 31 | ¹H NMR (400 MHz, DMSO-*d*₆, ppm) δ 10.76 (s, 1H), 10.50 (s, 1H), 8.69 (d, J = 4.1 Hz, 1H), 8.46 (d, J = 7.8 Hz, 1H), 8.41 (t, J = 7.7 Hz, 1H), 8.34 (s, 1H), 8.22 (d, J = 7.8 Hz, 1H), 7.17 (d, J = 10.6 Hz, 1H), 6.81 (d, J = 8.6 Hz, 1H), 6.63 (d, J = 8.2 Hz, 1H), 6.55 (d, J = 1.8 Hz, 1H), 4.37 (s, 1H), 4.24 (t, J = 5.3 Hz, 1H), 3.98 (s, 3H), 3.89 (t, J = 9.9 Hz, 1H), 3.68 (t, J = 5.1 Hz, 2H), 2.96 (t, J = 16.7 Hz, 4H), 2.11 (s, 4H), 1.99 (m, 1H), 1.94 (m, J = 10.0 Hz, 5H), 1.67 (d, J = 6.8 Hz, 2H), 1.57 (s, 1H), 1.45 (t, J = 7.0 Hz, 3H), 1.24 (s, 1H), 1.19 (d, J = 9.7 Hz, 2H). |
| 47 | ¹H NMR (400 MHz, DMSO-*d*₆, ppm): δ 12.36 (s, 1H), 10.83 (s, 1H), 8.72 (s, 1H), 8.45 (d, *J =* 7.7 Hz, 1H), 8.41 - 8.27 (m, 2H), 8.16 (d, *J =* 8.6 Hz, 1H), 7.58 (s, 1H), 7.23 (d, *J =* 7.9 Hz, 1H), 7.13 (d, *J =* 6.1 Hz, 2H), 5.94 (s, 1H), 4.96 (s, 2H), 4.42 (d, *J =* 11.8 Hz, 1H), 3.88 (dd, *J =* 11.6, 4.9 Hz, 1H), 2.82 (s, 2H), 2.74 - 2.61 (m, 5H), 2.36 - 2.30 (m, 2H), 2.26 - 2.10 (m, 2H), 2.08 - 1.99 (m, 1H), 1.92 (d, *J =* 10.6 Hz, 6H), 1.62 (m, 8H), 1.46 (s, 3H), 1.30 - 1.12 (m, 2H). |
| 66 | ¹HNMR (400 MHz, DMSO-*d*₆, ppm) δ 12.36 (s, 1H), 10.36 (s, 1H), 8.72 (s, 1H), 8.45 (d, *J* = 7.7 Hz, 1H), 8.37 (t, *J* = 7.8 Hz, 2H), 8.16 (d, *J* = 7.8 Hz, 1H), 7.57 (s, 1H), 7.29 (d, *J* = 8.5 Hz, 1H), 7.22 (d, *J* = 7.4 Hz, 2H), 5.93 (s, 1H), 4.97 (s, 2H), 4.43 (t, *J* = 11.6 Hz, 1H), 3.78 (t, *J* = 6.7 Hz, 2H), 3.28 (s, 1H), 2.82 (s, 2H), 2.70 (t, *J =* 6.6 Hz, 2H), 2.41 (s, 1H), 2.27 (s, 1H), 2.14 (d, *J =* 8.8 Hz, 2H), 1.91 (t*, J* = 11.3 Hz, 6H), 1.62 (s, 8H), 1.46 (s, 3H), 1.20 (q, *J* = 10.0 Hz, 3H). |
| 93 | ¹H NMR(400 MHz, DMSO-*d*₆, *ppm*) δ 12.36 (s, 1H), 10.90 (s, 1H), 8.72 (s, 1H), 8.45 (d, *J* = 7.7 Hz, 1H), 8.40 - 8.34 (m, 2H), 8.16 (dd, *J* = 7.8, 1.1 Hz, 1H), 7.78 - 7.62 (m, 3H), 7.58 (s, 1H), 5.94 (s, 1H), 4.48 - 4.39 (m, 1H), 4.08 (dd, *J* = 12.2, 4.8 Hz, 1H), 2.93 (d, *J* = 9.7 Hz, 2H), 2.76 - 2.68 (m, 3H), 2.57 (s, 1H), 2.27 (t*, J* = 13.6 Hz, 5H), 2.13 (s, 2H), 2.05 (dt, *J =* 8.1, 4.0 Hz, 1H), 1.93 (d, *J* = 10.0 Hz, 4H), 1.62 (s, 8H), 1.47 (s, 3H), 1.22 (d, *J =* 13.0 Hz, 2H). |
| 94 | ¹H NMR (400 MHz, DMSO-*d*₆, *ppm*) δ 10.82 (s, 1H), 10.51 (s, 1H), 9.18 (s, 1H), 9.09 (q, J = 2.8 Hz, 1H), 8.77 (s, 1H), 8.54 (q, J = 1.7 Hz, 1H), 7.69 (s, 1H), 7.21 (t, J = 8.1 Hz, 1H), 7.14 (d, J = 17.1 Hz, 3H), 7.06 (q, J = 3.7 Hz, 1H), 5.04 (m, J = 6.0 Hz, 1H), 4.96 (s, 2H), 3.88 (m, J = 5.7 Hz, 1H), 2.79 (s, 2H), 2.65 (m, J = 9.8 Hz, 2H), 2.40 (s, 2H), 2.21 (m, J = 7.3 Hz, 3H), 2.05 (q, J = 8.5 Hz, 3H), 1.88 (t, J = 12.2 Hz, 4H), 1.63 (d, J = 12.3 Hz, 2H), 1.54 (d, J = 6.0 Hz, 6H), 1.39 (q, J = 12.8 Hz, 6H), 1.11 (q, J = 10.1 Hz, 2H). |
| 95 | ¹H NMR (400 MHz, DMSO-*d*₆, *ppm)* δ 10.79 (d, *J =* 31.4 Hz, 2H), 8.56 - 8.47 (m, 2H), 8.24 (s, 1H), 8.13 (t, *J =* 8.1 Hz, 1H), 7.64 (d, *J* = 7.6 Hz, 1H), 7.14 (s, 1H), 6.81 (d, *J =* 8.5 Hz, 1H), 6.63 (d, *J =* 8.1 Hz, 1H), 6.55 (d, *J =* 2.1 Hz, 1H), 4.43 (s, 1H), 4.26 (s, 1H), 3.96 (s, 3H), 3.88 (s, 1H), 3.72 (s, 3H), 3.00 (s, 3H), 2.67 (s, 3H), 2.33 (s, 2H), 2.13 (s, 4H), 1.90 (d, *J =* 10.6 Hz, 4H), 1.69 (s, 1H), 1.45 (s, 3H), 1.25 - 1.17 (m, 2H). |
| 96 | ¹HNMR (400 MHz, DMSO-*d*₆, *ppm)* δ 10.84 (s, 2H), 8.57 - 8.50 (m, 2H), 8.25 (s, 1H), 8.13 (d, *J =* 8.2 Hz, 1H), 7.64 (d, *J =* 7.6 Hz, 1H), 7.21 (s, 1H), 7.14 (d, *J =* 6.9 Hz, 3H), 4.97 (s, 2H), 4.44 (s, 1H), 3.96 (s, 3H), 3.90 - 3.85 (m, 1H), 3.29 (s, 4H), 2.91 (s, 2H), 2.71 (s, 1H), 2.67 (s, 1H), 2.16 (d, *J =* 12.6 Hz, 3H), 2.03 (dd, *J =* 8.9, 4.6 Hz, 1H), 1.92 (d, *J =* 11.6 Hz, 6H), 1.68 (s, 2H), 1.48 (s, 3H), 1.21 (d, *J =* 12.9 Hz, 2H). |
| 97 | ¹H NMR (400 MHz, DMSO-*d*₆, ppm) δ 10.83 (s, 1H), 10.51 (s, 1H), 8.69 (s, 1H), 8.47 (d, *J* = 7.4 Hz, 1H), 8.42 (d, *J* = 7.8 Hz, 1H), 8.38 (d, *J* = 3.4 Hz, 1H), 8.22 (dd, *J* = 0.9, 7.7 Hz, 1H), 7.23 (d, *J* = 8.2 Hz, 1H), 7.17 (s, 1H), 7.16-7.07 (m, 2H), 5.32 (t, *J* = 4.7 Hz, 1H), 4.96 (s, 2H), 4.40 (m, *J* = 5.9 Hz, 1H), 3.98 (s, 3H), 3.87 (dd, *J* = 4.9, 11.5 Hz, 1H), 3.05 (d, *J* = 10.7 Hz, 2H), 2.85 (d, *J* = 8.8 Hz, 2H), 2.56-2.55 (m, 2H), 2.41-2.36 (m, 2H), 2.25-2.14(m, 3H), 2.12-2.05 (m, 4H), 2.02-1.98 (m, 2H), 1.97-1.88 (m, 2H), 1.68-1.58 (m, 2H), 1.50-1.42 (m, 1H), 1.36-1.30 (m, 1H). |
| 98 | ¹HNMR (400 MHz, DMSO-*d*₆, ppm) δ 10.75 (s, 1H), 10.50 (s, 1H), 8.69 (s, 1H), 8.53-8.32 (m, 3H), 8.22 (d, *J* = 7.8 Hz, 1H), 7.17 (s, 1H), 6.80 (d, *J* = 7.8 Hz, 1H), 6.63 (d, *J =* 8.6 Hz, 1H), 6.54 (t, *J =* 10.0 Hz, 1H), 4.43-4.34 (m, 1H), 4.28-4.18 (m, 1H), 3.98 (s, 3H), 3.88 (t, *J* = 9.4 Hz, 1H), 3.71-3.63 (m, 2H), 3.34-3 .33 (m, 7H), 3.30-3.29 (m, 4H), 3.08-2.92 (m, 5H), 2.17-2.05 (m, 6H). |
| 99 | ¹HNMR (400 MHz, DMSO-*d*₆, *ppm*) δ 12.36 (s, 1H), 10.74 (s, 1H), 8.72 (s, 1H), 8.48-8.32(m, 3H), 8.16 (d, *J* = 7.7 Hz, 1H), 7.58 (s, 1H), 6.80 (d, *J* = 8.6 Hz, 1H), 6.63 (d, *J* = 8.7 Hz, 1H), 6.55 (s, 1H), 5.94 (s, 1H), 4.45 (d, *J* = 6.0 Hz, 1H), 4.23 (q, *J =* 4.3 Hz, 1H), 3.88 (t, *J =* 9.6 Hz, 1H), 3.73-3.61 (m, 2H), 3.08-2.98 (m, 4H), 2.95 (d, *J =* 10.0 Hz, 1H), 2.65-2.58 (m, 2H), 2.46-2.41 (m, 2H), 2.23-2.15 (m, 3H), 2.13-2.06 (m, 4H), 2.03-1.96 (m, 1H), 1.78 (t, *J* = 10.3 Hz, 1H), 1.62 (s, 6H), 1.56-1.35 (d, *J* = 12.2 Hz, 1H), 1.35-1.22 (m, 1H), 1.16-0.90 (m, 1H), 0.87 (t, *J =* 7.0 Hz, 1H). |
| 100 | ¹HNMR (400 MHz, DMSO-*d*₆, *ppm*) δ 10.74 (s, 1H), 10.51 (s, 1H), 9.18 (s, 1H), 9.09 (dd, *J =* 1.5, 7.1 Hz, 1H), 8.76 (s, 1H), 8.54 (dd, *J =* 1.6, 4.0 Hz, 1H), 7.68 (s, 1H), 7.16 (s, 1H), 7.06 (dd, *J =* 4.0, 7.1 Hz, 1H), 6.80 (d, *J* = 8.5 Hz, 1H), 6.63 (dd, *J* = 1.9, 8.4 Hz, 1H), 6.55 (d, *J =* 1.8 Hz, 1H), 4.24 (dd, *J =* 2.5, 10.5 Hz, 1H), 3.88 (t, *J =* 9.8 Hz, 1H), 3.67 (t, *J* = 5.4 Hz, 2H), 3.05-2.87 (m, 3H), 2.62-2.56 (m, 2H), 2.47-2.45 (m, 1H), 2.44-2.40 (m, 1H), 2.39-2.36 (m, 1H), 2.150-1.96(m, 5H), 1.85 (d, *J =* 10.5 Hz, 2H), 1.67 (t, *J =* 10.6 Hz, 1H), 1.54 (d, *J* = 6.0 Hz, 6H), 1.46-1.22(m, 6H), 1.10 (q, *J =* 11.5 Hz, 2H). |
| 101 | ¹HNMR (400 MHz, DMSO-*d*₆, ppm) δ 12.36 (s, 1H), 10.77 (s, 1H), 8.71 (s, 1H), 8.45 (d, *J* = 7.8 Hz, 1H), 8.41-8.31 (m, 2H), 8.16 (d, *J* = 7.7 Hz, 1H), 7.57 (s, 1H), 7.00-6.77 (m, 1H), 6.76-6.53 (m, 2H), 5.94 (s, 1H), 4.51-4.40 (m, 1H), 4.34-4.23 (m, 1H), 4.15-3.86(m, 2H), 3.74-3.60 (m, 2H), 3.05-2.92 (m, 2H), 2.89-2.73 (m, 2H), 2.66-2.56 (m, 2H), 2.22-2.09(m, 4H), 2.03-1.95 (m, 2H), 1.95-1.85 (m, 4H), 1.69-1.65(m, 1H), 1.62 (s, 6H), 1.51-1.41 (m, 2H), 1.25-1.22 (m, 2H). |
| 102 | ¹H NMR (400 MHz, DMSO-*d*₆, *ppm*) δ 10.76 (s, 1H), 10.73 (s, 1H), 8.72 (s, 1H), 8.46 (d, *J =* 7.6 Hz, 1H), 8.41 (t, *J =* 7.8 Hz, 1H), 8.33 (s, 1H), 8.22 (d, *J =* 7.6 Hz, 1H), 7.19 (s, 1H), 6.81 (d, *J =* 8.5 Hz, 1H), 6.63 (dd, *J =* 1.6, 8.3 Hz, 1H), 6.55 (d, *J =* 1.6 Hz, 1H), 4.83 (m, *J =* 6.0 Hz, 1H), 4.43-4.33 (m, 1H), 4.25 (dd, *J =* 2.4, 10.6 Hz, 1H), 3.88 (t*, J =* 9.7 Hz, 1H), 3.73-3.66(m, 2H), 3.05-2.99 (m, 2H), 2.94 (d, *J =* 9.4 Hz, 1H), 2.66- 2.58 (m, 3H), 2.47-2.45(m, 1H), 2.44-2.39 (m, 2H), 2.17-2.08(m, 4H), 2.04-1.97 (m, 1H), 1.94-1.85 (m, 4H), 1.74-1.67 (m, 1H), 1.49-1.44(m, 2H), 1.40 (d, *J =* 6.0 Hz, 6H), 1.22-1.12 (m, 2H). |
| 103 | ¹HNMR (400 MHz, DMSO-*d*₆, ppm) δ 12.37 (s, 1H), 10.50 (s, 1H), 8.72 (s, 1H), 8.45 (d, *J* = 7.7 Hz, 1H), 8.41-8.34(m, 2H), 8.16 (d, *J* = 7.7 Hz, 1H), 7.84-7.70 (m, 3H), 7.58 (s, 1H), 5.94 (s, 1H), 4.44 (t, *J =* 11.3 Hz, 1H), 3.87 (t, *J =* 6.6 Hz, 2H), 2.93 (d, *J =* 8.6 Hz, 2H), 2.73 (t, *J =* 6.6 Hz, 2H), 2.46-2.44 (m, 1H), 2.37-2.22(m, 5H), 2.15 (d, *J* = 9.8 Hz, 2H), 2.00-1.89 (m, 4H), 1.69-1.60 (m, 8H), 1.52-1.41 (m, 3H), 1.29-1.16 (m, 2H). |
| 104 | ¹HNMR (400 MHz, DMSO-*d*₆, ppm) δ 10.79 (s, 1H), 10.50 (s, 1H), 8.68 (s, 1H), 8.46 (d, *J =* 7.7 Hz, 1H), 8.40 (t, *J =* 7.8 Hz, 1H), 8.33 (s, 1H), 8.21 (d, *J =* 7.7 Hz, 1H), 7.16 (s, 1H), 6.60 (d, J = 14.0 Hz, 1H), 6.51 (s, 1H), 4.43-4.31 (m, 1H), 4.20 (dd, *J* = 2.3, 10.8 Hz, 1H), 4.02 (t, *J =* 9.9 Hz, 1H), 3.98 (s, 3H), 3.71 (dd, *J =* 4.7, 11.7 Hz, 1H), 3.63 (d*, J =* 10.2 Hz, 1H), 3.17-3.09(m, 1H), 3.01 (t*, J =* 8.9 Hz, 1H), 2.73 (d, *J =* 8.5 Hz, 1H), 2.69-2.58 (m, 2H), 2.41-2.30(m, 4H), 2.26-2.20 (m, 1H), 2.18-2.08 (m, 3H), 2.01-1.85 (m, 5H), 1.49-1.35 (m, 3H), 1.25-1.14 (m, 2H). |
| 105 | ¹HNMR (400 MHz, DMSO-*d*₆, *ppm)* δ 10.79 (s, 1H), 10.73 (s, 1H), 8.72 (s, 1H), 8.58-8.36 (m, 2H), 8.33 (s, 1H), 8.22 (d, *J =* 7.6 Hz, 1H), 7.19 (s, 1H), 6.60 (d, *J = 14.0* Hz, 1H), 6.51 (s, 1H), 4.83 (m, *J =* 6.0 Hz, 1H), 4.50-4.27(m, 1H), 4.20 (dd, *J =* 2.4, 10.8 Hz, 1H), 4.02 (t, *J =* 9.9 Hz, 1H), 3.71 (dd, *J =* 4.8, 11.6 Hz, 1H), 3.67-3.58 (m, 1H), 3.17- 3.10 (s, 1H), 3.01 (t, *J =* 9.3 Hz, 1H), 2.73 *(d, J =* 8.6 Hz, 1H), 2.69-2.58(m, 2H), 2.41-2.32 (m, 4H), 2.26-2.07 (m, 5H), 2.03-1.82 (m, 6H), 1.40 (d, *J =* 6.0 Hz, 6H), 1.26-1.21 (m, 1H), 1.21-1.12 (m, 2H). |
| 106 | ¹HNMR (400 MHz, DMSO-*d*₆, *ppm*) δ 10.74 (s, 1H), 10.38 (s, 1H), 8.73 (s, 1H), 8.46 (d, *J =* 7.6 Hz, 1H), 8.41 (t, *J =* 7.6 Hz, 1H), 8.34 (s, 1H), 8.22 (d, *J =* 7.5 Hz, 1H), 7.26 (s, 3H), 7.19 (s, 1H), 5.01 (s, 2H), 4.84 (m, *J =* 6.1 Hz, 1H), 4.43-4.36 (m, 1H), 3.78 (t, *J =* 6.5 Hz, 3H), 2.71 (t, *J =* 6.7 Hz, 3H), 2.15 (d, *J =* 9.5 Hz, 3H), 2.11-1.98 (m, 2H), 1.92 (t, *J =* 10.7 Hz, 4H), 1.88-1.64 (m, 3H), 1.63-1.51 (m, 2H), 1.51-1.42 (m, 2H), 1.41 *(d, J =* 6.0 Hz, 6H), 1.31-1.09(m, 3H). |
| 107 | ¹HNMR (400 MHz, DMSO-*d*₆, *ppm*) δ 10.73 (s, 1H), 10.25 (s, 1H), 8.72 (s, 1H), 8.46 (d, *J* = 7.2 Hz, 1H), 8.41 (q, *J =* 5.1 Hz, 1H), 8.33 (s, 1H), 8.22 (dd, *J =* 1.0, 7.6 Hz, 1H), 7.19 (s, 1H), 6.85 (d, *J =* 8.9 Hz, 1H), 6.74 (d, *J =* 8.5 Hz, 1H), 6.69 (d, *J* = 2.0 Hz, 1H), 4.83 (m, *J =* 5.9 Hz, 1H), 4.41-4.31 (m, 1H), 4.31-4.23 (m, 1H), 3.91 (t, *J =* 9.8 Hz, 1H), 3.77-3.643(tm, 3H), 3.08-2.91 (m, 3H), 2.69-2.64 (m, 3H), 2.45-2.35 (m, 2H), 2.16-2.06 (m, 3H), 1.96-1.85 (m, 5H), 1.49-1.43 (m, 2H), 1.40 (d, *J =* 6.0 Hz, 6H), 1.29-1.12 (m, 3H). |
| 108 | ¹HNMR (400 MHz, DMSO-*d*₆, *ppm*) δ 12.38 (s, 1H), 10.74 (s, 1H), 8.64 (d, *J* = 2.8 Hz, 1H), 8.49-8.36 (m, 3H), 8.19 (d, *J* = 7.7 Hz, 1H), 6.83-6.75 (m, 1H), 6.67-6.59 (m, 1H), 6.57-6.51 (m, 1H), 6.43-6.33 (m, 1H), 6.14-6.05 (m, 1H), 4.28-4.19 (m, 1H), 3.93-3.83 (m, 1H), 3.73-3.61 (m, 2H), 3.01-2.89 (m, 3H), 2.69-2.56 (m,3H), 2.47-2.45 (m, 1H), 2.44-2.40 (m, 1H), 2.39-2.36 (m, 1H), 2.36-2.32 (m, 1H), 2.14-2.06 (m, 2H), 2.05-1.96 (m,3H), 1.86 (d, *J* = 10.2 Hz, 1H), 1.79-1.64 (m, 2H), 1.64-1.60 (m, 6H), 1.54-1.45 (d, 2H), 1.45-1.40 (m, 2H), 1.10 (q, *J* = 11.6 Hz, 2H). |
| 109 | ¹HNMR (400 MHz, DMSO-*d*₆, *ppm*) δ 10.75 (s, 1H), 10.44 (s, 1H), 9.42 (s, 1H), 8.57-8.33(m, 2H), 8.24 (dd, J = 1.5, 7.2 Hz, 1H), 7.62 (s, 1H), 7.10 (s, 1H), 6.80 (d, *J* = 8.6 Hz, 1H), 6.63 (q, *J* = 3.4 Hz, 1H), 6.55 (d, *J* = 1.8 Hz, 1H), 4.87 (m, *J* = 6.0 Hz, 1H), 4.24 (dd, *J* = 2.6, 10.6 Hz, 1H), 3.88 (t, *J* = 9.8 Hz, 1H), 3.73-3.62 (m, 2H), 3.06-2.86(m, 3H), 2.66- 2.54 (m, 3H), 2.48-2.43(m, 2H), 2.43-2.36(m, 2H), 2.18-1.95 (m, 5H), 1.84 (d, *J* = 11.7 Hz, 2H), 1.67 (t, *J* = 10.7 Hz, 1H), 1.48-1.42 (m, 2H), 1.40 (d, *J =* 6.0 Hz, 6H), 1.39-1.28 (d, *J =* 10.6 Hz, 2H), 1.09 (q, *J* = 11.4 Hz, 2H). |
| 110 | ¹HNMR (400 MHz, DMSO-*d*₆, *ppm*) δ 10.83 (s, 1H), 10.73 (s, 1H), 8.72 (s, 1H), 8.50-8.37(m, 2H), 8.33 (s, 1H), 8.22 (d, *J* = 7.5 Hz, 1H), 7.35-7.17 (m, 2H), 7.17-7.01 (m, 2H), 4.96 (s, 1H), 4.84 (m, *J* = 6.0 Hz, 1H), 4.37 (t, *J* = 11.5 Hz, 1H), 3.88 (dd, *J* = 4.9, 11.9 Hz, 1H), 2.83 (d, *J* = 7.9 Hz, 2H), 2.72-2.63 (m, 2H), 2.37-2.27 (m, 4H), 2.22-2.17 (m, 1H), 2.13 (d, *J* = 9.8 Hz, 2H), 2.07-2.01 (m, 1H), 1.91 (t, *J =* 10.9 Hz, 6H), 1.64 (d, *J =* 13.0 Hz, 2H), 1.51-1.43 (m, 3H), 1.41 (d, *J* = 6.0 Hz, 6H), 1.25-1.16 (m, 2H). |
| 111 | ¹HNMR (400 MHz, DMSO-*d*₆, *ppm*) δ 12.36 (s, 1H), 10.75 (s, 1H), 8.71 (s, 1H), 8.45 (d, *J =* 7.8 Hz, 1H), 8.40 - 8.31 (m, 2H), 8.18 - 8.14 (m, 1H), 7.58 (d, *J =* 7.1 Hz, 1H), 6.81 *(d, J =* 8.5 Hz, 1H), 6.63 (d, *J =* 8.5 Hz, 1H), 6.56 - 6.51 (m, 1H), 5.93 (s, 1H), 4.44 (s, 1H), 4.25 (d, *J* = 8.9 Hz, 1H), 3.88 (d, *J* = 9.7 Hz, 1H), 3.67 (dd, *J =* 10.8, 4.7 Hz, 2H), 3.03 (s, 1H), 2.97 - 2.88 (m, 2H), 2.67 (d, *J* = 3.5 Hz, 2H), 2.61 (s, 1H), 2.33 (s, 1H), 2.22 (s, 2H), 2.14 (t, *J* = 12.6 Hz, 4H), 1.96 (d, *J* = 9.6 Hz, 4H), 1.91 (s, 1H), 1.71 (d, *J* = 10.8 Hz, 2H), 1.62 (s, 6H), 1.15 *(d, J =* 12.6 Hz, 1H). |
| 112 | ¹HNMR (400 MHz, DMSO-*d*₆, *ppm*) δ 10.83 (s, 1H), 10.52 (s, 1H), 9.52 (s, 1H), 8.66-8.41(m, 2H), 8.33 (d, *J =* 7.2 Hz, 1H), 7.71 (s, 1H), 7.19 (s, 1H), 6.88 (d, *J* = 8.4 Hz, 1H), 6.71 (d, *J =* 8.3 Hz, 1H), 6.63 (d, *J =* 1.6 Hz, 1H), 4.96 (m, *J =* 6.0 Hz, 1H), 4.33 (dd, *J =* 2.2, 10.4 Hz, 1H), 3.96 (t, *J =* 9.8 Hz, 1H), 3.86-3.62 (m, 2H), 3.10 (t, *J* = 9.4 Hz, 1H), 3.06-2.94(m, 2H), 2.75-2.64 (m, 3H), 2.33-2.24 (m, 2H), 2.22-2.11 (m, 4H), 2.11-2.05(m, 1H), 2.03-1.94 (m, 2H), 1.80-1.66(m, 2H), 1.57-1.50(m, 2H), 1.49 (d, *J =* 6.0 Hz, 6H), 1.35-1.24(m, 1H), 1.12 *(q, J* = 11.8 Hz, 2H). |
| 113 | ¹H NMR (400 MHz, DMSO-*d*₆, *ppm*) δ 12.37 (s, 1H), 10.78 (s, 1H), 8.72 (s, 1H), 8.46 (d, *J* = 7.8 Hz, 1H), 8.41-8.33 (m, 2H), 8.17 (d, *J* = 7.7 Hz, 1H), 7.58 (s, 1H), 6.12 (d, *J* = 14.5 Hz, 1H), 6.02-5.94(m, 2H), 5.76 (d, *J* = 7.3 Hz, 1H), 4.43 (t, *J* = 11.2 Hz, 1H), 4.21 (d, *J* = 11.1 Hz, 1H), 4.12 (q, *J* = 11.6 Hz, 2H), 3.20 (s, 1H), 3.00 (d, *J* = 13.1 Hz, 2H), 2.74-2.68 (m, 1H), 2.67-2.58 (m, 4H), 2.42-2.30 (m, 3H), 2.17-2.06 (m, 3H), 1.97-1.82(m, 5H), 1.62 (s, 6H), 1.47-1.38 (m, 3H), 1.27-1.18(m, 2H). |
| 118 | ¹HNMR (400 MHz, DMSO-*d*₆, *ppm*) δ 12.36 (s, 1H), 10.21 (s, 1H), 8.71 (s, 1H), 8.45 (d, J = 7.7 Hz, 1H), 8.40 - 8.30 (m, 1H), 8.16 (d, J = 7.8 Hz, 1H), 7.57 (s, 1H), 6.95 (d, J = 8.9 Hz, 1H), 6.89 (s, 1H), 6.82 (d, J = 8.8 Hz, 1H), 5.93 (s, 1H), 4.43 (m, 1H), 3.78 (d, J = 11.4 Hz, 1H), 3.66 (t, J = 6.8 Hz, 2H), 3.53 (s, 1H), 2.95 (m, 3H), 2.80 (m, 1H), 2.71 (s, 1H), 2.70 - 2.64 (m, 3H), 2.44 (s, 1H), 2.38 (s, 2H), 2.14 (m, 2H), 2.09 - 2.00 (m, 1H), 1.91 (m, 3H), 1.80 (s, 1H), 1.62 (s, 6H), 1.45 (s, 3H), 1.24 (m, 2H). |
| 119 | ¹HNMR (400 MHz, DMSO-*d*₆, *ppm*) δ 12.35 (s, 1H), 10.73 (s, 1H), 8.71 (s, 1H), 8.45 (d, J = 7.7 Hz, 1H), 8.41 - 8.34 (m, 2H), 8.16 (d, J = 7.7 Hz, 1H), 7.57 (s, 1H), 6.85 (d, J = 9.2 Hz, 1H), 6.77 (d, J *=* 7.8 Hz, 2H), 5.93 (s, 1H), 4.43 (s, 1H), 3.74 (s, 1H), 3.66 (m, 1H), 2.92 (m, 3H), 2.80 (m, 2H), 2.71 - 2.55 (m, 1H), 2.47 (s, 1H), 2.37 (m, 3H), 2.12 (m, 3H), 2.01 (d, J = 5.0 Hz, 1H), 1.90 (m, 4H), 1.78 (m, 1H), 1.62 (s, 6H), 1.45 (s, 3H), 1.28 - 1.14 (m, 4H). |
| 114 | ¹HNMR (400 MHz, DMSO-*d*₆, *ppm*) δ 12.36 (s, 1H), 10.83 (s, 1H), 8.72 (s, 1H), 8.45 (d, J = 7.8 Hz, 1H), 8.41-8.32(m, 2H), 8.16 (d, J = 7.9 Hz, 1H), 7.57 (s, 1H), 7.23 (d, J = 8.2 Hz, 1H), 7.16-7.10 (m, 2H), 5.93 (s, 1H), 5.93 (s, 1H), 4.96 (s, 2H), 4.51-4.38 (m, 1H), 3.87 (dd, J = 4.8, 11.6 Hz, 1H), 2.81 (d, J = 6.8 Hz, 2H), 2.67-2.65 (m, 1H), 2.31-2.21 (m, 3H), 2.21-2.10(m, 3H), 2.10-2.01 (m, 2H), 2.01-1.96 (m, 2H), 1.96-1.87 (m, 4H), 1.76-1.52 (m, 10H), 1.15 (q, J = 12.0 Hz, 2H). |

### Biological test and evaluation

The present application will be further described and explained with reference to the following test examples, but the test examples do not limit the scope of the present application.

**Test Example 1** Detection of HiBiT-IRAK4 proteolysis levels in K562 IRAK4-HiBiT cells co-incubated with compounds of the present application
Reagents, devices, and consumables in experiments:
Nano-Glo Lytic Detection Assay, purchased from Promega;
EnVision, purchased from PerkinElmer;
Name of cell line: K562 IRAK4-HiBiT stable transformation cell strain (capable of stably expressing IRAK4-HiBiT fusion protein), constructed by Shanghai Bioduro Biologics Co., Ltd., where a K562 cell strain was purchased from ATCC. The culture medium is IMDM+10% FBS+1% P/S (volume percentage, where FBS is fetal bovine serum and P/S is Penicillin-Streptomycin, a 1:1 mixture of penicillin and streptomycin antibiotic solution (10,000 units/mL)).

### Experimental steps:

Cell culture: K562 IRAK4-HiBiT cells were incubated at 37°C, and passaged every 2-3 d, with a seeding density of 200,000 cells/mL.

IRAK4-HiBiT proteolysis experiment:
Step 1: Cell seeding and treatment of compound
   1. A 10 mM compound mother liquor was prepared using DMSO, and a compound solution with a working concentration of 1,000 times (the highest working concentration is 10 µM, diluted 3.162 times, with a total of 10 concentration gradients) was prepared using Bravo automatic liquid processing platform.
   2. 40 nL of a compound working solution was transferred to a 384 well plate, and an equal volume of DMSO was added to positive control wells.
   3. 20 µL of a culture solution was added per well and shaken for 10 min.
   4.20 µL of a K562 IRAK4-HiBiT cell suspension was added per well (6,000 cells/well), and an equal volume of culture medium was added to negative control wells.
   5. The well plate was rotated at 1,000 rpm for 1 min.
   6. The cells were incubated in a 5% CO₂ incubator at 37°C for 2 h or 6 h.
Step 2: HiBiT Lytic experiment
   1. The well plate was placed at room temperature to equilibrate for 30 min.
   2. 20 µL of Nano-Glo HiBiT Lytic reagent (trade name: Nano-Glo^{®} HiBiT Lytic Detection Reagent) and the well plate was kept in the dark.
   3. The well plate was placed on an orbital shaker at 300 rpm for 5 min.
   4. The cells were incubated at room temperature for 10 min.
   5. The luminous value was read by Envision.
   6. Data was calculated and processed. Degradation degree%=(negative control - experimental well)/(negative control - positive control) * 100%. Using XL-fit software, a degradation curve was obtained by fitting using a 4 Parameter Logistic Model, and the values of Relative DC₅₀ (nM) and Absolute DC₅₀ (nM) were obtained.

The experimental results are shown in Table 1 and Table 2 below:

**Table 1**

| **Compound** | **6 h** | | |
|---|---|---|---|
| | **Relative DC₅₀ (nM)** | **Absolute DC₅₀ (nM)** | **Dmax** |
| **1** | 0.46 | 0.61 | 91.14% |
| **2** | 5.43 | 6.32 | 96% |
| **46** | 0.79 | 0.92 | 93.58% |
| **47** | 0.54 | 0.65 | 93% |
| **94** | 3.97 | 3.82 | 97% |
| **95** | 4.83 | 3.87 | 97% |
| **96** | 0.44 | 0.48 | 97% |
| **97** | 1.60 | 1.64 | 93% |
| **98** | 0.2 | <1.00 | 89% |
| **114** | 3.99 | 4.85 | 85% |
| **115** | 81.37 | 146.47 | 77% |
| **116** | 4.48 | 8.37 | 83% |
| **117** | 10.33 | 11.67 | 89% |
| **126** | 12.32 | 10.76 | 97% |
| **128** | 16.33 | 13.87 | 94% |
| **129** | 4.32 | 4.41 | 97% |
| **130** | 8.51 | 8.13 | 97% |
| **131** | 14.85 | 11.37 | 98% |

**Table 2**

| **Compound** | **2 h** | | |
|---|---|---|---|
| | **Relative DC₅₀ (nM)** | **Absolute DC₅₀ (nM)** | **Dmax** |
| **1-a** | 0.58 | 0.42 | 90% |
| **1-b** | 0.71 | 0.54 | 88% |
| **4** | 8.71 | 9.85 | 90% |
| **93** | 0.93 | 0.74 | 94% |
| **99** | 13.47 | 40.34 | 66% |
| **100** | 17.59 | 17.65 | 89% |
| **101** | 4.28 | 7.20 | 87% |
| **113** | 16.06 | 16.8 | 89% |
| **118** | 0.96 | 4.62 | 61% |
| **119** | 1.02 | 1.11 | 93% |
| **120** | 27.85 | 38.95 | 90% |
| **120-b-2** | 21.41 | 23.75 | 93% |
| **121** | 10.84 | 13.99 | 92% |

Other compounds of the present application were tested according to the above experimental method, and it was found that the relative DC₅₀ (nM) values of the compounds of the present application were 0.01 to 100, and the relative DC₅₀ (nM) values of preferred compounds of the present application were less than 100; the absolute DC₅₀ (nM) values of the compounds of the present application were 0.01 to 100, and the absolute DC₅₀ (nM) values of preferred compounds of the present application were less than 100; and the Dmax of the compounds of the present application was 80% or higher, and the Dmax of preferred compounds of the present application was 90% or higher.

### Experimental conclusion:

The compounds of the present application have good degradation activity towards IRAK4 protein.

### Test Example 2: Study on inhibitory effect of compounds of the present application on LPS induced IL-6 release in human PBMC cells

### I. Recovery and seeding of PBMC, and treatment of compounds (human PBMC cells were purchased from Oricell, item number: Fpb003F)

1. Preparation of compounds
   Sample dilutions of a series of concentrations were obtained by dilution in DMSO using Bravo, and then 80 nL was transferred into a cell plate using ECHO. The final concentration of DMSO in the cell culture medium is 0.1%.
2. Frozen cells were quickly thawed in a 37°C water bath and stirred continuously.
3. 25 mL of fresh preheated culture medium was added into a 50 mL centrifuge tube, and then the cells were added dropwise. Then the cells were centrifuged at 2,000 rpm for 10 min.
4. The supernatant was discarded and the cells were resuspended in 28.5 mL of fresh preheated complete RPMI 1640 culture medium.
5. The total number of cells required was calculated based on cell concentration to perform the assay. 7×10e4 cells were added per well (70 µL).
6. The cell plate was placed and incubated in a 5% CO₂ incubator at 37°C for 2 h.

### II. LPS treatment and collection of supernatant

1. LPS: A stock solution (1 mg/mL) was diluted in dH₂O, aliquoted, and stored at -80°C.
2. 8-fold LPS was added per well (10 µl/well) (with a final concentration of 5 ng/mL). The cells were incubated in a 5% CO₂ incubator at 37°C for 4 h.
3. 70 µl of supernatant was collected per well using Bravo, and then IL-6 HTRF assay was performed. The supernatant may be stored at -80°C.

### III. HTRF assay

1. Preparation of standards and sample dilutions.
2. 16 µL of each sample was taken using Bravo and added to wells. Then 16 µL of each standard was taken and added to corresponding wells.
3.4 µL of a premixed IL6 antibody working solution was added to each of all wells.
4. The cell plate was sealed and incubated at room temperature for 2 h.
5. The results were read and are as shown in Table 3:

**Table 3**

| Compound No. | IC₅₀ (nM) |
|---|---|
| 1-a | 0.91 |
| 1-b | 0.86 |
| 1 | 1.15 |
| 4 | 4.08 |
| 46 | 13.7 |
| 47 | 0.52 |
| 93 | 0.18 |
| 94 | 64.5 |
| 96 | 0.23 |
| 100 | 0.64 |
| 113 | 0.19 |
| 120-b-2 | 0.75 |
| 121 | 0.10 |

Other compounds of the present application were tested according to the above experimental method, and it was found that the IC₅₀ (nM) values of the compounds of the present application in inhibiting IL-6 release in human PBMC cells were 0.01 to 500, and the IC₅₀ (nM) values of preferred compounds of the present application were less than 100.

### Experimental conclusion:

The compounds of the present application have a good inhibitory effect on LPS induced IL-6 release in human PBMC cells.

### Test Example 3: Study on pharmacokinetic behavior of compounds of the present application in mice

Experimental drug: Compounds of the present application, self-made.

### Experimental plan:

Three healthy ICR mice (SPF grade, from Beijing Vital River Laboratory Animal Technology Co., Ltd.), male, weighing 18-25 g, were intravenously injected with a compound (1 mg/kg) at a volume of 5 mL/kg. The compound was prepared using 5% DMSO+10% Solutol+85% Saline (w/v), and the animals were not fasted before the experiment.

Three healthy ICR mice (SPF grade, from Beijing Vital River Laboratory Animal Technology Co., Ltd.), male, weighing 18-25 g, were intragastrically administered a compound (5 mg/kg) at a volume of 10 mL/kg. The compound was prepared using 5% DMSO+10% Solutol+85% Saline (w/v), and the animals were not fasted before the experiment.

Blood samples (about 0.05 mL each) were collected from the cheeks at 5 min, 15 min, 30 min, 1 h, 2 h, 4 h, 6 h, 8 h, and 24 h after intravenous administration, and at 5 min, 15 min, 30 min, 1 h, 2 h, 4 h, 6 h, 8 h, and 24 h after intragastrical administration, and anticoagulated with heparin sodium. Following collection, the blood samples were placed on ice and the plasma was centrifuged within 1 h (centrifugation conditions: 6,000 g, 3 min, 2 °C to 8°C). The concentration of the compounds in the plasma was determined by liquid chromatography tandem mass spectrometry. The plasma samples were stored in a -80°C freezer before analysis. With plasma concentration data at different time points, pharmacokinetic parameters were calculated by Phoenix WinNonlin8.2.0, and the results are shown in Table 4:

**Table 4**

| Compound | Intravenous injection (1 mg/kg) | | | | Intragastric administration (5 mg/kg) | | | |
|---|---|---|---|---|---|---|---|---|
| | Cₘₐₓ (ng/mL) | AUClast (h*ng/mL) | CL (mL/kg/min) | Vss (L/kg) | Cₘₐₓ (ng/mL) | AUCₗₐₛₜ (h*ng/mL) | T_{1/2} (h) | F% |
| 1 | 1740 | 12937 | 0.905 | 1.05 | 5153 | 82491 | 8.03 | 93.86 |
| 1-a | 832 | 8794 | 1.25 | 1.64 | 1067 | 19336 | 17.5 | 31.7 |
| 1-b | 1083 | 11208 | 1.0 | 1.23 | 2623 | 44547 | 15.3 | 79.5 |
| 4 | 1317 | 10531 | 1.27 | 1.07 | 2127 | 34166 | 14.8 | 60.1 |
| 46 | 128 | 932 | 6.80 | 21.4 | 207 | 3815 | 27.3 | 68.7 |
| 93 | 783 | 6214 | 2.98 | 0.958 | 844 | 9153 | 4.19 | 29.5 |
| 96 | 243 | 2056 | 4.59 | 7.97 | 239 | 3640 | 14.7 | 35.4 |
| 120-b-1 | 888 | 7249 | 2.10 | 1.19 | 1457 | 16903 | 5.32 | 44.5 |
| 120-b-2 | 725 | 4925 | 3.03 | 1.80 | 1790 | 24277 | 9.45 | 98.6 |

The experimental results indicate that the compounds of the present application have a low clearance rate, high plasma exposure, high oral utilization, and good pharmacokinetic properties.

Although the specific embodiments of the present application are described above, those skilled in the art should understand that these are merely examples, and various changes or modifications can be made to these embodiments without departing from the principles and essence of the present application. Therefore, the protection scope of the present application is limited by the appended claims.

## Claims

1. A compound, a stereoisomer thereof, or a pharmaceutically acceptable salt thereof, wherein the compound is selected from compounds as shown in general formula (I): 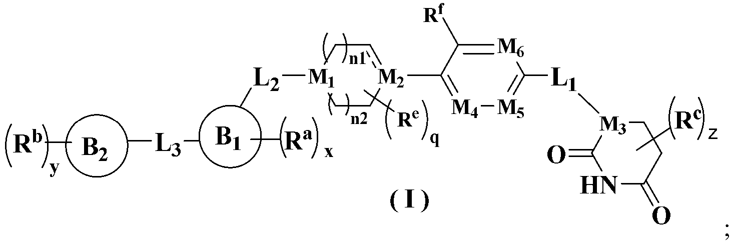 wherein
------ represents a bond or non-existence;
M₁ is selected from N or CR₁;
M₂ is selected from N, C, or CR₂;
M₃ is selected from N or CR₃;
M₄ is selected from N or CR₄;
M₅ is selected from N or CR₅;
M₆ is selected from N or CR₆;
R₁, R₂, R₃, R₄, R₅, and R₆ are each independently selected from hydrogen, deuterium, halogen, cyano, hydroxyl, oxo, alkyl, alkoxyl, aminoalkyl, hydroxyalkyl, haloalkyl, haloalkoxyl, cycloalkyl, or heterocyclyl;
ring B₁ is selected from aryl or heteroaryl;
ring B₂ is selected from aryl, heteroaryl, or heterocyclyl;
R^{a} is each independently selected from hydrogen, deuterium, hydroxyl, halogen, cyano, alkyl, alkoxyl, hydroxyalkyl, haloalkyl, haloalkoxyl, -P(O)RR', cycloalkyl, or heterocyclyl, the alkyl, alkoxyl, hydroxyalkyl, haloalkyl, haloalkoxyl, cycloalkyl, and heterocyclyl being optionally further substituted with one or more substituents selected from deuterium, halogen, hydroxyl, cyano, or alkyl;
R and R' are each independently selected from hydrogen, deuterium, halogen, alkyl, alkoxyl, haloalkyl, or haloalkoxyl;
R^{b}, R^{c}, R^{c}, and R^{f} are each independently selected from hydrogen, deuterium, halogen, cyano, oxo, alkyl, alkoxyl, hydroxyalkyl, haloalkyl, haloalkoxyl, cycloalkyl, or heterocyclyl, the alkyl, alkoxyl, hydroxyalkyl, haloalkyl, haloalkoxyl, cycloalkyl, and heterocyclyl being optionally further substituted with one or more substituents selected from deuterium, halogen, alkyl, alkoxyl, hydroxyalkyl, haloalkyl, or haloalkoxyl;
or, R^{c} and R^{f} are linked to form cycloalkyl, heterocyclyl, aryl, or heteroaryl, the cycloalkyl, heterocyclyl, aryl, and heteroaryl being optionally further substituted with one or more substituents selected from deuterium, halogen, oxo, hydroxyl, alkyl, alkoxyl, hydroxyalkyl, haloalkyl, haloalkoxyl, cycloalkyl, or heterocyclyl;
or, R₂ and R^{f} are linked to form cycloalkyl, heterocyclyl, aryl, or heteroaryl, the cycloalkyl, heterocyclyl, aryl, and heteroaryl being optionally further substituted with one or more substituents selected from deuterium, halogen, oxo, alkyl, alkoxyl, hydroxyalkyl, haloalkyl, haloalkoxyl, cycloalkyl, or heterocyclyl;
or, R^{f} is linked to C or N on a ring where M₂ is located to form cycloalkyl, heterocyclyl, aryl, or heteroaryl, the cycloalkyl, heterocyclyl, aryl, and heteroaryl being optionally further substituted with one or more substituents selected from deuterium, halogen, oxo, alkyl, alkoxyl, hydroxyalkyl, haloalkyl, haloalkoxyl, cycloalkyl, or heterocyclyl;
or, any two R^{b} are linked to form cycloalkyl, heterocyclyl, aryl, or heteroaryl, the cycloalkyl, heterocyclyl, aryl, and heteroaryl being optionally further substituted with one or more substituents selected from deuterium, halogen, cyano, amino, cyano, oxo, alkyl, alkoxyl, hydroxyalkyl, haloalkyl, haloalkoxyl, cycloalkyl, or heterocyclyl;
or, L₂ and R^{c} are linked to form cycloalkyl, heterocyclyl, aryl, or heteroaryl, the cycloalkyl, heterocyclyl, aryl, and heteroaryl being optionally further substituted with one or more substituents selected from deuterium, halogen, cyano, amino, cyano, oxo, alkyl, alkoxyl, hydroxyalkyl, haloalkyl, haloalkoxyl, cycloalkyl, or heterocyclyl;
L₁ is selected from a bond, -NH-, -S-, -O-, -CH₂-, CH₂CH₂-, -C(O)NH-, -NHC(O)-, or -C(O)-;
L₂ is -Ak1-Cy1-Ak2-Cy2-Ak3-,
Ak1, Ak2, and Ak3 being each independently selected from -(CH₂)ₙ₃-, -O-, -C(O)-, -NH-, -NR₇-, -CH₂NR₇-, -(CR₈R₉)ₙ₄-, alkynylene, or a bond,
Cyl and Cy2 being each independently selected from a bond, cycloalkylene, heterocyclylene, arylene, or heteroarylene, the cycloalkylene, heterocyclylene, arylene, and heteroarylene being optionally further substituted with 1 to 4 substituents selected from deuterium, halogen, amino, hydroxyl, cyano, nitro, oxo, alkyl, haloalkyl, alkoxyl, hydroxyalkyl, or haloalkoxyl;
R₇, R₈, and R₉ are each independently selected from hydrogen, deuterium, halogen, alkyl, cyano, hydroxyl, cycloalkyl, haloalkyl, deuterated alkyl, halocycloalkyl, hydroxyalkyl, or alkoxyl; or, R₈ and R₉ are linked to form cycloalkyl, heterocyclyl, aryl, or heteroaryl, the cycloalkyl, heterocyclyl, aryl, and heteroaryl being optionally further substituted with one or more substituents selected from deuterium, halogen, hydroxyl, amino, cyano, oxo, alkyl, alkoxyl, haloalkyl, haloalkoxyl, or hydroxyalkyl;
or, R^{a} and L₂ are linked to form cycloalkyl, heterocyclyl, aryl, or heteroaryl, the cycloalkyl, heterocyclyl, aryl, and heteroaryl being optionally further substituted with one or more substituents selected from deuterium, halogen, amino, cyano, hydroxyl, oxo, alkyl, alkoxyl, hydroxyalkyl, haloalkyl, haloalkoxyl, cycloalkyl, or heterocyclyl;
L₃ is selected from a bond, -NH-C(O)-, -C(O)-NH-, -NH-C(S)-, or -C(S)-NH-;
x, y, z, and q are each independently selected from 0, 1, 2, 3, or 4; and
n1, n2, n3, and n4 are each independently selected from 0, 1, 2, or 3.

2. The compound, the stereoisomer thereof, or the pharmaceutically acceptable salt thereof according to claim 1, wherein the compound is further shown in general formula (II-A): 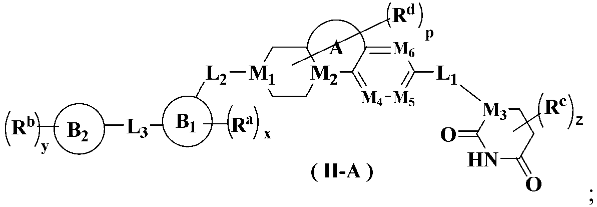 wherein
R^{d} is each independently selected from hydrogen, deuterium, halogen, oxo, hydroxyl, C₁₋₆ alkyl, C₁₋₆ alkoxyl, hydroxy C₁₋₆ alkyl, C₁₋₆ haloalkyl, C₁₋₆ haloalkoxyl, C₃₋₆ cycloalkyl, or 3-6 membered heterocyclyl;
preferably, R^{d} is each independently selected from hydrogen, deuterium, fluoro, chloro, oxo, hydroxyl, C₁₋₃ alkyl, C₁₋₃ alkoxyl, hydroxy C₁₋₃ alkyl, C₁₋₃ haloalkyl, C₁₋₃ haloalkoxyl, C₃₋₆ cycloalkyl, or 3-6 membered heterocyclyl;
or, R^{d} and L₂ are linked to form a C₃₋₆ cycloalkyl or 3-6 membered heterocyclyl, the C₃₋₆ cycloalkyl and 3-6 membered heterocyclyl being optionally further substituted with one or more substituents selected from deuterium, halogen, cyano, oxo, C₁₋₆ alkyl, C₁₋₆ alkoxyl, hydroxy C₁₋₆ alkyl, C₁₋₆ haloalkyl, C₁₋₆ haloalkoxyl, C₃₋₆ cycloalkyl, or 3-6 membered heterocyclyl;
ring A is selected from 5-7 membered heterocyclyl or 5-6 membered heteroaryl; and
p is selected from 0, 1, 2, 3, or 4.

3. The compound, the stereoisomer thereof, or the pharmaceutically acceptable salt thereof according to claim 1, wherein the compound is further shown in general formula (II-B): 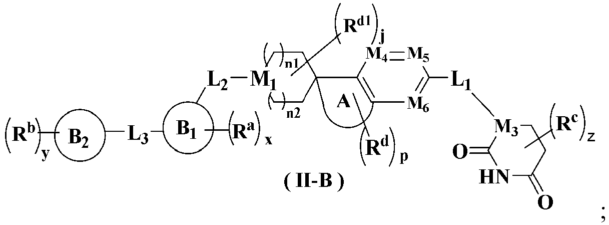 wherein
R^{d} is each independently selected from hydrogen, deuterium, halogen, oxo, C₁₋₆ alkyl, C₁₋₆ alkoxyl, hydroxy C₁₋₆ alkyl, C₁₋₆ haloalkyl, C₁₋₆ haloalkoxyl, C₃₋₆ cycloalkyl, or 3-6 membered heterocyclyl;
ring A is selected from 5-6 membered heterocyclyl or 5-6 membered heteroaryl; and
R^{d1} is each independently selected from hydrogen, deuterium, halogen, oxo, C₁₋₆ alkyl, C₁₋₆ alkoxyl, hydroxy C₁₋₆ alkyl, C₁₋₆ haloalkyl, C₁₋₆ haloalkoxyl, C₃₋₆ cycloalkyl, or 3-6 membered heterocyclyl; and
p and j are each independently selected from 0, 1, 2, 3, or 4.

4. The compound, the stereoisomer thereof, or the pharmaceutically acceptable salt thereof according to claim 2, wherein the 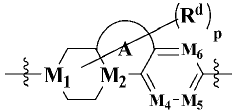 is selected from 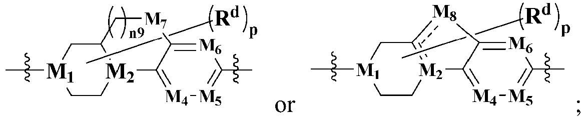
preferably, 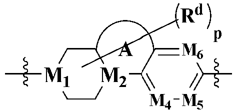 is selected from 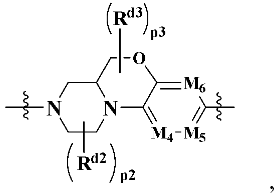 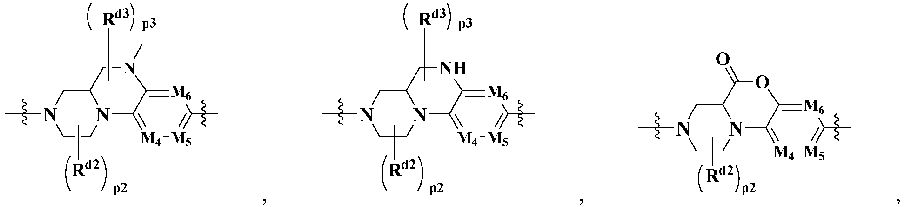 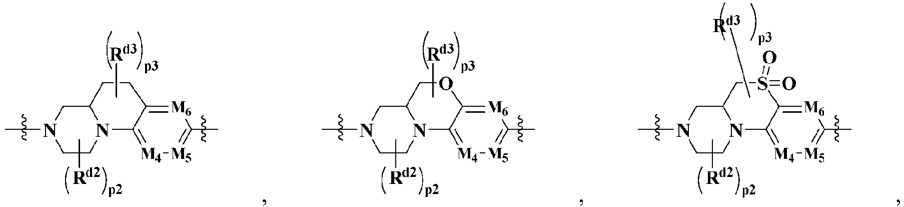 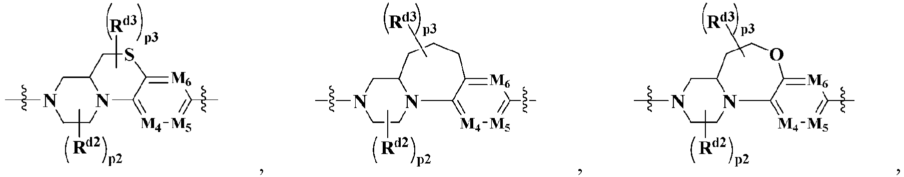 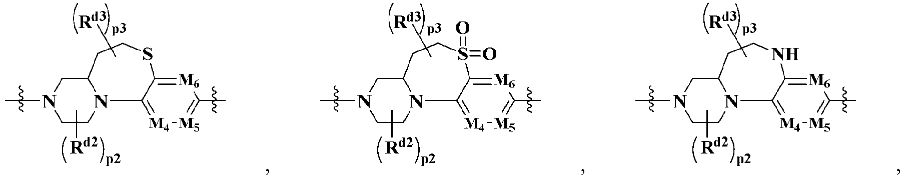 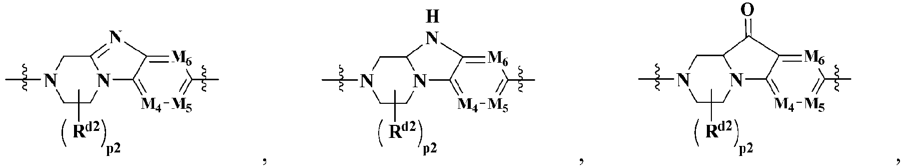 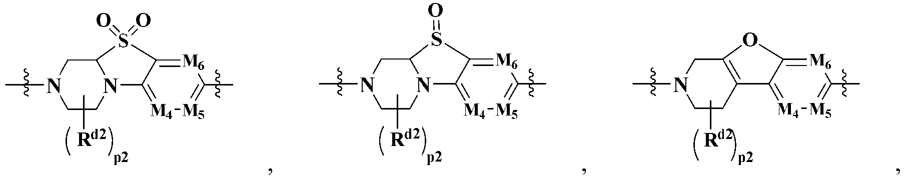 or 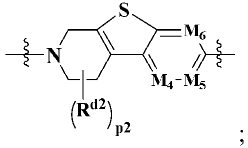
M₇ is selected from O, CH₂, C(O), S, S(O), S(O)₂, or NR₁₀; R₁₀ is selected from hydrogen, deuterium, C₁₋₃ alkyl, C₁₋₃ alkoxyl, C₁₋₃ haloalkyl, C₁₋₃ haloalkoxyl, C₃₋₆ cycloalkyl, or 3-6 membered heterocyclyl, the C₁₋₃ alkyl, C₁₋₃ alkoxyl, C₁₋₃ haloalkyl, C₁₋₃ haloalkoxyl, C₃₋₆ cycloalkyl, and 3-6 membered heterocyclyl being optionally further substituted with one or more substituents selected from deuterium, hydroxyl, cyano, amino, oxo, fluoro, or chloro;
M₈ is selected from N, O, S, C(O), CH₂, CH, S(O), or S(O)₂;
R^{d2} and R^{d3} are each independently selected from hydrogen, deuterium, halogen, amino, cyano, oxo, hydroxyl, C₁₋₃ alkyl, C₁₋₃ alkoxyl, C₁₋₃ haloalkyl, C₁₋₃ haloalkoxyl, C₃₋₆ cycloalkyl, or 3-6 membered heterocyclyl, the C₁₋₃ alkyl, C₁₋₃ alkoxyl, C₁₋₃ haloalkyl, C₁₋₃ haloalkoxyl, C₃₋₆ cycloalkyl, and 3-6 membered heterocyclyl being optionally further substituted with one or more substituents selected from deuterium, hydroxyl, cyano, amino, oxo, fluoro, or chloro;
p2 and p3 are each independently selected from 1, 2, 3, or 4; and
n9 is selected from 1, 2, or 3.

5. The compound, the stereoisomer thereof, or the pharmaceutically acceptable salt thereof according to claim 3, wherein the 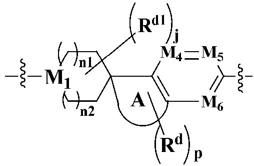 is selected from 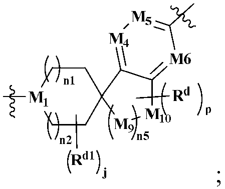
preferably, the 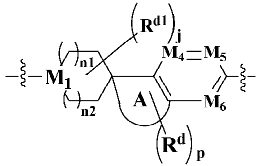 is selected from 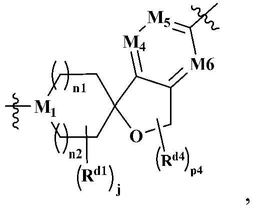 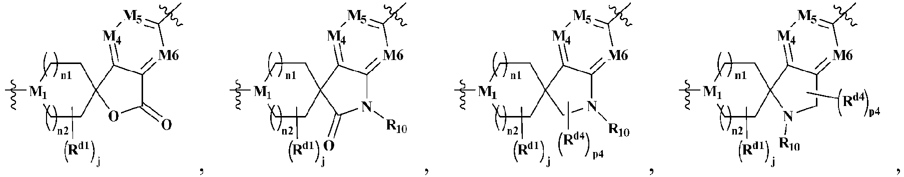 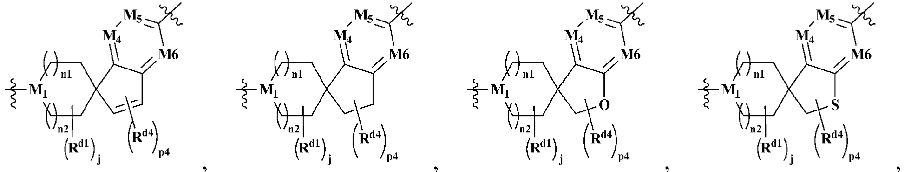 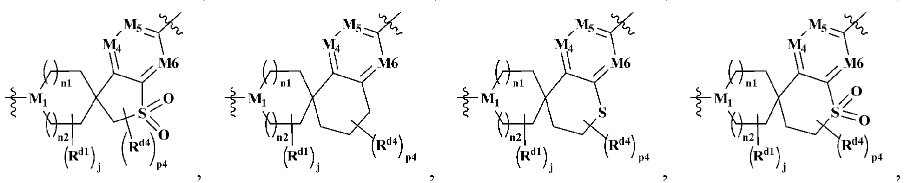 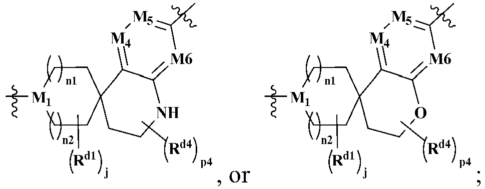
M₉ and M₁₀ are each independently selected from CH₂, C(O), NR₁₀, CH, O, S, S(O), or S(O)₂;
R₁₀ is selected from hydrogen, deuterium, halogen, C₁₋₃ alkyl, C₁₋₃ alkoxyl, C₁₋₃ haloalkyl, C₁₋₃ haloalkoxyl, C₃₋₆ cycloalkyl, or 3-6 membered heterocyclyl, the C₁₋₃ alkyl, C₁₋₃ alkoxyl, C₁₋₃ haloalkyl, C₁₋₃ haloalkoxyl, C₃₋₆ cycloalkyl, and 3-6 membered heterocyclyl being optionally further substituted with one or more substituents selected from deuterium, hydroxyl, cyano, amino, oxo, fluoro, or chloro;
R^{d4} is each independently selected from hydrogen, deuterium, halogen, amino, cyano, oxo, C₁₋₃ alkyl, C₁₋₃ alkoxyl, C₁₋₃ haloalkyl, C₁₋₃ haloalkoxyl, C₃₋₆ cycloalkyl, or 3-6 membered heterocyclyl, the C₁₋₃ alkyl, C₁₋₃ alkoxyl, C₁₋₃ haloalkyl, C₁₋₃ haloalkoxyl, C₃₋₆ cycloalkyl, and 3-6 membered heterocyclyl being optionally further substituted with one or more substituents selected from deuterium, hydroxyl, cyano, amino, oxo, fluoro, or chloro;
p4 is each independently selected from 1, 2, 3, or 4; and
n5 is selected from 1, 2, or 3.

6. The compound, the stereoisomer thereof, or the pharmaceutically acceptable salt thereof according to any one of claims 1 to 5, wherein the ring B1 is selected from indazolyl, pyrazolyl, benzimidazolyl, pyridinotriazolyl, pyridinopyrazolyl, pyridinoimidazolyl, or pyrimidoimidazolyl;
preferably, the 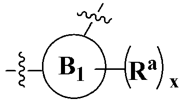 is selected from 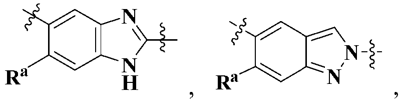 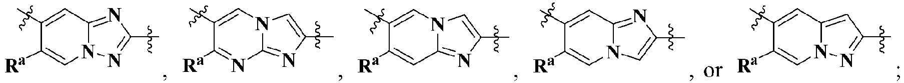 the ring B₂ is selected from pyridyl, phenyl, pyridonyl, pyridazinonyl, pyrimidopyrazolyl, pyridinopyrrolyl, pyrimidinyl, pyrimidopyrrolyl, or pyridinopyrazolyl;
preferably, the 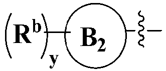 is selected from 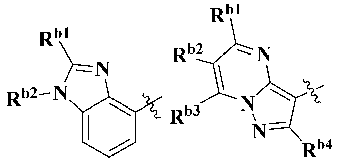 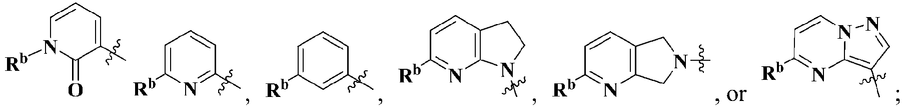
R^{b1}, R^{b2}, R^{b3}, and R^{b4} are each independently selected from hydrogen, deuterium, halogen, cyano, hydroxyl, C₁₋₆ alkyl, C₁₋₆ alkoxyl, C₁₋₆ haloalkyl, C₁₋₆ hydroxyalkyl, C₁₋₆ haloalkoxyl, C₃₋₆ cycloalkyl, or 3-6 membered heterocyclyl;
or, R^{b1} and R^{b2} are linked to form 5-6 membered heterocyclyl, the 5-6 membered heterocyclyl being optionally further substituted with one or more substituents selected from deuterium, halogen, cyano, hydroxyl, C₁₋₃ alkyl, C₁₋₃ alkoxyl, C₁₋₃ haloalkyl, C₁₋₃ haloalkoxyl, C₃₋₆ cycloalkyl, or 3-6 membered heterocyclyl.

7. The compound, the stereoisomer thereof, or the pharmaceutically acceptable salt thereof according to any one of claims 1 to 6, wherein the R^{a} is each independently selected from hydrogen, deuterium, halogen, cyano, C₁₋₆ alkyl, C₁₋₆ alkoxyl, hydroxy C₁₋₆ alkyl, C₁₋₆ haloalkyl, C₁₋₆ haloalkoxyl, -P(O)RR', C₃₋₆ cycloalkyl, or 3-6 membered heterocyclyl, the C₁₋₆ alkyl, C₁₋₆ alkoxyl, hydroxy C₁₋₆ alkyl, C₁₋₆ haloalkyl, C₁₋₆ haloalkoxyl, C₃₋₆ cycloalkyl, and 3-6 membered heterocyclyl being optionally further substituted with one or more substituents selected from deuterium, halogen, hydroxyl, cyano, or C₁₋₃ alkyl;
preferably, R^{a} is each independently selected from hydrogen, deuterium, halogen, cyano, C₁₋₃ alkyl, C₁₋₃ alkoxyl, hydroxy C₁₋₃ alkyl, C₁₋₃ haloalkyl, C₁₋₃ haloalkoxyl, -P(O)RR', C₃₋₆ cycloalkyl, or 3-6 membered heterocyclyl, the C₁₋₃ alkyl, C₁₋₃ alkoxyl, hydroxy C₁₋₃ alkyl, C₁₋₃ haloalkyl, C₁₋₃ haloalkoxyl, C₃₋₆ cycloalkyl, and 3-6 membered heterocyclyl being optionally further substituted with one or more substituents selected from deuterium, halogen, hydroxyl, cyano, or C₁₋₃ alkyl;
or, the R^{b}, R^{c}, R^{c}, and R^{f} are each independently selected from hydrogen, deuterium, halogen, cyano, oxo, C₁₋₆ alkyl, C₁₋₆ alkoxyl, hydroxy C₁₋₆ alkyl, C₁₋₆ haloalkyl, C₁₋₆ haloalkoxyl, C₃₋₆ cycloalkyl, or 3-6 membered heterocyclyl, the C₁₋₆ alkyl, C₁₋₆ alkoxyl, hydroxy C₁₋₆ alkyl, C₁₋₆ haloalkyl, C₁₋₆ haloalkoxyl, C₃₋₆ cycloalkyl, and 3-6 membered heterocyclyl being optionally further substituted with one or more substituents selected from deuterium, halogen, hydroxyl, C₁₋₃ alkyl, C₁₋₃ alkoxyl, hydroxy C₁₋₃ alkyl, C₁₋₃ haloalkyl, or C₁₋₃ haloalkoxyl;
preferably, the R^{b}, R^{c}, R^{c}, and R^{f} are each independently selected from hydrogen, deuterium, fluoro, chloro, bromo, cyano, oxo, C₁₋₃ alkyl, C₁₋₃ alkoxyl, hydroxy C₁₋₃ alkyl, C₁₋₃ haloalkyl, C₁₋₃ haloalkoxyl, C₃₋₆ cycloalkyl, or 3-6 membered heterocyclyl, the C₁₋₃ alkyl, C₁₋₃ alkoxyl, hydroxy C₁₋₃ alkyl, C₁₋₃ haloalkyl, C₁₋₃ haloalkoxyl, C₃₋₆ cycloalkyl, and 3-6 membered heterocyclyl being optionally further substituted with one or more substituents selected from deuterium, fluoro, chloro, hydroxyl, C₁₋₃ alkyl, C₁₋₃ alkoxyl, hydroxy C₁₋₃ alkyl, C₁₋₃ haloalkyl, or C₁₋₃ haloalkoxyl;
or, R₁, R₂, R₃, R₄, R₅, and R₆ are each independently selected from hydrogen, deuterium, halogen, cyano, oxo, C₁₋₆ alkyl, C₁₋₆ alkoxyl, C₁₋₆ hydroxyalkyl, C₁₋₆ haloalkyl, C₁₋₆ haloalkoxyl, C₃₋₆ cycloalkyl, or 3-6 membered heterocyclyl;
preferably, R₁, R₂, R₃, R₄, R₅, and R₆ are each independently selected from hydrogen, deuterium, fluoro, chloro, cyano, oxo, C₁₋₃ alkyl, C₁₋₃ alkoxyl, C₁₋₃ hydroxyalkyl, C₁₋₃ haloalkyl, C₁₋₃ haloalkoxyl, C₃₋₆ cycloalkyl, or 3-6 membered heterocyclyl;
or, R and R' are each independently selected from hydrogen, deuterium, halogen, C₁₋₆ alkyl, C₁₋₆ alkoxyl, C₁₋₆ haloalkyl, or C₁₋₆ haloalkoxyl; and
preferably, R and R' are each independently selected from hydrogen, deuterium, fluoro, chloro, C₁₋₃ alkyl, C₁₋₃ alkoxyl, C₁₋₃ haloalkyl, or C₁₋₃ haloalkoxyl.

8. The compound, the stereoisomer thereof, or the pharmaceutically acceptable salt thereof according to any one of claims 1 to 7, wherein the L₂ is -Ak1-Cy1-Ak2-Cy2-Ak3-,
the Ak1, Ak2, and Ak3 are each independently selected from -(CH₂)ₙ₃-, -O-, -C(O)-, -NH-, -NR₇-, -CH₂NR₇-, -(CR₈R₉)ₙ₄-, or a bond;
the Cy1 and Cy2 are each independently selected from a bond, cyclohexylene, piperidinylene, or piperazinylene, the cyclohexylene, piperidinylene, and piperazinylene being optionally further substituted with 1 to 4 substituents selected from deuterium, halogen, hydroxyl, cyano, oxo, C₁₋₃ alkyl, hydroxy C₁₋₃ alkyl, C₁₋₃ alkoxyl, C₃₋₄ cycloalkyl, 3-4 membered heterocyclyl, C₁₋₃ haloalkyl, or C₁₋₃ haloalkoxyl;
the R₇, R₈, and R₉ are each independently selected from hydrogen, deuterium, halogen, C₁₋₃ alkyl, cyano, hydroxyl, C₃₋₄ cycloalkyl, C₁₋₃ haloalkyl, C₁₋₃ deuterated alkyl, C₁₋₃ halocycloalkyl, hydroxy C₁₋₃ alkyl, or C₁₋₃ alkoxyl;
preferably, L₂ is selected from 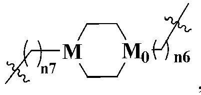 , M and M₀ being each independently selected from CR₁₁ or N; R₁₁ is selected from hydrogen, deuterium, halogen, hydroxyl, C₁₋₃ alkyl, C₁₋₃ hydroxyalkyl, C₁₋₃ haloalkyl, or C₁₋₃ haloalkoxyl; and
n6 and n7 are each independently selected from 0, 1, 2, 3, or 4.

9. The compound, the stereoisomer thereof, or the pharmaceutically acceptable salt thereof according to any one of claims 2, 4, 6 to 8, wherein the compound of general formula (II-A) is further shown in general formula (IV), general formula (IV-1), or general formula (IV-2):

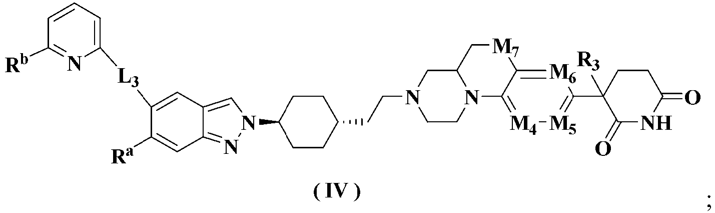

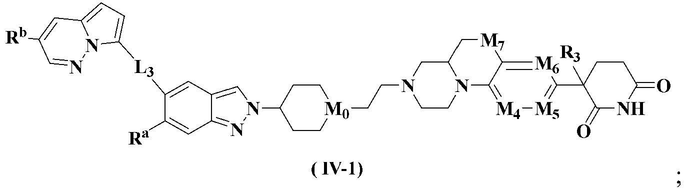

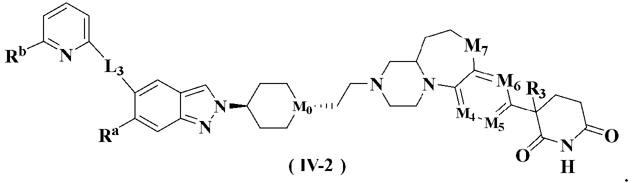

10. The compound, the stereoisomer thereof, or the pharmaceutically acceptable salt thereof according to claim 9, wherein the compound of general formula (II-A) is further shown in general formula (IV-A) or general formula (IV-B):

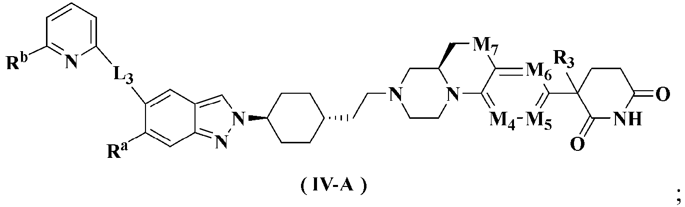

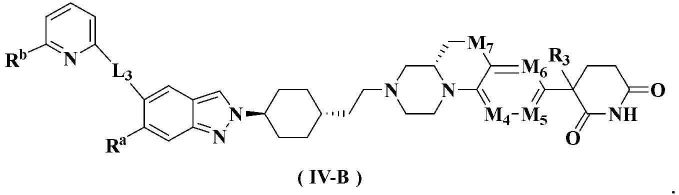

11. The compound, the stereoisomer thereof, or the pharmaceutically acceptable salt thereof according to any one of claims 3, 5 to 8, wherein the compound of general formula (II-B) is further shown in general formula (V):

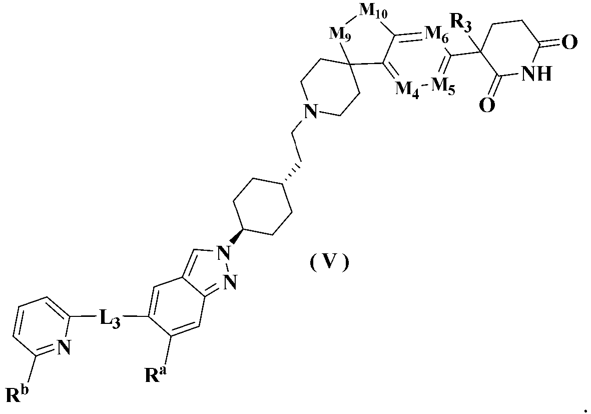

12. The compound, the stereoisomer thereof, or the pharmaceutically acceptable salt thereof according to any one of claims 1 to 11, satisfying one or more of the following conditions:
(1) the R^{a} is selected from C₁₋₃ alkyl, C₁₋₃ alkoxyl, hydroxy C₁₋₃ alkyl, C₁₋₃ haloalkyl, or C₁₋₃ haloalkoxyl; preferably C₁₋₃ alkoxyl or hydroxy C₁₋₃ alkyl; more preferably methoxyl, ethoxyl, propoxyl, hydroxymethyl, hydroxyethyl, or hydroxypropyl;
(2) the R^{b} is selected from C₁₋₃ alkyl, C₁₃ alkoxyl, hydroxy C₁₋₃ alkyl, C₁₋₃ haloalkyl, or C₁₋₃ haloalkoxyl; preferably C₁₋₃ haloalkyl or C₁₋₃ haloalkoxyl; more preferably difluoromethyl, trifluoromethyl, or trifluoromethoxyl;
(3) the L₃ is selected from a bond, -NH-C(O)-, or -C(O)-NH-;
(4) the R₃ is selected from hydrogen, deuterium, fluoro, chloro, cyano, C₁₋₃ alkyl, C₁₋₃ alkoxyl, C₁₋₃ hydroxyalkyl, C₁₋₃ haloalkyl, or C₁₋₃ haloalkoxyl; preferably hydrogen, deuterium, fluoro, chloro, C₁₋₃ alkyl, C₁₋₃ alkoxyl, or C₁₋₃ haloalkyl; more preferably hydrogen, deuterium, fluoro, methyl, ethyl, propyl, trifluoromethyl, methoxyl, ethoxyl, or propoxyl;
(5) the M₀ is selected from N or CH;
(6) the M₄ is N or CR₄, R₄ being selected from hydrogen, deuterium, fluoro, chloro, C₁₋₃ alkyl, C₁₋₃ alkoxyl, C₁₋₃ hydroxyalkyl, C₁₋₃ haloalkyl, or C₁₋₃ haloalkoxyl; preferably hydrogen, deuterium, fluoro, chloro, methyl, ethyl, methoxyl, ethoxyl, hydroxymethyl, or trifluoromethyl;
(7) the M₅ is N or CR₅, R₅ being selected from hydrogen, deuterium, fluoro, chloro, C₁₋₃ alkyl, C₁₋₃ alkoxyl, C₁₋₃ hydroxyalkyl, C₁₋₃ haloalkyl, or C₁₋₃ haloalkoxyl; preferably hydrogen, deuterium, fluoro, chloro, methyl, ethyl, methoxyl, ethoxyl, hydroxymethyl, or trifluoromethyl;
(8) the M₆ is N or CR₆, R₆ being selected from hydrogen, deuterium, fluoro, chloro, C₁₋₃ alkyl, C₁₋₃ alkoxyl, C₁₋₃ hydroxyalkyl, C₁₋₃ haloalkyl, or C₁₋₃ haloalkoxyl; preferably hydrogen, deuterium, fluoro, chloro, methyl, ethyl, methoxyl, ethoxyl, hydroxymethyl, or trifluoromethyl;
(9) the M₇ is O, CH₂, C(O), S, S(O), or S(O)₂; preferably O, CH₂, or S;
(10) the M₉ is CH₂, C(O), O, or S; and
(11) M₁₀ is CH₂, C(O), NR₁₀, O, or S, R₁₀ being selected from hydrogen, deuterium, C₁₋₃ alkyl, C₁₋₃ alkoxyl, C₁₋₃ haloalkyl, or C₁₋₃ haloalkoxyl; preferably hydrogen, deuterium, methyl, ethyl, propyl, methoxyl, ethoxyl, hydroxymethyl, or trifluoromethyl.

13. The compound, the stereoisomer thereof, or the pharmaceutically acceptable salt thereof according to any one of claims 1 to 12, wherein the compound is selected from compounds in Table 5.

14. A compound as shown in formula (A-a-a): 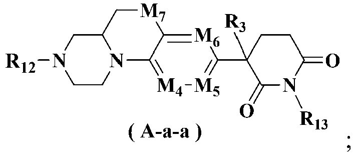 wherein M₄ is N or CR₄; M₅ is N or CR₅; M₆ is N or CR₆; M₇ is O, S, or CH₂; R₃ is selected from deuterium, halogen, C₁₋₃ alkyl, C₁₋₃ alkoxyl, or C₁₋₃ haloalkyl; R₄ is selected from hydrogen, deuterium, halogen, C₁₋₃ alkyl, C₃₋₅ cycloalkyl, C₁₋₃ alkoxyl, or C₁₋₃ haloalkyl; R₅ is selected from hydrogen, deuterium, fluoro, chloro, C₁₋₃ alkyl, C₁₋₃ alkoxyl, C₁₋₃ hydroxyalkyl, C₁₋₃ haloalkyl, or C₁₋₃ haloalkoxyl; R₆ is selected from hydrogen, deuterium, fluoro, chloro, C₁₋₃ alkyl, C₁₋₃ alkoxyl, C₁₋₃ hydroxyalkyl, C₁₋₃ haloalkyl, or C₁₋₃ haloalkoxyl; R₁₂ and R₁₃ are each independently selected from hydrogen or amino protecting groups.

15. The compound of formula (A-a-a) according to claim 14, wherein the compound of formula (A-a-a) is further shown in formula (A-a-a-1) and formula (A-a-a-2): 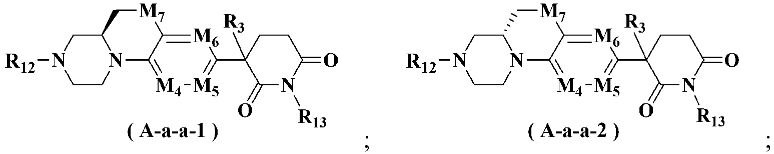 preferably, the compound of formula (A-a-a) is selected from intermediate E, intermediate E-a, intermediate E-b, intermediate F, intermediate G, intermediate I, intermediate M, intermediate O, intermediate P, intermediate Q, intermediate R, or intermediate S.

16. A pharmaceutical composition, comprising the compound, the stereoisomer thereof, or the pharmaceutically acceptable salt thereof according to any one of claims 1 to 13, and a pharmaceutically acceptable carrier.

17. Use of the compound, the stereoisomer thereof, or the pharmaceutically acceptable salt thereof according to any one of claims 1 to 13, or the pharmaceutical composition according to claim 16, in the preparation of a drug for treating or preventing IRAK4 mediated diseases.

18. The use according to claim 17, wherein the diseases are selected from autoimmune diseases, inflammatory diseases, cancers, viral diseases, neurodegenerative diseases, genetic disorders, hormone-related diseases, metabolic disorders, organ-transplantation-related diseases, immunodeficiency disorders, destructive bone diseases, proliferative disorders, infectious diseases, cell-death-related conditions, or cardiovascular diseases.
